# EUROPÄISCHE PATENTSCHRIFT

(11) **EP 2 863 925 B1**
(45) Veröffentlichungstag und Bekanntmachung des Hinweises auf die Patenterteilung: **14.09.2016**
(21) Anmeldenummer: 13730264.2
(22) Anmeldetag: 21.06.2013
(51) Int. Cl.: A61K 31/7028, A61P 31/04, A61P 33/00, C07H 15/18, C07H 15/12

(54) **SIALINSÄUREDERIVATE**
SIALIC ACID DERIVATIVES
DÉRIVÉ D'ACIDE SIALIQUE

(30) Priorität: 21.06.2012 EP 12172939
(43) Veröffentlichungstag der Anmeldung: 29.04.2015
(73) Patentinhaber: Brossmer, Reinhard, 69120 Heidelberg (DE); Prescher, Horst, 4057 Basel (CH)
(72) Erfinder: Brossmer, Reinhard, 69120 Heidelberg (DE); Prescher, Horst, 4057 Basel (CH)
(74) Vertreter: Rippel, Hans Christoph
(86) Internationale Anmeldenummer: PCT/EP2013/063020
(87) Internationale Veröffentlichungsnummer: WO 2013/190103

(56) Entgegenhaltungen:
- WO-A2-03/000709
- WO-A2-2007/056525
- COLLINS B E ET AL: "High-affinity ligand probes of CD22 overcome the threshold set by cis ligands to allow for binding, endocytosis, and killing of B cells", THE JOURNAL OF IMMUNOLOGY, THE AMERICAN ASSOCIATION OF IMMUNOLOGISTS, US, Bd. 177, Nr. 5, 1. September 2006 (2006-09-01), Seiten 2994-3003, XP003013261, ISSN: 0022-1767 in der Anmeldung erwähnt
- HAJJAJ ABDU-ALLAH ET AL: "Design and Synthesis of a Multivalent Heterobifunctional CD22 Ligand as a Potential Immunomodulator", SYNTHESIS, Bd. 2011, Nr. 18, 1. September 2011 (2011-09-01), Seiten 2968-2974, XP055027695, ISSN: 0039-7881, DOI: 10.1055/s-0030-1260151

## Beschreibung

Die Erfindung betrifft Derivate der Sialinsäure, Verfahren zur deren Herstellung und deren Verwendung zur Herstellung von Konjugaten.

Sialinsäure ist der Oberbegriff für eine Familie von 9-Kohlenstoffatom-Zuckern, die sämtliche Derivate der Neuraminsäure (Neu) und der Keto-desoxy-nonuloson-Säure (KDN) darstellen. Typischerweise befinden sich diese an den exponierten nichtreduzierenden Enden von Oligosaccharidketten. Sialinsäuren spielen vielfältige Rollen in Säugetieren und im menschlichen Organismus (Schauer (2004) Zoology, 107, 49-64; Varki (2008) Trends in Mol Med, 14, 8, 351- 360). Desweiteren werden sie von vielen Pathogenen genutzt um z.B. eine effiziente Infektion zu erreichen oder um dem Immunsystem des Wirtes zu entkommen (Glycoconjugate J. 2006, vol. 23, issue 1-2, alle Artikel). Viele solcher Funktionen werden über Proteine reguliert, welche Sialinsäuren erkennen (Lehmann et al (2006) Cell- Mol. Life Sci. 63, 1331-1354).

Eine Untergruppe solcher Proteine sind die Siglecs. Sie sind Lektine vom Ig-Typ, die durch eine N-terminale V-Set Domäne gekennzeichnet sind, welche eine spezifische Erkennung von Sialinsäuren ermöglicht. Ein Überblick über die bis heute bekannt gewordenen Typen von Siglec-Proteinen und mit Siglec-Inhibitoren potentiell behandelbare Krankheiten findet sich in Trends in "Pharmacological Sciences 2009, 30 (5), 240-248" und "Current Medicinal Chemistry 2011, 18, 3537-3550" und den enthaltenen Referenzen.

CD22 (Siglec-2) ist stark auf B-Zellen exprimiert. Es ist bekannt dass B-Zell basierte Erkrankungen, insbesondere Lymphome und Autoimmunkrankheiten mittels CD22 Liganden behandelt werden können (Tedder et al (2005) Advances in Immunology 88, 1-50; Fiorina et al (2008) Diabetis 57, 3013-3024).

Weiter ist bekannt, dass über die Verknüpfung von einem CD22-Liganden mit einem pharmakologisch wirksamen Molekül Konjugate zur Behandlung von CD22 und B-Zell vermittelten Krankheiten hergestellt werden können. Es wurden beispielweise bereits sialinsäurehaltige Siglec Trisaccharid-Liganden zur Herstellung von Cargo tragenden Polymeren entwickelt (Collins et al (2006) Journal of Immunology 177, 2994-3003 und WO 2007056525). Ebenfalls ist bekannt, dass sich monovalente sialinsäurehaltige Siglec Trisaccharid-Liganden und anti-Siglec-Antikörper zur Herstellung von Siglec spezifischen Transportvehikeln, insbesondere von Liposomen und Virsukapsiden, eignen (O'Donnell et al. (2010) Invest. New Drugs 28, 260-267; Chen et al. (2010) Blood 115, 23 4778-4786; Kaltgrad et al. (2008) J. Am. Chem Soc. 130, 4578-4579; Rhee (2011) Biomacromolecules 12, 3977-3981; WO 2012018377). Weiter wurden sialinsäurehaltige trisaccharid Siglec-Liganden zur Verknüpfung mit einem Cargo zur Herstellung von nicht-kovalent verknüpften Oligomeren entwickelt (O'Reilly (2011) J. Immunol. 186, 1554-1563). Weiter wurde an sialinsäurehaltige Siglec Trisaccharid-Liganden ein Cargo geknüpft, welches auf der Zelloberfläche eine Immunreaktion gegen das Cargo und die Siglec tragende Zelle auslöst (O'Reilly (2008) J. Am. Chem Soc. 130, 7736-7745; WO 2007056870).

Weiter wurden polymere CD22 Liganden mit einem Antigen verbunden, um Toleranz gegen das Antigen zu erzielen (Mihaylova et al. (2009), Mol. Immunol. 47, 1, 123-130; Courtney et al. (2009) PNAS 106, 8, 2500-2505; Duong et al. (2010) J. Exp. Medicine 207, 1, 173-187). Auch wurden Liposomen gleichzeitig mit CD22 Liganden oder anti-CD22-Antikörpern und Antigenen besetzt um Toleranz gegen das Antigen zu induzieren (WO 2012018380).

Die beschriebenen Derivate der Sialinsäure enthalten alle Galaktose, wodurch sie als potentielle Liganden für den Asialoglycoprotein-Rezeptor und für Galektine sind (Steirer et al. (2009) J. Biol. Chem. 284, 6, 3777-3783).

Obwohl die bekannten Derivate sich zur Verknüpfung mit einem Cargo eignen und die hergestellten Konjugate die gewünschten Eigenschaften haben, besteht doch ein breiter Raum für Verbesserungen, insbesondere was Affinität und Selektivität betrifft. Weiter besteht Raum für Verbesserungen bezüglich der notwendigen Anzahl an Liganden pro Cargo um ein Konjugat mit gewünschten Eigenschaften zu erhalten. Ebenso besteht Raum für Verbesserungen bezüglich der möglichen Verknüpfungsmöglichkeiten mit einem Cargo. Weiter besteht Raum für Verbesserungen bezüglich pharmakologischer Verträglichkeit und Verabreichungsformen sowie der Stabilität in Plasma und Leber.

Aufgabe der Erfindung ist es, Verbindungen bereitzustellen, mit denen zumindest in Teilbereichen Vorteile in den genannten Bereichen erzielt werden.

Es wurde gefunden, dass sich bestimmte dimere Sialinsäurederivate eine erhöhte Affinität zu CD22 aufweisen und sich gleichzeitig zur Verknüpfung mit einem Cargo eignen.

Gegenstand der Erfindung ist daher eine Verbindung der Formel (I), 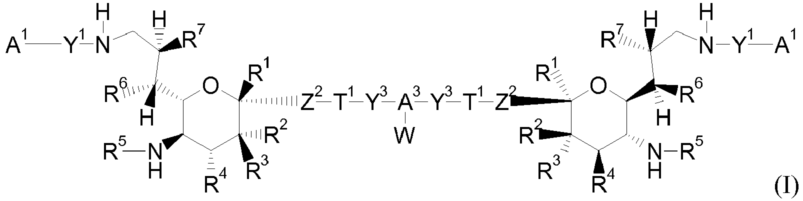 wobei die Symbole folgende Bedeutungen haben:
- A¹: ist gleich 4-Biphenyl, 4-(2-Thienyl)benzoyl, 4-(3-Thienyl)benzoyl, 1-Naphtyl und 2-Naphtyl, wobei die genannten Reste unsubstituiert oder ein- oder mehrfach durch eine Gruppe X¹ substituiert sind;
- X¹: ist gleich oder verschieden Fluor, Chlor, Hydroxy, Carboxy, SO₃M, OSO₃M, SO₂NH₂, SO₂CF₃, Alkyl, Cycloalkyl, Halogenalkyl, Alkyloxy, Halogenalkyloxy, Alkylcarbonyloxy, Alkylcyclocarbonyloxy, Aminocarbonyl, Alkylaminocarbonyl, Cycloalkylaminocarbonyl, Alkylcarbonylamino, Cycloalkylcarbonylamino, Alkylamino, Cycloalkylamino, Dialkylamino, Dicycloalkylamino oder Alkylcycloalkylamino wobei die Alkylgruppen in diesen Resten 1 bis 4 und die Cycloalkylgruppen 3 oder 4 Kohlenstoffatome enthalten;
- Y¹: ist gleich ∼(C₁-C₂-Alkyl)-, ∼C(O)- oder ∼CH₂C(O)- wobei ∼ die Bindung zur Gruppe A¹ bezeichnet;
- Z²: ist gleich -O-, -S- oder -CH₂-;
- T¹: ist gleich eine geradkettige oder verzweigte Alkandiylgruppe mit 3 bis 10 C-Atomen, wobei
(i) gegebenenfalls eine oder mehrere nicht terminale CH₂-Gruppen durch -O- und/oder -S- ersetzt sind und/oder
(ii) gegebenenfalls ein oder mehrere H-Atome durch F und/oder Cl ersetzt sind und/oder
(iii) gegebenenfalls eine nicht terminale -CH₂CH₂- Gruppe durch -NHCO-ersetzt ist;
- Y³: ist gleich -C(O)-, ~C(O)-NH- oder ~NH-C(O)-, wobei ∼ die Bindung zu Gruppe A³ bezeichnet;
- A³: ist
a) ein C₁-C₈-Alkantriyl, wobei gegebenenfalls mehrere nicht terminale CH₂-Gruppen durch O, S, S(O), S(O)₂, NR^{x} und/oder C(O) ersetzt sind und wobei in den genannten Gruppen gegebenenfalls ein oder mehrere H-Atome durch eine Gruppe X² ersetzt sind,
b) ist ein gesättigter, partiell ungesättigter oder aromatischer, mono- oder polycyclischer Kohlenwasserstoffrest mit 3 bis 14 C-Atomen oder ein dreibis achtgliedriger, aromatischer, partiell ungesättigter oder gesättigter mono oder polycyclischer heterocyclischer Rest, wobei die genannten Gruppen jeweils gegebenenfalls durch eine oder mehrere Gruppen X² substituiert sind,
c) ein tertiärer Stickstoff;
- X²: ist gleich Fluor, Chlor, Alkyl, Halogenalkyl oder Alkyloxy wobei die Alkylgruppen in diesen Resten 1 bis 2 Kohlenstoffatome enthalten;
- W: ist -Y⁵-T²-V oder -V;
- V: ist ∼C(O)O-4-Nitrophenyl, ∼C(O)O-Pentafluorphenyl, Malein-2-yl-säureanhydrid, ∼C(O)-1-Azetidin-2-on, ∼4-O-Phenyl-C(O)-1-Azetidin-2-on, ∼N=C=O, ∼N=S=O, ∼C(O)N₂, ∼S(O)₂Cl, ∼C(NH₂)OR^{y}, ∼P(CH₂OH)₃, ∼SH, ∼SC(O)CH₃, ∼NH₂, ∼OH, ∼CH=CH₂, ∼C=CH, ∼COOM, ∼C(O)H, ∼C(O)CH₃, ∼C(O)C(O)H, ∼I, ∼N₃, Ethyl-2-(3-Indol)amin-1∼, ∼S(O)₂N₃, Phenyl-CH=CH-C(N₂)-C(O)O∼, ∼CH=CHCH₂OC(O)R^{y}, ∼CH=CHCH₂OC(O)NHR^{y}, ∼OC(O)OR^{y}, ∼C(O)NHNH₂, ∼N-Maleinimid, Aziridin-2∼, Pyridin-2-S-S∼, Phenyl-1-Carboxy-2-Nitro-5-S-S∼, ∼S(O)₂CH=CH₂, ∼C(O)-S-Phenyl, ∼C(O)CH=N₂, ∼C(O)O-N-Succinimidyl, oder C(O)O-N-Sulfosuccinimidyl;
- Y⁵: ist eine Bindung, -O-, -S-, -NR^{x}-, -C(O)-, ∼C(O)-NR^{x}- oder ∼NR^{x}-C(O)-, wobei ∼ die Bindung zu Gruppe A³ bezeichnet;
- T²: ist eine geradkettige oder verzweigte Alkandiylgruppe mit 1 bis 200 C-Atomen, wobei
(i) gegebenenfalls eine oder mehrere nicht terminale CH₂-Gruppen durch -O-, NH und/oder -S- ersetzt sind und/oder
(ii) gegebenenfalls ein oder mehrere nicht terminale H-Atome durch F, Cl, (=O), NR^{z} und/oder NR^{y}, ersetzt sind und/oder
(iii) gegebenenfalls eine oder mehrere nicht terminale -CH₂CH₂- Gruppe durch-NHCO- oder -S-S- ersetzt ist;
- R¹: ist gleich C(O)OM;
- R², R³: sind gleich H;
- R⁴, R⁶, R⁷: sind gleich oder verschieden OH oder OR^{z};
- R⁵: ist gleich C(O)CH₃ oder C(O)CH₂OH;
- M: ist gleich ein C₁-C₄-Alkyl, C₃-C₄-Cycloalkyl oder ein Kation;
- R^{x}: ist gleich oder verschieden H, R^{y} oder R^{z};
- R^{y}: ist gleich oder verschieden C₁-C₄-Alkyl, C₃-C₄-Cycloalkyl, Phenyl oder Benzyl und
- R^{z}: ist gleich oder verschieden -C(O)-C₁-C₄-Alkyl, -C(O)-C₃-C₄-Cycloalkyl, -C(O)-Phenyl oder C(O)-CH₂-Phenyl.

Gegenstand der Erfindung ist auch die Verwendung einer Verbindung der Formel
(I) in einem Verfahren zur Verknüpfung mit einem Cargo. Beispielsweise werden niedermolekulare Wirkstoffe (niedermolekulare pharmakologisch wirksame Substanzen), radioaktiv markierte Stoffe, Zytostatika, RNA, DNA, Proteine oder ein anderes therapeutisch anwendbares Cargo verknüpft. Bevorzugt sind eine niedermolekulare, pharmakologisch wirksame Substanz, ein Zytostatikum, ein Protein, ein antigenes Protein, ein Enzym, ein Antigen, ein niedermolekulares Antigen, eine DNA, eine RNA, ein Oligonucleotid, eine radioaktiver Stoff, eine metallorganischer Komplex oder ein Peptid. Beispielsweise können die Konjugate zur Verwendung in einem Verfahren zur Regulation des Immunsystems, beispielsweise bei Impfungen oder Transplantationen sowie zur Behandlung von Krankheiten, insbesondere Allergien, Autoimmunerkrankungen, chronischen Entzündungen, Querschnittslähmung, multipler Sklerose, Viruserkrankungen, beispielsweise AIDS, bakterielle Erkrankungen, parasitäre Erkrankungen, Krankheiten, bei denen die Immunantwort im Rahmen der B-Zellenaktivierung gestört ist, wie Common Variable Immunodeficiency (CVID) und lgA-Defizienz, bei Krankheiten der blutbildenden Organe und des Blutes sowie bei Krebs, beispielsweise Lymphome und Myelome genutzt werden.

Ein weiterer Gegenstand der Erfindung ist auch eine Verbindung der Formel (I) zur Verknüpfung mit einem Cargo, bevorzugt einem Cargo ausgewählt aus der Gruppe bestehend aus RNA, DNA, Peptiden, niedermolekularen Antigenen, antigenen Proteinen, Enzymen und niedermolekularen pharmakologisch wirksamen Substanzen, zur Verwendung in einem Verfahren zur Regulierung von Stoffwechselvorgängen, Immunreaktionen, Immunisierungen oder Desensibilisierungen des Zielorganismus.

Ein weiterer Gegenstand der Erfindung ist ein pharmakologisch wirksamen Konjugates einer erfindungsgemäßen Verbindung der Formel (I) und eines Cargo, vorzugsweise ausgewählt aus der Gruppe bestehend aus RNA, DNA, Peptiden, Zytostatika, Enzymen, metallorganischen Komplexen und niedermolekularen pharmakologisch wirksamen Substanzen, zur Verwendung in einem Verfahren zur Behandlung von Infektionen, Tumorerkrankungen oder Allergien.

Ein weiterer Gegenstand der Erfindung ist auch eine Verbindung der Formel (I) zur Verknüpfung mit einem Cargo, vorzugsweise ausgewählt aus der Gruppe bestehend aus RNA, DNA, Peptiden, Enzymen, metallorganischen Komplexen und niedermolekularen pharmakologisch wirksamen Substanzen, zur Verwendung in einem Verfahren zur Behandlung von Allergien und Immunreaktionen.

Ein weiterer Gegenstand der Erfindung ist ein pharmakologisch wirksamen Konjugates einer Verbindung der Formel (I) und eines Cargo, vorzugsweise ausgewählt aus der Gruppe bestehend aus RNA, DNA, Peptiden, Enzymen, metallorganischen Komplexen und niedermolekularen pharmakologisch wirksamen Substanzen, zur Verwendung zur Verwendung einem Verfahren zur Behandlung von Allergien und Immunreaktionen.

Ebenfalls Gegenstand der Erfindung ist auch die Verwendung einer Verbindung der Formel (I) in einem Verfahren zur Verknüpfung mit Liposomen, Nanopartikeln, metallorganischen Komplexen, Metallnanopartikeln wie zB. Goldanopartikeln, Micro-Miscelles, Kohlenstoffnanoröhrchen und anderen Transportvehikeln. Das Vehikel kann pharmakologisch wirksame Substanzen, Proteine, Antigene oder Impfstoffe sowie Impf-Adjuvantien beinhalten.

Ebenfalls Gegenstand der Erfindung ist auch die Verwendung einer Verbindung der Formel (I) in einem Verfahren zur Herstellung von Liposomen, Nanopartikeln, Micro-Miscelles, Kohlenstoffnanoröhrchen und anderen Transportvehikeln.

Ebenfalls Gegenstand der Erfindung ist auch die Verwendung einer Verbindung der Formel (I) in einem Verfahren zur Verknüpfung mit Molekülen zu diagnostischen Zwecken. Beispielsweise kann eine Verbindung der Formel (I) mit einem fluoreszentem Molekül, einem Positronenemitter oder einem anderem diagnostisch nutzbarem Cargo verknüpft werden.

Ebenfalls Gegenstand der Erfindung ist auch die Verwendung einer Verbindung der Formel (I) in einem Verfahren zur Herstellung von polyvalenten Liganden. Durch Verknüpfung mit einem polyvalenten Trägermolekül können wirksame Substanzen mit verbesserten Eigenschaften hergestellt werden. Diese Substanzen können zur Verwendung in einem Verfahren zur Regulation des Immunsystems, beispielsweise bei Impfungen sowie zur Behandlung von Krankheiten, deren Verlauf oder Aktivität durch die Siglec-Inhibitoren positiv beeinflusst werden kann, insbesondere Allergien, Autoimmunerkrankungen, chronischen Entzündungen, Querschnittslähmung, multipler Sklerose, Krebs, Viruserkrankungen, beispielsweise AIDS, Bakterielle Erkrankungen, bespielsweise Streptokokken, parasitären Erkrankungen, beispielsweise Chagas Krankheit, Krankheiten, bei denen die Immunantwort im Rahmen der B-Zellenaktivierung gestört ist, wie Common Variable Immunodeficiency (CVID) und lgA-Defizienz, bei Krankheiten der blutbildenden Organe und des Blutes sowie bei Krebs, beispielsweise Lymphome und Myelome genutzt werden.

Weiterhin Gegenstand der Erfindung ist die Verwendung einer erfindungsgemäßen Sialinsäurederviates der Formel (I) zur Verknüpfung mit einem Cargo tragenden Polymer, wobei das Cargo bevorzugt ausgewählt ist aus der Gruppe bestehend aus RNA, DNA, Zytostatika, Peptiden, niedermolekularen Antigenen, antigenen Proteinen, Enzymen, metallorganischen Komplexen und niedermolekularen pharmakologisch wirksamen Substanzen,
"Cargo" bezeichnet im Sinne der Erfindung eine niedermolekulare, pharmakologisch wirksame Substanz, ein Zytostatikum, ein Protein, ein antigenes Protein, ein Enzym, ein Antigen, ein niedermolekulares Antigen, eine DNA, eine RNA, ein Oligonucleotid, einen radioaktiven Stoff, einen metallorganischen Komplex oder ein Peptid, bevorzugt eine niedermolekulare, pharmakologisch wirksame Substanz, ein Protein, ein Antigen, ein Oligonucleotid, einen radioaktiven Stoff oder ein Peptid, wobei dieses an die Gruppe W der Verbindung der Formel (I) gebunden ist.

Die Verbindungen der Formel (I) weisen überraschend hohe Affinität zu CD22 auf. Sie enthalten außer Sialinsäure keine weiteren Kohlenhydrate. Sie enthalten eine reaktive Gruppe, welche einfach zu variieren ist und so die Konjugation mit einer Vielzahl von Cargo-Molekülen ermöglicht. Über die reaktive Gruppe können ebenfalls weitere neue reaktive Gruppen zur Verknüpfung mit einem Cargo eingeführt werden. Sie beinhalten gegebenenfalls eine spaltbare Gruppe. Im Gegensatz zu Antikörpern ist eine chemische Herstellung ohne den Einsatz von Zellkulturen möglich. Die Sialinsäurederivate der Formel (I) können je nach Cargo über spaltbare oder unspaltbare Linker verknüpft werden. Die Liganden können mit dem Cargo so verknüpft werden, dass die Aktivität des Cargo nicht beeinträchtigt wird oder erst in der Zielzelle wieder hergestellt wird. Es können einheitliche und in ihrer Struktur definierbare Konjugate der Verbindungen der Formel (I) hergestellt werden.

Der Begriff , Verbindung der Formel (I)" umfasst alle stereoisomeren Formen der Verbindung der Formel (I), insbesondere E/Z oder Cis/trans-Isomere bei substituierten Doppelbindungen oder Ringen und sowie Stereoisomere, die sich durch die Chiralitätszentren der Verbindungen der Formel (I) ergeben, insbesondere Enantiomere und Diastereoisomere in reiner Form oder in Form von Gemischen jeglicher Zusammensetzung, wobei die einzelnen Chiralitätszentren jeweils in der (S)- oder (R)-Form vorliegen.

Die Herstellung der einzelnen Stereoisomeren kann beispielsweise durch Aufbesserung der Isomerengemische nach üblichen Methoden, wie Chromatographie oder Kristallisation, oder durch Verwendung von isomeren reinen Ausgangsstoffen erfolgen. Die Aufbesserung der Isomere kann auf Stufe der Edukte, Zwischenprodukte oder Endprodukte der Formel (I) erfolgen. Zu den erfindungsgemäß umfassten Isomeren zählen auch alle tautomeren Formen von Verbindungen der Formel (I) und alle mesomorphen Formen.

Im Allgemeinen kommen die Salze derjenigen Kationen oder die Säureadditionssalze derjenigen Säuren in Betracht, deren Kationen, beziehungsweise Anionen, die Verwendung zur Herstellung von Konjugaten mit den Verbindungen (I) nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch R^{Y} ersetzt sein können, vorzugsweise Ammonium, Dimethylammonium, Diisopropylammonium, Tetramethylammonium, Triethylammonium, Tetrabutylammonium, 2-(2-Hydroxyeth-1-oxy)eth-1-yl-ammonium, Di-(2-hydroxyeth-l-yl)-ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)-sulfoxonium, in Betracht. Bevorzugt sind Na, Li, K, Ca, Mg und Ammonium (gegebenenfalls substituiert), besonders bevorzugt sind Na, Li und K, insbesondere bevorzugt ist Na.

Anionen von Säureadditionsalzen sind beispielsweise Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat, die Anionen von C₁-C₄-Alkansäuren, vorzugsweise Formiat, Acetat, Propionat und Butyrat, und anderen organischen Säuren, wie Pivalinsäure, Maleinsäure, Bernsteinsäure, Pimelinsäue, Fumarsäure, Apfelsäure, Sulfaminsäure, Phenylpropionsäure, Gluconsäure, Ascorbinsäure, Nicotinsäure, Zitronensäure und Adipinsäure.

Weiterhin umfasst der Begriff, Verbindung der Formel (I)" Solvate, beispielsweise Hydrate oder Addukte mit Alkoholen, sowie alle Kristallmodifikationen.

Soweit nicht anders angegeben können Symbole, die mehrfach verwendet werden, unabhängig voneinander gleiche oder verschiedene Bedeutungen haben.

Bei den in der Formel (I) angegebenen Definitionen der Symbole bedeuten:
Halogen: Fluor, Chlor;
Alkyl: gesättigte, geradkettige oder verzweigte Kohlenwasserstoffreste mit beispielsweise 1 bis 4 Kohlenstoffatomen, wie Methyl, Ethyl, Propyl, 1-Methylethyl, Butyl, 1-Methylpropyl, 2-Methylpropyl oder 1,1-Dimethylethyl;
Cycloalkyl: gesättigte cyclische Kohlenwasserstoffe mit 3 oder 4 Kohlenstoffatomen, wie Cyclopropyl, Cyclobutyl und 1-Methylcyclopropyl;
Halogenalkyl: eine Alkylgruppe, bevorzugt Methylgruppe, wobei in dieser Gruppe Wasserstoffatome teilweise oder vollständig durch Halogenatome ersetzt sind: wie Chlormethyl, Fluormethyl oder Trifluormethyl;
Alkyloxy: Alkyloxygruppen mit gesättigtem Alkylrest, wobei dieser aus der vorstehend genannten Gruppe der Alkyle ist und 1 bis 2 Kohlenstoffatome enthält;
Halogenalkyloxy: Halogenalkyloxygruppen mit Halogenalkylrest, wobei dieser aus der vorstehend genannten Gruppe der Halogenalkyle ist;
Alkylcarbonyloxy: Alkylcarbonyloxygruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylamino: Dialkylaminogruppen mit gesättigtem, geradkettigem, verzweigtem oder cyclischem Alkylresten, wobei dieser aus der vorstehend genannten Gruppe der Alkyle ist und 1 bis 4 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkantriyl bedeutet für Rest A³: gesättigte, geradkettige Alkantriylgruppe mit beispielsweise 1 bis 6 Kohlenstoffatomen, wie Methan-1,1,1-triyl, Ethan-1,1,2-triyl, Propan-1,2,3-triyl, Propan-1,1,3-triyl, Butan-1,2,4-triyl, Butan-1,1,4-triyl, Pentan-1,1,5-triyl, Pentan-1,2,5-triyl, Pentan-1,3,5-triyl, Hexan-1,1,6-triyl, Hexan-1,2,6-triyl oder Hexan-1,3,6-triyl;
Alkandiyl bedeutet für Rest T¹: gesättigte, geradkettige oder verzweigte Alkandiylgruppe mit beispielsweise 3 bis 10 Kohlenstoffatomen, wie Propan-1,3-diyl, Butan-1,4-diyl, Pentan-1,5-diyl, Hexan-1,6-diyl, Heptan-1,7-diyl, Octan-1,8-diyl, Nonan-1,9-diyl oder Decan-1,10-diyl;
Alkandiyl bedeutet für Rest T²: gesättigte, geradkettige Alkandiylgruppe mit beispielsweise 1 bis 200 Kohlenstoffatomen, wie Methan-1,1-diyl, Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl;
Mono- oder polycyclischer, aromatischer, partiell ungesättiger oder gesättiger C₃-C₁₄-Kohlenwasserstoffrest bedeutet für A³ beispielsweise:
   - a): gleich oder verschieden C₆-C₁₄-Aryltriyl, insbesondere Phenylen-1,2,4-triyl, Phenylen-1,3,5-triyl und Naphthalin-1,2,4-triyl;
   - b): C₃-C₈-Cycloalkyltriyl, beispielseise Cyclopropan-1,2,3-triyl, Cyclohexan-1,2,4-triyl und Cyclohexan-1,3,5-triyl;
Drei- bis achtgliedriger gesättigter, partiell ungesättigter oder aromatischer heterocyclischer Rest bedeutet für A³ beispielsweise:
   - a): 5-gliedriges Heteroaryltriyl, enthaltend ein bis vier Stickstoffatome oder ein bis drei Stickstoffatome und/oder ein Schwefel- oder Sauerstoffatom, insbesondere Thiophen-2,3,5-triyl und 1*H*(1,2,3)Triazol-1,4,5-triyl;
Vorteilhaft haben die Symbole in der Formel (I) folgende Bedeutungen:
   - A¹: ist vorteilhaft gleich eine Gruppe 4-Biphenyl, 1-Naphtyl und 2-Naphtyl wobei die genannten Gruppen unsubstituiert oder durch eine oder mehrere Gruppen X¹ substuiert sind.
   - X¹: ist vorteilhaft gleich oder verschieden Fluor, Chlor, Hydroxy, Alkyl, Halogenalkyl, Alkyloxy, Halogenalkyloxy, Alkylcarbonyloxy, wobei die Alkylgruppen in diesen Resten 1 bis 2 Kohlenstoffatome enthalten.
   - Y¹: ist vorteilhaft gleich ∼C(O)- oder ∼CH₂C(O)-, wobei ∼ die Bindung zur Gruppe A¹ bezeichnet.
   - Z²: ist vorteilhaft gleich -O-.
   - T¹: ist vorteilhaft gleich eine geradkettige oder verzweigte Alkandiylgruppe mit 4 bis 8 C-Atomen, wobei
   (i) gegebenenfalls eine oder mehrere nicht terminale CH₂-Gruppen durch -O- und/oder -S- ersetzt sind und/oder
   (ii)gegebenenfalls eine nicht terminale -CH₂CH₂- Gruppe durch -NHCO- ersetzt ist.
   - Y³: ist vorteilhaft gleich eine Bindung, ∼C(O)-NH- oder ∼NH-C(O)-, wobei ∼ die Bindung zu Gruppe A³ bezeichnet.
   - A³: ist vorteilhaft ein
   a) C₁-C₅Alkantriyl, wobei gegebenenfalls eine oder mehrere nicht terminale CH₂-Gruppen durch O, S, NR^{x} und/oder C(O) ersetzt sind und wobei in den genannten Gruppen gegebenenfalls ein oder mehrere H-Atome durch eine Gruppe X² ersetzt sind,
   b) Phenylen-1,2,4-triyl oder 1*H*(1,2,3)Triazol-1,4,5-triyl, wobei die genannten Gruppen jeweils gegebenenfalls durch eine oder mehrere Gruppen X² substituiert sind,
   c) tertiärer Stickstoff.
   - X²: ist vorteilhaft gleich Fluor, Chlor, Methyl, Methyloxy.
   - W: ist vorteilhaft -Y⁵T²V oder -V.
   - V: ist vorteilhaft ist ∼C(O)O-4-Nitrophenyl, ∼C(O)O-Pentafluorphenyl, Malein-2-yl-säureanhydrid, ∼N=C=O, ∼N=S=O, ∼C(O)N₂, ∼P(CH₂OH)₃, ∼SH, ∼NH₂, ∼OH, ∼CH=CH₂, ∼C≡H, ∼COOM, ∼C(O)H, ∼C(O)CH₃, ∼C(O)C(O)H, ∼I, ∼N₃, ∼C(O)NHNH₂, ∼N-Maleinimid, Aziridin-2∼, Pyridin-2-S-S∼, Phenyl-1-Carboxy-2-Nitro-5-S-S∼, ∼C(O)O-N-Succinimidyl.
   - Y⁵: ist vorteilhaft eine Bindung, -O-, -NH-, -C(O)-, ∼C(O)-NH- oder ~NH-C(O)-, wobei ∼ die Bindung zu Gruppe A³ bezeichnet.
   - T²: ist vorteilhaft eine geradkettige oder verzweigte Alkandiylgruppe mit 1 bis 200 C-Atomen, wobei
   (i) gegebenenfalls eine oder mehrere nicht terminale CH₂-Gruppen durch -O-oder -NH- ersetzt sind und/oder
   (ii) gegebenenfalls ein oder mehrere nicht terminale H-Atome durch F oder (=O) ersetzt sind und/oder
   (iii) gegebenenfalls eine oder mehrere nicht terminale -CH₂CH₂- Gruppe durch-NHCO- oder -S-S- ersetzt ist.
   - R¹: ist vorteilhaft gleich C(O)OM.
   - R², R³: sind vorteilhaft gleich H.
   - R⁴, R⁶, R⁷: sind vorteilhaft gleich oder verschieden OH oder OR^{z}.
   - R⁵: ist vorteilhaft gleich C(O)CH₃.
   - M: ist vorteilhaft gleich ein C₁-C₂-Alkyl oder ein Kation.
   - R^{x}: ist vorteilhaft gleich oder verschieden H, R^{y} oder R^{z}.
   - R^{y}: ist vorteilhaft gleich oder verschieden C₁-C₃-Alkyl, Cyclopropyl, Phenyl oder Benzyl.
   - R^{z}: ist vorteilhaft gleich oder verschieden -C(O)-C₁-C₄-Alkyl, -C(O)-Phenyl oder-C(O)-CH₂-Phenyl.

Vorteilhaft bedeuten die in der Formel (I) angegebenen Definitionen der Symbole:
Halogen: Fluor, Chlor;
Alkyl: gesättigte, geradkettige oder verzweigte Kohlenwasserstoffreste mit beispielsweise 1 bis 3 Kohlenstoffatomen, wie Methyl, Ethyl, Propyl und 1-Methylethyl;
Cycloalkyl: Cyclopropyl;
Halogenalkyl: Chlormethyl, Fluormethyl und Trifluormethyl;
Alkyloxy: Alkyloxygruppen mit gesättigtem Alkylrest, wobei dieser aus der vorstehend genannten Gruppe der Alkyle ist und 1 bis 2 Kohlenstoffatome enthält;
Halogenalkyloxy: Halogenalkyloxygruppen mit Halogenalkylrest, wobei dieser aus der vorstehend genannten Gruppe der Halogenalkyle ist;
Alkylcarbonyloxy: Alkylcarbonyloxygruppen mit gesättigtem, geradkettigem oder verzweigtem Alkylrest, wobei dieser aus der vorstehend genannten Gruppe der Alkyle ist und 1 bis 3 Kohlenstoffatome enthält;
Alkantriyl bedeutet für Rest A³: gesättigte, geradkettige Alkantriylgruppe mit beispielsweise 1 bis 5 Kohlenstoffatomen, wie Methan-1,1,1-triyl, Ethan-1,1,2-triyl, Propan-1,2,3-triyl, Propan-1,1,3-triyl, Butan-1,2,4-triyl, Butan-1,1,4-triyl, Pentan-1,1,5-triyl, Pentan-1,2,5-triyl oder Pentan-1,3,5-triyl;
Alkandiyl bedeutet für Rest T¹: gesättigte, geradkettige oder verzweigte Alkandiylgruppe mit beispielsweise 4 bis 8 Kohlenstoffatomen, wie Butan-1,4-diyl, Pentan-1,5-diyl, Hexan-1,6-diyl, Heptan-1,7-diyl oder Octan-1,8-diyl;
Alkandiyl bedeutet für Rest T²: gesättigte, geradkettige Alkandiylgruppe mit beispielsweise 1 bis 200 Kohlenstoffatomen, wie Methan-1,1-diyl, Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl;

Vorteilhaft sind Verbindungen der Formel (I), bei denen alle Symbole, Definitionen und Indizes die vorteilhaften Bedeutungen haben.

Bevorzugt haben die Symbole in der Formel (I) folgende Bedeutungen:
- A¹: ist bevorzugt gleich 4-Biphenyl, wobei die genannte Gruppe unsubstituiert oder durch eine oder mehrere Hydroxylgruppen substuiert ist.
- Y¹: ist bevorzugt gleich ∼C(O)-, wobei ∼ die Bindung zur Gruppe A¹ bezeichnet.
- Z²: ist bevorzugt gleich -O-.
- T¹: ist bevorzugt gleich Hexan-1,6-diyl, wobei gegebenenfalls eine nicht terminale CH₂-Gruppen durch -S- ersetzt ist.
- Y³: ist bevorzugt gleich ∼C(O)-NH-, wobei ∼ die Bindung zu Gruppe A³ bezeichnet.
- A³: ist bevorzugt Propan-1,1,3-triyl oder Phenyl-1,2,4-triyl.
- W: ist bevorzugt -Y⁵-T²-V oder -V.
- V: ist vorteilhaft ist ∼SH, ∼NH₂, ∼CH=CH₂, ∼C≡CH, ∼COOM, ∼I, ∼N₃, ∼C(O)NHNH₂, ∼N-Maleinimid, ∼C(O)O-N-Succinimidyl.
- Y⁵: ist bevorzugt eine Bindung, -O- oder -NHCO-, wobei ∼ die Bindung zur Gruppe A³ bezeichnet.
- T²: ist bevorzugt eine geradkettige oder verzweigte Alkandiylgruppe mit 1 bis 200 C-Atomen, wobei
(i) gegebenenfalls eine oder mehrere nicht terminale CH₂-Gruppen durch ∼O-ersetzt sind und/oder
(ii) gegebenenfals eine oder mehrere nicht terminale -CH₂CH₂-Gruppen durch -NHCO- oder -S-S- ersetzt sind.
- R¹: ist bevorzugt gleich C(O)OM.
- R², R³: sind bevorzugt gleich H.
- R⁴, R⁶, R⁷: sind bevorzugt gleich OH oder OC(O)CH₃.
- R⁵: ist bevorzugt gleich C(O)CH₃.
- M: ist bevorzugt CH₃ oder Natrium.

Bevorzugt bedeuten die in der Formel (I) angegebenen Definitionen der Symbole:
Alkandiyl bedeutet für Rest T² bevorzugt: gesättigte, geradkettige Alkandiylgruppe mit beispielsweise 1 bis 200 Kohlenstoffatomen, wie Methan-1,1-diyl, Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl.

Bevorzugt sind Verbindungen der Formel (I), bei denen alle Symbole, Definitionen und Indizes die bevorzugten Bedeutungen haben.

Weiterhin bevorzugte Verbindungen der Formel (I) sind solche der Formeln (Ia) - (Ib), wobei die Symbole die in der Formel (I) angegebenen Bedeutungen und Bevorzugungen haben.

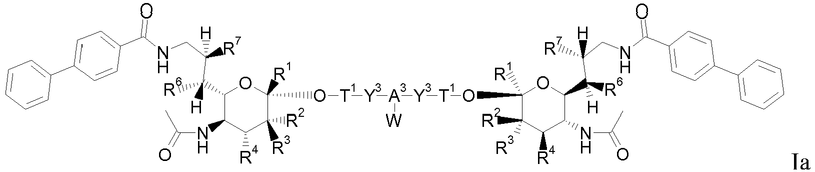

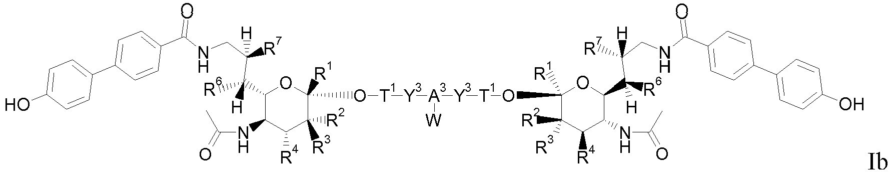

Besonders bevorzugt sind weiterhin Verbindungen der Formeln (Iaa)-(Iba), wobei die Symbole die in der Formel (I) angegebenen Bedeutungen haben:

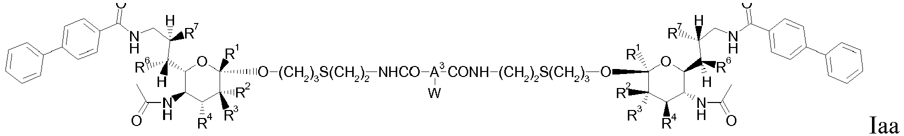

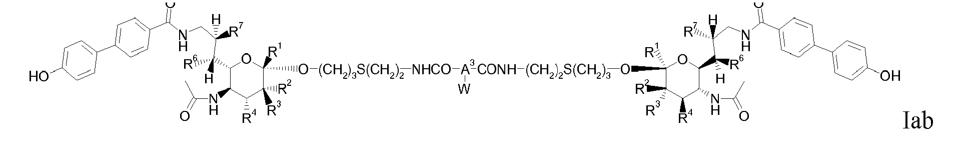

Ebenso besonders bevorzugt sind Verbindungen der Formeln (Iac)-(Iaf), wobei die Symbole die in der Formel (I) angegebenen Bedeutungen haben:

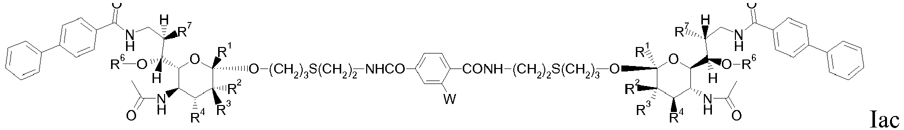

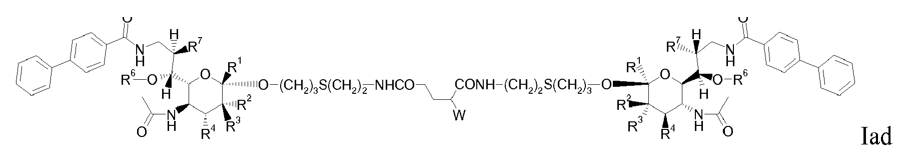

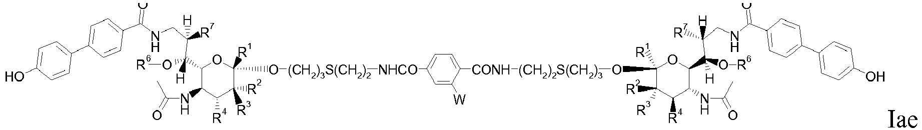

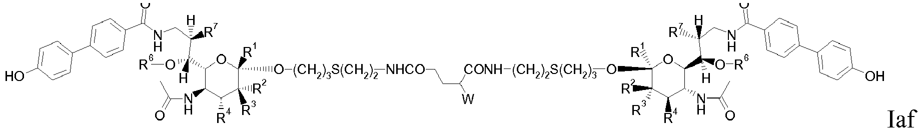

Die erfindungsgemäßen Verbindungen (I) sind unter Anwendung im Prinzip bekannter Syntheseverfahren erhältlich. Bevorzugt werden die Verbindungen nach den nachfolgend näher erläuterten erfindungsgemäßen Herstellungsverfahren, insbesondere den Syntheseschemata I - IX, erhalten: 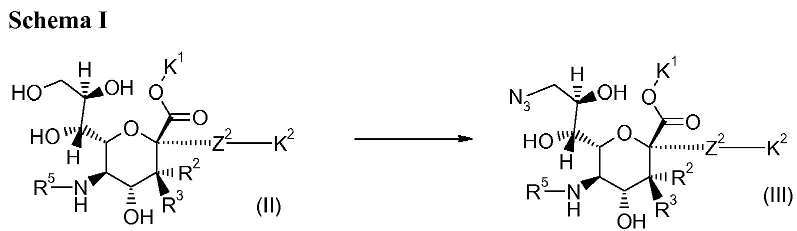
K¹ ist Alkyl, insbesondere C₁-C₃-Alkyl
K² ist Alkenyl, geschütztes Aminoalkyl
Z² ist gleich O, S oder CH₂

Verbindungen der Formel (III) können durch Einführung einer Azidogruppe aus den Verbindungen der Formel (II) hergestellt werden (Schema I). Die Einführung von Azidogruppen ist beispielsweise beschrieben in Angewandte Chemie 2005, 117, 5320-5374 und Chem Rev 1988, 88 (2), 297-368. Beispielhaft wurde Verbindung 6 hergestellt. 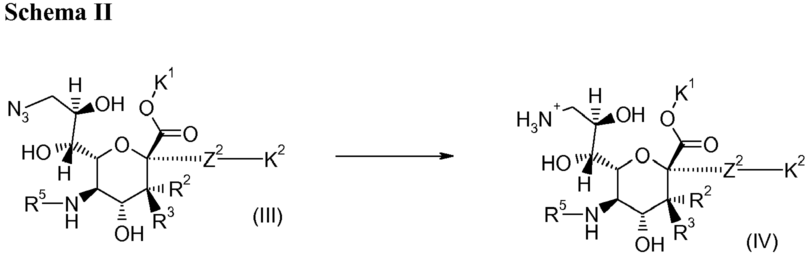
K¹ ist Alkyl, insbesondere C₁-C₃-Alkyl
K² ist z.B.: Alkenyl, geschütztes Aminoalkyl
Z² ist gleich O, S oder CH₂

Verbindungen der Formel (IV) können durch Reduzierung der Azidogruppe in den Verbindungen der Formel (III) hergestellt werden (Schema II). Die Reduktion von Azidogruppen ist beispielsweise beschrieben in Angewandte Chemie 2005, 117, 5320-5374 und Chem Rev 1988, 88 (2), 297-368

Beispielhaft wurde Verbindung 7 hergestellt. 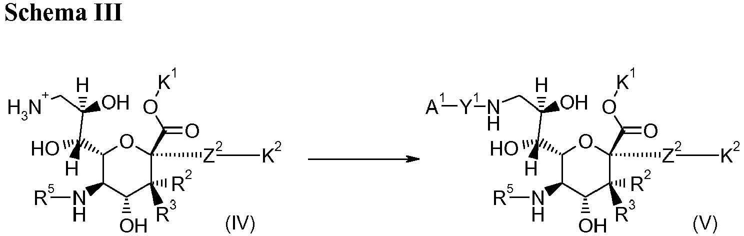
K¹ ist ein Alkyl, insbesondere C₁-C₃-Alkyl
K² ist z.B.: Alkenyl, geschütztes Aminoalkyl
Z² ist gleich O, S oder CH₂

Verbindungen der Formel (V) können durch Bildung einer Amid-Bindung aus dem Amin in Verbindungen der Formel (IV) hergestellt werden (Schema III). Reaktionen von Aminen zu Amiden sind beispielsweise beschrieben in Tetrahedron 2005, 61, 10827-10852. Beispielhaft wurde Verbindung 8 und *30* hergestellt. 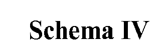 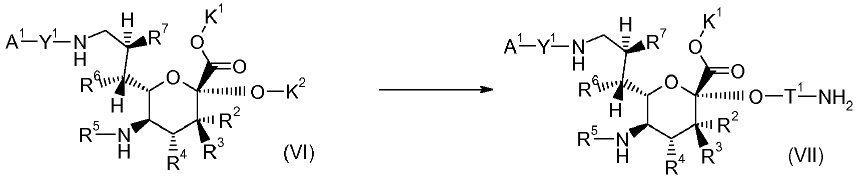
K¹ ist Alkyl, insbesondere C₁-C₃-Alkyl
K² ist ein Alkenyl, geschütztes Aminoalkyl

Verbindungen der Formel (VII) können durch Reaktion einer terminalen Doppelbindung der Verbindungen der Formel (VI) mit einer reaktiven Gruppe hergestellt werden (Schema IV). Reaktionen von Doppelbindung sind beispielsweise beschrieben in J. Org. Chem. 2000, 65, 958-963. Weiter können Verbindungen der Formel (VII) durch Entschützen der Aminogruppe in Verbindungen der Formel (IV) hergestellt werden (Schema VI). Schutzgruppen und Reaktionen zum Entschützen von Aminogruppen sind beispielsweise beschrieben in "Protecting Groups" Philip J. Kocienski, 3rd Edition, Thieme 2005 und Chem. Rev. 2009, 109, 2455-2504.
Beispielhaft wurde Verbindung *9* und 31 hergestellt.

Die Herstellung von dimeren Ausgangsprodukten ist beispielhaft in Schema V beschrieben. 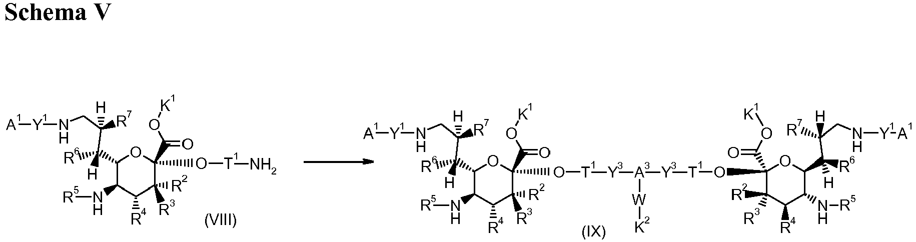
K¹ ist Alkyl, insbesondere Methyl
K² ist gleich eine Schutzgruppe für W

Verbindungen der Formel (IX) können durch Reaktion der Aminogruppe in Verbindungen der Formel (VIII) mit divalenten, reaktiven Verbindungen hergestellt werden (Schema V). Reaktionen von Aminen mit reaktiven Verbindungen sind beispielsweise beschrieben in Tetrahedron 2005, 61, 10827-10852 und Chem. Eur. J. 2009, 15, 9404-9416. Die Reaktion kann auch durch den Einsatz von Kupplungsreagenzien oder durch in situ Aktivierungen einer divalenten Verbindung durchgeführt werden. Kupplungsreagenzien und deren Reaktionen sind beschrieben in Tetrahedron 2005, 61, 10827-10852 und Chem. Eur. J. 2009, 15, 9404-9416.
Beispielhaft wurden Verbindungen *10, 14, 17, 20* und *21* hergestellt.

Die Herstellung von Sialinsäurederivaten der Formel (I) ist beispielhaft in den Schemata VI bis VIII beschrieben. 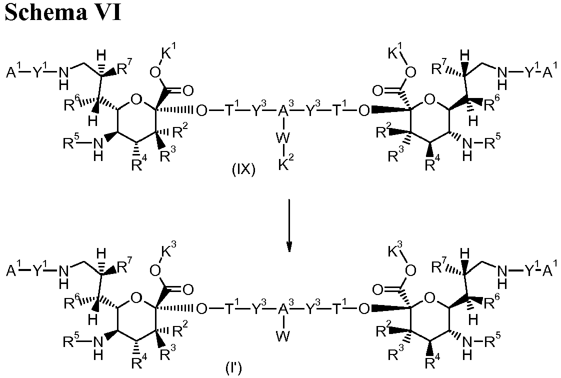
K¹ ist Alkyl, insbesondere Methyl
K² ist eine Schutzgruppe für W
K³ ist K¹ oder M

Verbindungen der Formel (I') können durch Entschützen der Gruppen W in Verbindungen der Formel (IX) hergestellt werden (Schema VI). Schutzgruppen und Reaktionen zum Entschützen sind beispielsweise beschrieben in "Protecting Groups" Philip J. Kocienski, 3rd Edition, Thieme 2005 und Chem. Rev. 2009, 109, 2455-2504.
Beispielhaft wurden Verbindungen *15, 11, 28* und *29* hergestellt. 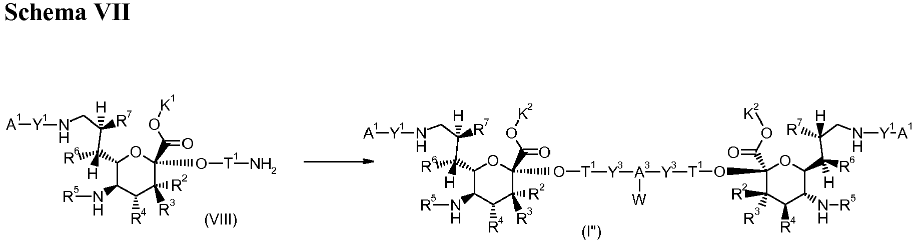
K¹ ist Alkyl, insbesondere Methyl
K² ist K¹ oder M

Verbindungen der Formel (I") können durch Reaktion der Aminogruppe in Verbindungen der Formel (VIII) mit divalenten, reaktiven Verbindungen hergestellt werden (Schema VII). Reaktionen von Aminen mit reaktiven Verbindungen sind beispielsweise beschrieben in Tetrahedron 2005, 61, 10827-10852 und Chem. Eur. J. 2009, 15, 9404-9416. Die Reaktion kann auch durch den Einsatz von Kupplungsreagenzien oder durch in situ Aktivierungen einer divalenten Verbindung durchgeführt werden. Kupplungsreagenzien und deren Reaktionen sind beschrieben in Tetrahedron 2005, 61, 10827-10852 und Chem. Eur. J. 2009, 15, 9404-9416.
Beispielhaft wurden Verbindungen *13, 18* und *32* hergestellt. 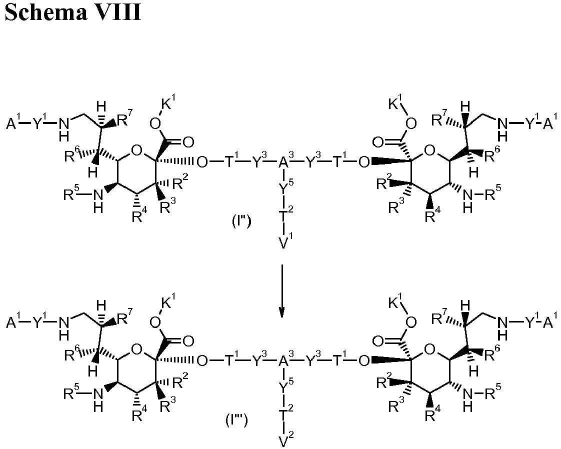
K¹ ist Alkyl oder M
V¹ ist ein eine reaktive Gruppe, insbensondere ein Amin
V² ist eine weitere reaktive Gruppe

Verbindungen der Formel (I'") können durch Reaktionen der Gruppe V¹ in Verbindungen der Formel (I") mit einer Gruppe V² enthaltenden Substanz hergestellt werden (Schema VIII). Die Gruppe V¹ bildet nach der Reaktion einen Teil von T². Die Gruppe V² kann auch während der Reaktion entstehen oder mit einer Schutzgruppe versehen sein, welche im Anschluss entschützt werden kann. Schutzgruppen und Reaktionen zum Entschützen sind beispielsweise beschrieben in "Protecting Groups" Philip J. Kocienski, 3rd Edition, Thieme 2005 und Chem. Rev. 2009, 109, 2455-2504.
Beispielhaft wurden Verbindungen *23, 24, 26, 27, 28* und 41 hergestellt.

Die Herstellung von Konjugaten mit Sialinsäurederivaten der Formel (I) ist beispielhaft in Schema IX beschrieben. 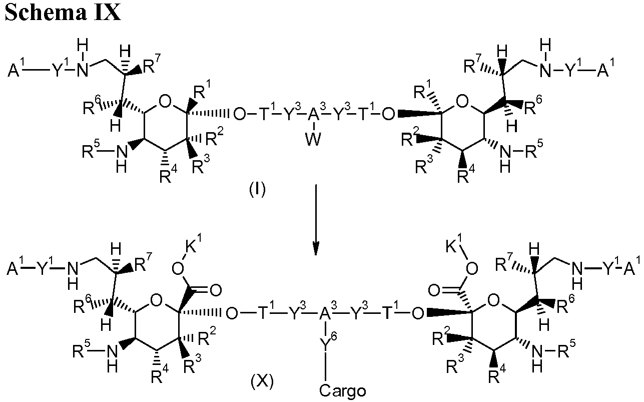 Y⁶ ist der durch die Verknüpfung entstandene Linker

Verbindungen der Formel (X) können durch Reaktion der Gruppe W in Verbindungen der Formel (I) mit einem Cargo hergestellt werden (Schema IX). Dabei kann das Cargo ein oder mehrfach mit einer Verbindung der Formel (I) reagieren.
Beispielhaft wurden Verbindungen *35, 36, 37, 38, 39* und *40* hergestellt.

Die Verbindungen der Formel (I) eignen sich zur Herstellung von pharmakologisch wirksamen Verbindungen und Wirkstoffen für Arzneimittelzubereitungen. Aus Verbindungen der Formel (I) hergestellte pharmakologisch wirksame Verbindungen und Wirkstoffe wirken zur Behandlung von Krankheiten, deren Verlauf oder Aktivität durch Siglec-tragende Zellen beeinflusst werden, insbesondere Allergien, Autoimmunerkrankungen, chronische Entzündungen, Querschnittslähmung, multiple Sklerose, Krebs, Viruserkrankungen, beispielsweise AIDS, sowie bei bakterielle Erkrankungen, bespielsweise Streptokokken, parasitären Erkrankungen, Krankheiten, bei denen die Immunantwort im Rahmen der B-Zellenaktivierung gestört ist, wie Common Variable Immunodeficiency (CVID) und lgA-Defizienz.
Bevorzugte Indikationen sind Allergien, Krebs, Autoimmunerkrankungen und Impfungen.

Behandlung bedeutet im Sinne der Offenbarung eine therapeutische Behandlung, sowohl zur Heilung als auch zur Linderung von Symptomen, wie auch eine vorbeugende Behandlung. Aus Verbindungen der Formel (I) hergestellte pharmakologisch wirksame Verbindungen und Wirkstoffe können in Kombination mit anderen pharmakologisch wirksamen Substanzen, insbesondere solchen, welche die Wirksamkeit der aus Verbindungen der Formel (I) hergestellten pharmakologisch wirksamen Verbindungen und Wirkstoffe verstärken, eingesetzt werden.

Beschrieben wird auch ein Verfahren zur Behandlung einer B-Zell vermittelten Krankheit, insbesondere aus der Gruppe der Allergien, Autoimmunerkrankungen, chronischen Entzündungen, Querschnittslähmung, multipler Sklerose, Krebs, Viruserkrankungen, beispielsweise AIDS, Krankheiten, bei denen die Immunantwort im Rahmen der B-Zellenaktivierung gestört ist, wie Common Variable Immunodeficiency (CVID) und lgA-Defizienz, wobei man einer von der Krankheit betroffenen Person eine vorzugsweise therapeutisch wirksame Menge einer aus einer Verbindung der Formel (I) hergestellten pharmakologisch wirksamen Verbindung verabreicht.

Die Verbindungen der Formel (I) können auch zur Herstellung ein Konjugates für andere als die genannten Zwecke eingesetzt werden, beispielsweise als Diagnostika, in Verfahren zur Bestimmung der Aktivität von Siglec-Liganden, als biochemische Sonden oder als Zwischenprodukte zur Herstellung von weiteren, insbesondere pharmakologisch aktiven, Verbindungen.
Die Erfindung wird durch die Beispiele näher erläutert ohne sie dadurch zu beschränken.

### Beispiele

### A. Synthesebeispiele werden in Schemata 1 bis 6 dargestellt.

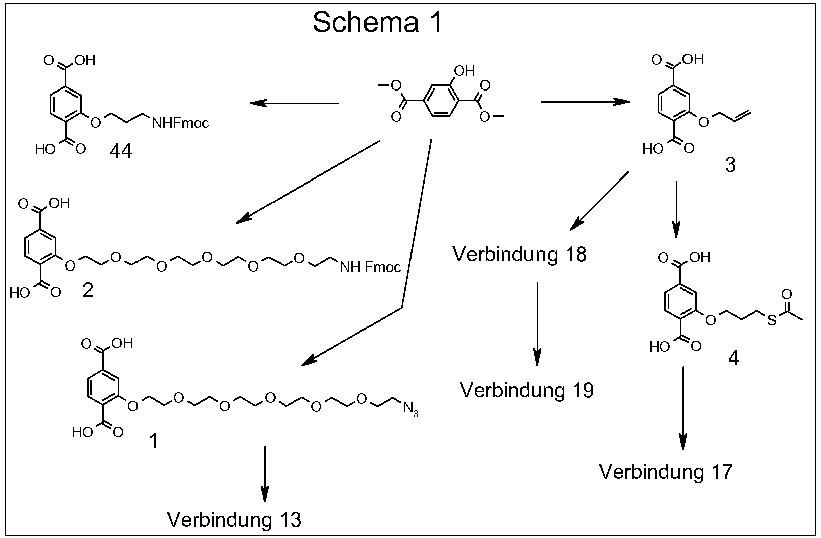

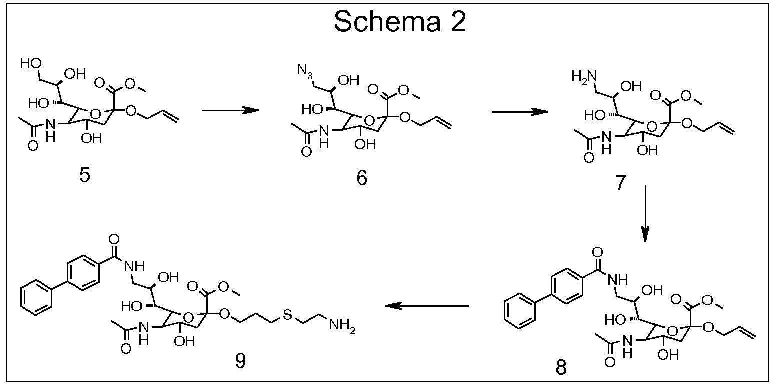

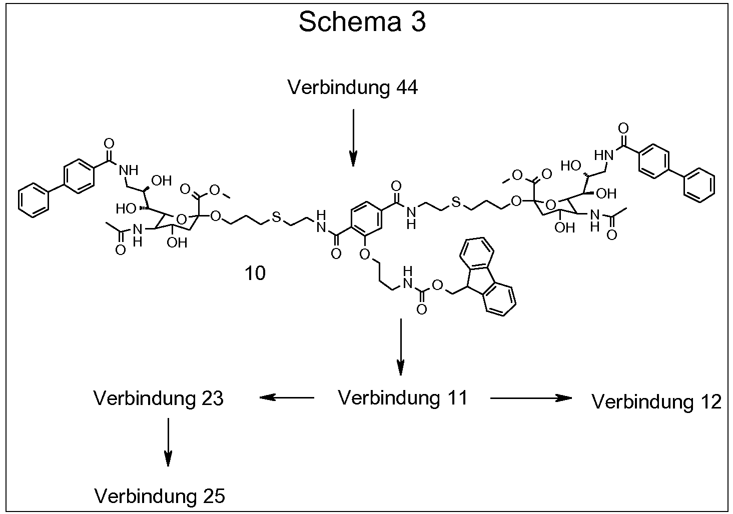

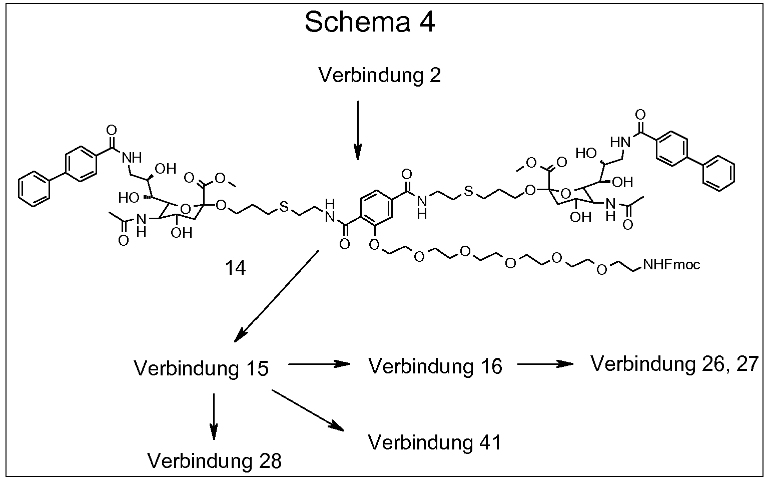

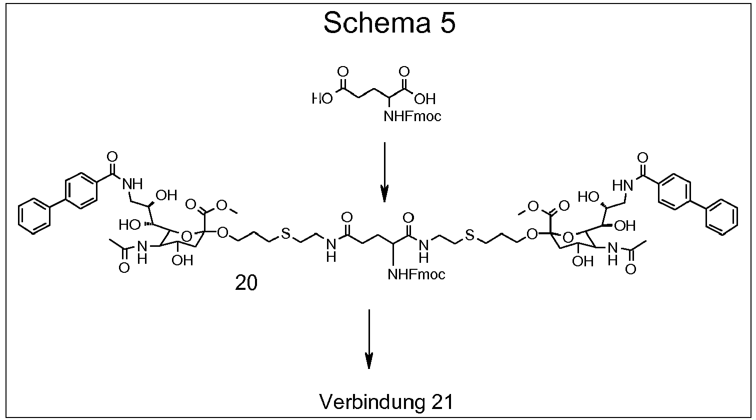

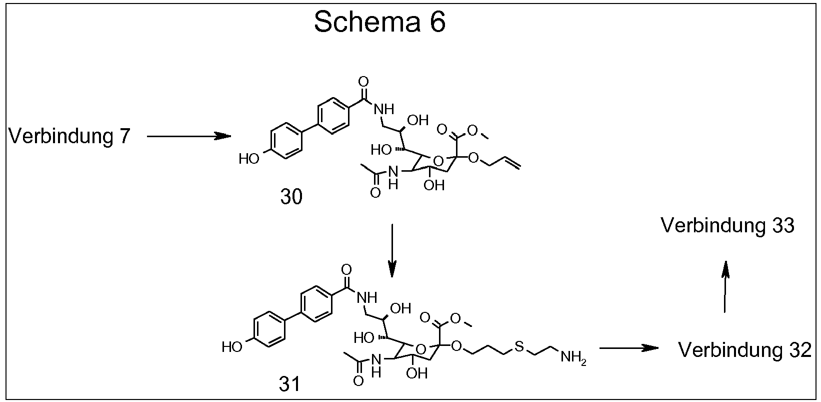

Die Verbindungen wurden wie im Folgenden beschrieben erhalten:
Alle verwendeten aber nicht beschriebenen Verbindungen wurden gekauft oder nach bekannten Literaturvorschriften hergestellt.

Für Reinigungen mit Kieselgel wurde Kieselgel Si60 43-60µm verwendet. Pro Gramm Substanz wurden ca. 100g Kieselgel benutzt.

Für Reinigungen über RP-18-Kieselgel (YMC CO LTD., YMC ODS-AQ) wurde dieses in Ethanol suspendiert, in eine Säule gefüllt, mit Wasser vorgewaschen und die Substanz als Lösung oder Suspension mit Wasser aufgetragen. Das Säulenvolumen betrug ca. 5cm in der Höhe und ca. 1 cm im Durchmesser. Das Lösungsmittel wurde mit geringem Druck, erzeugt durch einen handbetriebenen Druckball, durch die Säule gedrückt. Laufmittel sind in Klammern angegeben. Gradienten sind mit dem Zeichen >> ausgedrückt z.B.: "(H2O » EtOH)" bedeutet, dass ein Gradient von Wasser zu Ethanol benutzt wurde).

Lösungsmittel wurden mittels eines Vakuumrotationsverdampfers bei reduziertem Druck und einer Badtemperatur von 40°C vollständig entfernt. In den folgenden Synthesen ist dieser Arbeitschritt, als "entfernen des Lösungmittels" oder "Konzentration" benannt.

Lyophilisiert wurde, wenn nicht anders erwähnt, aus Wasser oder einer Wasser-Dioxan Mischung.

Photochemische Reaktionen wurden mittels des Photochemical Reactor, Model #RPR-100, The Souther New England Ultraviolet Company, durchgeführt.

Die Reaktionen und Substanzen wurden mit Dünnschichtchromatographie kontrolliert. Hierfür wurden kieselgelbeschichtete Aluminiumplatten mit Fluorescenceindikator verwendet (Merck TLC Silica gel 60 F₂₅₄). Die Substanzdetektion erfolgte unter UV-Licht bei 366 und 254nm. Die Chromatogramme wurden anschließend mit verdünnter Schwefelsäure besprüht und erhitzt, um die Kohlenhydrate nachzuweisen. Amine wurden zum Nachweis mit Ninhydrin-Lösung besprüht und erhitzt, Thiole mit Nitroprussid-Lösung. Einzelheiten und weitere Nachweisverfahren sind in "Anfärbereagenzien für Dünnschicht- und Papierchromatographie" Merck, 1970 erläutert.

Alle Substanzen wurden mit Massenspektrometrie geprüft.

| | |
|---|---|
| Maldi-TOF: | Matrix: 2,5 Dihydroxybenzoesäure Methode: dried droplet |
| | Gerät: Bruker BIFLEX III |
| ESI: | Bruker ApexQe hybrid 9.4 T FT-ICR (ESI) |
| NMR: | Varian 500MHz or 300MHz system |

### Abkürzungen

| | |
|---|---|
| abs. | absolut (wasserfrei) |
| AIBN | Azaisobutyronitril |
| CH3CN | Acetonitril |
| DMAP | Dimethylaminopyridin |
| DMF | N,N-Dimethylformamid |
| DIAD | Diisopropylazodicarboxylat |
| DIPEA | N,N-Diisopropylethylamin |
| EE | Ethylacetat |
| EtOH | Ethanol |
| Fmoc | Fluorenylmethyloxycarbonyl |
| HAc | Essigsäure |
| HATU | O-(7-Azabenzotriazol-1-yl)-N,N,N',N'-tetramethyluronium |
| Fmoc | (9H-Fluoren-9-ylmethoxy)carbonyl |
| MeOH | Methanol |
| RT | Raumtemperatur |
| RF | Rückfluss |
| TEA | Triethylamin |
| Z | Benzyloxycarbonyl |
| » | Gradient |
| eq | Equivalente |

### 2-(18-azido-1,4,7,10,13,16-hexaoxa-octadecyl)-Terephthalsäure (Verbindung 1)

300 mg (1eq) Terephthalsäuredimethylester und 658 mg (1eq) 1-Tosyl-17-azido-3,6,9,12,15-pentaoxa-heptadecyl wurden in 5ml abs. DMF gelöst, mit 1971 mg (10eq) K₂CO₃ versetzt und bei 65°C über Nacht gerührt. Unter Rühren wurde 2 M Citronensäure zugegeben, die saure Lösung mit CH₂Cl₂ ausgeschüttelt, die org. Phase mit MgSO₄ getrocknet, filtriert und eingeengt. Der Rückstand wurde mit Wasser versetzt und MeOH zugegeben bis eine klare Lösung erhalten wurde. Die Lösung wurde durch Zugabe von 2 M NaOH für 2 h bei einem pH 12-13 gehalten, dann wurde mit 2 M HCl angesäuert, mit EE ausgeschüttelt, die org. Phase mit ges. NaCl gewaschen, mit MgSO₄ getrocknet, filtriert, eingeengt und gefriergetrocknet. Ausbeute 450mg

### 2-(18-Fmoc amino-1,4,7,10,13,16-hexaoxa-octadecyl)-Terephthalsäure (Verbindung 2)

240 mg (1eq) Terephthalsäuredimethylester und 386 mg (1,1eq) 17-azido-1-hydroxy-3,6,9,12,15-pentaoxa-Heptadecan wurden in 5 ml abs. DMF gelöst und zweimal mit abs. DMF abgedamft. Nach Zugabe von 449 mg (1,5eq) Triphenylphosphin wurde auf 0 °C gekühlt, 330 µl DIAD (1,5eq) zugegeben und bei RT über Nacht gerührt. Nach Zugabe von 2 ml MeOH wurde eingeengt, in Dichlormethan aufgenommen, mit Wasser gewaschen, getrocknet (MgSO₄), filtriert, eingeengt und über Kieselgel gereinigt (n-Hexan>>EE). Der Rückstand wurde in 5 ml MeOH und 1 ml H₂O gelöst, mit 20 mg Pd auf Kohle (20%) und 0,8 ml HAc(20%) versetzt, 2 h hydriert, filtriert, eingeengt, in H₂O gelöst, mit verdünnter HCl angesäuert (pH 3) und über RP18 gereinigt (H₂O>>EtOH). Das Produkt wurde in 1 ml MeOH und 10 ml H₂O gelöst und mit 0,1 ml NaOH (2M) versetzt. Nach 3 h wurde mit verd. HCl neutralisiert, eingeengt, in 1 ml H₂O und 6 ml Dioxan gelöst und der pH mit TEA auf 8-9 eingestellt. Nach dreimaliger Zugabe von 70 mg Fmoc-NHS wurde eingeengt, der Rückstand über Kieselgel (EE, dann EtOH:H₂O 2:1) und über RP18 (H₂O HCl pH 4»EtOH) gereinigt und lyophilisiert. Ausbeute 98 mg

### 2-Allyloxy-Terephthalsäure (Verbindung 3)

100 mg 2-Hydroxyterephthalsäuredimethylester und 662 mg Allylbromid wurden in 1ml DMF gelöst, und es wurden 264 mg CsCO₃ zugegeben. Die Suspension wurde 3 h bei 60 °C gerührt, mit 5 ml HAc(20%) und 15 ml H₂O versetzt und mit Dichlormethan ausgeschüttelt. Die org. Phase wurde getrocknet (MgSO₄), filtriert und eingeengt. Der Rückstand wurde mit 10 ml Dioxan gelöst, 5 ml H₂O und 1 ml 2 M NaOH zugegeben. Nach 20 h wurde mit verd. HCl auf pH 3 eingestellt und über RP18 gereinigt. Ausbeute 72 mg

### 2-(3-Acetylthiopropyloxy) Terephthalsäure (Verbindung 4)

10 mg Verbindung 3 gelöst in 1 ml MeOH und 1 ml H₂O wurden mit 137 mg Thioessigsäure versetzt und mit UV-Licht 8 h bestrahlt. Die Lösung wurde eingeengt und über RP18 (H₂O>>MeOH) gereinigt. Ausbeute 9 mg

### Verbindung 5

Hergestellt nach "J. Carbohydrate Chemistry, 1987, 6 (1), 161-165

### Verbindung 6

Zu einer Lösung von 4 g Verbindung 5 und 2,16 g (4eq) trockenem Lithiumazid in 40 ml abs. DMF wurden unter Rühren und 8,02 g (2,2eq) Tetrabrommethan gelöst. Die gelbe Lösung wurde bei 0° C mit 3,18 g (1,1eq) Triphenylphosphin versetzt, langsam auf RT erwärmt und 20 h gerührt. Nach Zugabe von 20 ml MeOH wurde 1 h bei Raumtemperatur gerührt, wobei es zu geringer Gasentwicklung kam. Das Lösungsmittel wurde entfernt, der Rückstand mit wenig H₂O versetzt und 3x mit Toluol gewaschen. Anschließend wurde das Produkt mit EE extrahiert, mit MgSO₄ getrocknet, filtriert und konzentriert. Ausbeute: 2,75 g einer leicht gelblichen Substanz.

Alternativ wurde Lithiumazid durch Natriumazid ersetzt, welches sich nicht vollständig löst. Nach Zugabe des Methanols wurde zwei Tage bei RT gerührt.

### Verbindung 7

2,2 g der Verbindung 6 wurden in 100 ml MeOH gelöst, 4,45 g (3eq) Triphenylphosphin und 6 ml H₂O zugegeben und die Suspension 18 h bei Raumtemperatur gerührt. Unter Rühren wurden 20 ml 20%ige Essigsäure und 90 ml H20 zugegeben, eine halbe Stunde gerührt, die Suspension auf 100 ml konzentriert, und dreimal mit 100 ml Dichlormethan ausgeschüttelt. Die wässrige Phase wurde lyophilisiert. Ausbeute: 2,67 g Feststoff Alternativ wurde 1eq HCl statt 20 ml 20%iger HAc zugegeben.

### Verbindung 8

Eine Lösung von 1,0 g Verbindung 7 in 6 ml abs. DMF wurde nach Zugabe von 607 µl (1,5eq) Diisopropylethylamin und 832 mg (1,1eq) 4-Biphenylcarbonsäure-Nitrophenylester 17h bei RT gerührt. Das Lösungsmittel wurde entfernt und der Rückstand über eine Kieselgelsäule (EtOH:EE:HAc(20%) 1:8:1) gereinigt. Ausbeute: 1,06 g

### Verbindung 9

Eine Lösung von 460 mg Verbindung 8 in 3 ml MeOH wurde mit 193 mg (2eq) Cysteaminhydrochlorid und 3 ml H₂O versetzt und 20 min mit Stickstoff gespült. Nach Zugabe einer katalytischen Menge (2-3 mg) AIBN wurde 24h mit UV-Licht (λ=366 nm) bestrahlt, das Lösungsmittel entfernt, der Rückstand mit H₂O gelöst und über eine RP18 Säule gereinigt (HCl pH 4 >> MeOH / HCl pH 4). Die Produktfraktionen wurden mit verd. NaOH neutralisiert, das MeOH entfernt und lyophilisiert. Ausbeute: 525 mg

### Verbindung 10

Zu einer Lösung von 26 mg (1eq) Verbindung 44 und 81 mg (2,2eq) Verbindung 9 in 3 ml abs. DMF wurden 47 mg (2,2eq) HATU und 58 µl DIPEA zugegeben. Nach 10 min wurde eingeengt und über RP18 (H_{2O}>>EtOH) gereinigt. Ausbeute: 77 mg

### Verbindung 11

30 mg Verbindung 10 wurden in 0,8 ml abs. DMF gelöst. Nach Zugabe von 0,2 ml Piperidine wurde 20 min gerührt, eingeengt, mit 1 ml DMF nachgedampft und über RP18 (H₂O/HAc>>EtOH) gereinigt. Ausbeute: 23 mg; DC-RF: 0,43 in EtOH:EE:HAc(20%) 1:3:1; ¹H NMR (500 MHz, CD₃OD) δ ppm 7.91-7.86 (m, 5H), 7.69-7.64 (m, 4H, Ar), 7.63-7.59 (m, 4H, Ar), 7.50 (d, *J* = 1.03 Hz, 1H, Ar), 7.47-7.41 (m, 5H, Ar), 7.39-7.34 (m, 2H, Ar), 4.24 (t, *J* = 5.75 Hz, 2H, ArOCH₂), 4.11-4.05 (m, 2H, H8 H8'), 3.94-3.76 (m, 4H, H9a H9a' OCH₂a OCH₂a'), 3.82 (s, 3H, OCH₃), 3.82 (s, 3H, OCH₃'), 3.71-3.44 (m, 18H, H4 H4' H5 H5' H6 H6' H7 H7' H9b H9b' OCH₂b OCH₂b'CH₂NH CH₂NH' CH₂NH₂), 2.72 (t, *J* = 6.78 Hz, 4H, CH₂S CH₂S'), 2.69-2.58 (m, 6H, H3-e H3-e' SCH₂ SCH₂'), 1.98 (s, 3H, COCH₃), 1.98 (s, 3H, COCH₃'), 1.83-1.64 (m, 6H, CH₂CH₂CH₂ CH₂CH₂CH₂' CH₂CH₂NH₂, H3-a H3-a') ¹³C NMR (125 MHz, CD₃OD) δ ppm 175.1 171.0 170.3 168.9 168.4 157.7 145.6 141.2 139.4 139.3 134.4 134.3 131.4 130.0 129.9 129.1 129.0 128.1 128.0 127.3 122.1 120.7 112.9 100.3 74.8 72.2 71.1 68.5 68.4 63.7 53.9 53.4 45.1 41.7 41.0 40.4 39.5 32.1 32.0 30.9 30.8 29.5 29.2 29.0 22.8; Masse: Maldi-TOFMS m/z [C71H91N7O21S2+Na]⁺ 1464.6 Gefunden: 1464,1

### Verbindung 12

Zu einer Lösung von 20 mg Verbindung 10 in wenig EtOH wurde Wasser zugegeben bis eine Trübung auftrat und der pH wurde mit 2 M NaOH auf 12-13 eingestellt. Nach 2 h wurde mit verd. HCl neutralisiert, eingeengt und über RP18 gereinigt. Ausbeute: 13mg

### Verbindung 13

Hergestellt analog zu Verbindung 10. R_{f} 0,74 (1:3:1 EtOH:EE:HAc20%); ¹H NMR (500MHz, CD₃OD): 7.96 (d, *J* = 8.12 Hz, 1H, Ar), 7.91-7.87 (m, 4H, Ar), 7.69-7.64 (m, 4H, Ar), 7.63-7.59 (m, 4H, Ar), 7.50 (d, *J* = 1.42 Hz, 1H, Ar), 7.44 (dd, *J* = 8.07, 1.53 Hz, 1H, Ar), 7.45-7.40 (m, 4H, Ar), 7.37-7.33 (m, 2H, Ar), 4.27 (dd, *J* = 5.07, 3.83 Hz, 2H, ArOCH₂), 4.10-4.03 (m, 2H, H8 H8'), 3.92-3.76 (m, 14H, H4 H4' H5 H5' H6 H6' OCH₂a OCH₂a' OCH₃ OCH₃'), 3.70-3.43 (m, 34H, H7 H7' H9a H9a' H9b H9b' OCH₂b OCH₂b' CH₂NH CH₂NH' CH₂N3 5x CH₂OCH₂), 2.72 (t, *J* = 6.79 Hz, 2H, SCH₂), 2.72 (t, *J* = 7.10 Hz, 2H, SCH₂') 2.68-2.59 (m, 6H, H3-e H3-e' SCH₂ CH₂SCH₂'), 1.98 (s, 6H, COCH₃ COCH₃'), 1.83-1.77 (m, 4H, CH₂CH₂CH₂ CH₂CH₂CH₂'), 1.74 (t, *J* = 12.33 Hz, 1H, H3-a), 1.73 (t, *J* = 12.37 Hz, 1H, H3-a') ¹³C-NMR (125MHz, CD₃OD): δ ppm 175.1 171.0 170.3 170.2 168.8 167.0 158.3 145.6 141.2 139.8 134.4 132.6 130.1 129.1 129.0 128.1 128.0 125.1 120.8 113.6 100.4 74.8 72.3 71.7 71.6 71.5 71.1 70.2 69.8 68.6 63.7 53.9 53.4 51.8 45.1 41.8 41.0 40.8 32.1 31.9 30.8 29.1 22.8; HRMS-ESI (m/z): calcd for C80H107N9O26S2 (M+2H) 837.8457 found 837.8440

### Verbindung 14

Hergestellt analog zu Verbindung 10.

### Verbindung 15

Methode A: Hergestellt analog zu Verbindung 11.
Methode B: 55 mg Verbindung 13 wurden in 5 ml MeOH gelöst, mit 0,35 ml 20%iger HAc und einer Spatespitze Pd auf Kohle (10%) versetzt und 4h hydriert. Die Suspension wurde filtriert, das Lösungsmittel entfernt, der Rückstand mit Wasser auf eine RP18 Säule aufgetragen, die Säule mit verd. HCl gespült, die Substanz mit H₂O>>EtOH eluiert, eingeengt und gefriergetrocknet. Ausbeute 40mg; R_{f} 0,44 (1:3:1 EtOH:EE:HAc20%); ¹H-NMR (500MHz, CD₃OD): δ ppm 7.94-7.87 (m, 5H, Ar), 7.70-7.66 (m, 4H, Ar), 7.64-7.60 (m, 4H, Ar), 7.52 (d, *J* = 1.22 Hz, 1H, Ar), 7.49-7.41 (m, 5H, Ar), 7.36 (t, J = 7.31, 7.31 Hz, 2H, Ar), 4.58 (s, 2H, CH₂CH₂NH₃⁺), 4.29 (dd, *J* = 4.44, 3.94 Hz, 2H, ArOCH₂), 4.11-4.04 (m, 2H, H8 H8'), 3.93-3.76 (m, 14H, H5 H5' H6 H6' H9a H9a' OCH₂a OCH₂a' OCH₃ OCH₃'), 3.72-3.42 (m, 30H, H4 H4' H7 H7' H9b H9b' OCH₂b OCH₂b' CH₂NH CH₂NH' 4xCH₂OCH₂ OCH₂), 3.09-3.05 (m, 2H, CH₂NH₃⁺), 2.74 (t, *J* = 6.70, 6.70 Hz, 2H CH₂S), 2.73 (t, *J* = 7.04, 7.04 Hz, 2H, CH₂S'), 2.66 (dd, *J* = 13.14, 4.87 Hz, 2H, H3-e H3-e'), 2.67-2.59 (m, 4H SCH₂ SCH₂'), 1.98 (s, 6H, NHCOCH₃ NHCOCH₃'), 1.84-1.77 (m, 4H, CH₂CH₂CH₂ CH₂CH₂CH₂'), 1.76-1.69 (m, 2H, H3-a H3-a') ¹³C NMR (120 MHz, CD₃OD): δ ppm 175.1 171.0 170.3 170.2 168.8 167.4 158.1 145.6 141.2 141.1 139.7 134.4 132.3 130.1 129.2 129.1 129.0 128.1 128.1 126.4 122.1 120.9 113.5 100.3 74.8 72.3 71.1 71.0 68.5 72.2 71.5 71.4 71.3 71.2 71.1 71.0 70.3 69.8 67.9 63.7 53.9 53.5 53.4 45.1 41.8 41.0 40.7 32.1 31.9 30.9 29.2 22.8; Masse: Maldi-TOFMS m/z [C80H109N7O26S2+Na]⁺ 1670.7 Gefunden: 1670.3

### Verbindung 16

Hergestellt analog zu Verbindung 12. R_{f} 0,42 (1:2:1 EtOH:EE:HAc20%); ¹H NMR (500MHz, CD₃OD): δ ppm 7.92-7.85 (m, 4H, Ar), 7.68-7.53 (m, 10H, Ar), 7.51-7.39 (m, 5H, Ar), 7.35 (t, *J* = 7.31, 7.31 Hz, 2H, Ar), 4.29-4.25 (m, 2H, ArOCH₂), 4.14-4.04 (m, 2H, H8 H8'), 3.95-3.43 (m, 40H, H4 H4' H5 H5' H6 H6' H7 H7' H9a H9a' H9b H9b' OCH₂a OCH₂a' OCH₂b OCH₂b' CH₂NH CH₂NH' 5x CH₂OCH₂), 3.05-3.09 (m, 2H, CH₂NH₃⁺), 2.78 (dd, *J* = 12.32, 3.18 Hz, 2H, H3-e H3-e'), 2.73 (t, *J* = 6.33, 6.33 Hz, 4H, SCH₂ SCH₂'), 2.69-2.60 (m, 4H, CH₂S CH₂S'), 2.01 (s, 6H, COCH₃ COCH₃'), 1.86-1.78 (m, 4H, CH₂CH₂CH₂ CH₂CH₂CH₂'), 1.70-1.57 (m, 2H, H3-a H3-a') ¹³C-NMR (125MHz, CD₃OD): δ ppm 175.5 173.6 170.1 169.9 168.9 167.3 158.1 145.5 141.1 139.7 134.4 134.3 134.0 133.9 133.2 133.1 132.3 132.2 130.1 130.0 129.2 129.0 128.1 128.0 126.2 120.9 113.5 101.4 101.3 74.5 72.5 71.5 71.4 71.3 71.2 70.9 70.3 69.8 69.3 68.1 67.8 63.9 54.1 44.7 42.4 42.3 41.0 40.9 40.7 32.2 32.0 31.2 29.3 22.8; HRMS-ESI (m/z): calcd for C78H103N7Na2O26S2 (M+H) 1664.6262 found 1664.6324

### Verbindung 17

Hergestellt analog zu Verbindung 10. R_{f} 0,72 in 1:3:1 EtOH:EE:HAc(20%); ¹H-NMR (500MHz, CD₃OD): δ ppm 7.93 (d, *J* = 8.41 Hz, 4H, Ar), 7.89 (dd, *J* = 8.37, 3.85 Hz, 1H, Ar), 7.72 (d, *J* = 8.41 Hz, 4H, Ar), 7.67 (dd, *J* = 8.30, 0.96 Hz, 4H, Ar), 7.61 (d, *J* = 7.33 Hz, 1H, Ar), 7.46 (t, *J* = 7.63, 7.63 Hz, 4H, Ar), 7.45-7.41 (m, 1H, Ar), 7.38 (t, *J* = 7.39, 7.39 Hz, 2H, Ar), 4.06 (ddd, *J* = 8.52, 7.36, 3.51 Hz, 2H, H8 H8'), 3.89 (td, *J* = 9.22, 5.80, 5.80 Hz, 2H, OCH₂a OCH₂a'), 3.86 (dd, *J* = 10.92, 7.51 Hz, 2H, ArOCH₂), 3.81 (dd, *J* = 15.64, 9.24 Hz, 2H, H9a H9a'), 3.84-3.81 (m, 8H, CH₃ CH₃' H5 H5'), 3.68 (dd, *J* = 10.47, 1.39 Hz, 2H, H6 H6'), 3.67-3.48 (m, 10H, CH₂NH CH₂NH' H4 H4' H9b H9b' OCH₂b OCH₂b'), 3.46 (dd, *J* = 8.71, 1.19 Hz, 2H, H7 H7'), 2.75-2.56 (m, 12H, CH₂SCH₂ CH₂SCH₂' CH₂SCO H3-e H3-e'), 1.98 (s, 6H, NHCOCH₃ NHCOCH₃'), 1.92 (s, 3H, SCOCH₃), 1.84-1.70 (m, 8H, CH₂CH₂CH₂ CH₂CH₂CH₂' H3-a H3-a' CH₂CH₂SCO) ¹³C NMR (125 MHz, CD₃OD): δ ppm 195.6 175.1 173.3 171.0 170.4 145.7 141.3 134.4 130.1 129.1 129.0 128.2 128.1 128.0 120.5 112.9 100.4 74.8 72.2 71.1 68.6 69.0 63.6 53.9 53.4 45.1 41.8 40.4 32.0 30.9 29.1 30.8 26.0 22.7 22.6; Masse: Maldi-TOFMS m/z [C73H92N6O22S3+Na]⁺ 1524,6 Gefunden: 1524,6

### Verbindung 18

Hergestellt analog zu Verbindung 10. Einsatz an Verbindung 3: 23 mg Ausbeute 132 mg; R_{f}: 0,68 in 1:3:1, EtOH:EE:HAC20%;

### Verbindung 19

132 mg Verbindung 18 wurden in 5 ml Pyridin gelöst. Bei 0°C wurden 340 mg Essigsäureanhydrid und eine katalytische Menge DMAP zugegeben. Die Lösung wurde über Nacht bei RT gerührt, eingengt und über Kieselgel (EE>>EtOH) gereinigt. Ausbeute 136 mg; R_{f}: 0,55 in 5:1 EE:EtOH; ¹H-NMR (500MHz, CD₃OD): δ ppm 7.90 (d, *J* = 8.06 Hz, 1H, Ar), 7.82 (d, *J* = 3.29 Hz, 2H, Ar), 7.80 (d, *J* = 3.30 Hz, 2H, Ar), 7.67 (d, *J* = 5.55 Hz, 2H, Ar), 7.65 (d, *J* = 5.64 Hz, 2H, Ar), 7.63-7.59 (m, 4H, Ar), 7.50 (d, *J* = 1.40 Hz, 1H, Ar), 7.47-7.41 (m, 5H, Ar), 7.36 (t, *J* = 7.35, 7.35 Hz, 2H, Ar), 6.11 (tdd, *J* = 16.97, 10.71, 5.41, 5.41 Hz, 1H, CH=CH₂), 5.53-5.45 (m, 2H, H4 H4'), 5.42 (ddd, *J* = 17.05, 2.78, 1.36 Hz, 1H, CH=CH₂a), 5.31 (ddd, *J* = 10.50, 2.52, 1.29 Hz, 1H, CH=CH₂b), 5.27 (dd, *J* = 9.08, 2.11 Hz, 2H, H7 H7'), 4.80 (ddd, *J* = 12.10, 10.61, 4.59 Hz, 2H, H8 H8'), 4.70 (td, *J* = 5.57, 1.37, 1.37 Hz, 1H, ArOCH₂a), 4.22 (td, *J* = 10.77, 1.88, 1.88 Hz, 1H, ArOCH₂b), 4.02 (t, *J* = 10.51, 10.51 Hz, 2H, H5 H5'), 3.92-3.86 (m, 4H, OCH₂a OCH₂a' H9a H9a'), 3.80 (s, 3H, COOCH₃), 3.80 (s, 3H, COOCH₃'), 3.61-3.53 (m, 4H, CH₂NH CH₂NH'), 3.44-3.39 (m, 2H, OCH₂b OCH₂b'), 3.37 (dd, *J* = 14.45, 7.68 Hz, 1H, H9b), 3.37 (dd, *J* = 14.57, 7.45 Hz, 1H, H9b'), 2.77-2.72 (m, 4H, CH₂S CH₂S'), 2.68-2.60 (m, 6H, SCH₂ SCH₂' H3-e H3-e'), 2.17 2.12 2.11 1.98 (4xs, 18H, 6xCOCH₃), 1.86-1.78 (m, 12H, 2xCOCH₃ CH₂CH₂CH₂ CH₂CH₂CH₂' H3-a H3-a'); ¹³C NMR (125 MHz, CD₃OD): δ ppm 173.5 172.7 172.6 172.3 172.2 171.8 170.1 170.0 169.9 169.8 168.8 167.3 157.9 145.7 141.2 139.5 134.3 133.8 132.3 130.1 129.2 128.9 128.2 128.1 126.4 120.6 119.4 113.5 100.1 73.3 70.8 70.6 70.0 69.9 71.2 64.5 53.3 50.2 41.4 41.0 40.4 39.2 32.2 32.0 31.1 31.0 29.1 28.9 22.7 21.5 21.2 20.8; Masse: Maldi-TOFMS m/z [C83H100N6O27S2+H]⁺ 1677,61 Gefunden: 1677,6

### Verbindung 20

Hergestellt analog zu Verbindung 10.

### Verbindung 21

Hergestellt analog zu Verbindung 12. HRMS-ESI (m/z): calcd for C63H81N7Na2O20S2 (M-2Na) 659.7494 found 659.7539

### Verbindung 22

Hergestellt analog zu Verbindung 10 mit N-(6-Fmocaminohexanoyl)glutaminsäure. Verseifung erfolgte analog Verbindung 12. R_{f} 0,21 in 1:3:1 EtOH:EE:HAc(20%); ¹H NMR (500 MHz, CD₃OD) δ ppm 7.94-7.89 (m, 4H, Ar), 7.73-7.69 (m, 4H, Ar), 7.67-7.65 (m, 4H, Ar), 7.46 (t, *J* = 7.66 Hz, 4H, Ar), 7.37 (t, *J* = 7.39 Hz, 2H, Ar), 4.30 (dd, *J* = 8.87, 5.13 Hz, 1H, CHNH), 4.12-4.07 (m, 2H, H8 H8'), 3.91-3.81 (m, 4H, OCH₂a OCH₂a' H9a H9a'), 3.73-3.63 (m, 6H, H5 H5' H6 H6' H4 H4'), 3.59-3.49 (m, 4H, H9b H9b' OCH₂b OCH₂b'), 3.47-3.43 (m, 2H, H7 H7'), 3.39-3.27 (m, 4H, CH₂NH CH₂NH), 2.94 (t, *J* = 7.53 Hz, 1H, CH₂NH₂), 2.87-2.81 (m, 2H, H3-e H3-e'), 2.67-2.54 (m, 8H, CH₂SCH₂ CH₂SCH₂'), 2.33-2.24 (m, 4H, CH₂CO CH₂CO'), 2.10-1.88 (m, 2H, CH₂CH), 2.01 (s, 3H, COCH₃), 2.01 (s, 3H, COCH₃'), 1.82-1.75 (m, 4H, SCH₂CH₂), 1.70-1.55 (m, 6H, H3-a H3-a' CH₂CH₂CH₂CH₂NH₂), 1.42-1.34 (m, 2H, CH₂CH₂CH₂NH₂); ¹³C NMR (125 MHz, CD₃OD): δ ppm 175.9 175.5 175.1 174.5 174.0 170.0 145.6 141.3 134.5 130.1 129.1 129.0 128.2 128.1 102.0 74.3 72.7 71.5 71.4 69.7 69.6 63.9 54.7 54.3 44.7 44.6 42.8 40.7 40.3 39.9 36.3 33.4 32.2 31.3 29.3 29.2 28.3 26.8 26.0 22.7; HRMS-ESI (m/z): calcd for C69H92N8Na2021S2 (M-2Na) 716.2915 found 716.2900

### Verbindung 23

91 mg Adipinsäure wurden in 2 ml abs. DMF gelöst und 14 mg HATU und 173 mg DIPEA zugegeben. Nach 30 s wurden 45 mg Verbindung 11 zugegeben, nach 5 Min wurde eingeengt, und über RP18 gereinigt. Ausbeute: 27 mg; R_{f} 0,58 in 1:3:1 EtOH:EE:HAc(20%); ¹H-NMR (500MHz, CD₃OD): δ ppm 7.90 (d, *J* = 2.32 Hz, 2H, Ar), 7.89 (d, *J* = 2.67 Hz, 2H, Ar), 7.86 (d, *J* = 8.05 Hz, 1H, Ar), 7.66 (d, *J* = 7.83 Hz, 4H, Ar), 7.61 (dd, *J* = 7.25, 1.07 Hz, 4H, Ar), 7.49 (d, *J* = 1.29 Hz, 1H, Ar), 7.46-7.40 (m, 5H, Ar), 7.37-7.33 (m, 2H, Ar), 4.15 (t, *J* = 5.99, 5.99 Hz, 2H, ArOCH₂), 4.08 (ddd, *J* = 11.25, 7.26, 3.46 Hz, 2H, H8 H8'), 3.92-3.78 (m, 12H, H9a H9a' H5 H5' OCH₂a OCH₂a' OCH₃ OCH₃'), 3.69 (dd, *J* = 10.45, 1.34 Hz, 2H, H6 H6'), 3.68-3.44 (m, 12H, H4 H4' H7 H7' H9b H9b' OCH₂b OCH₂b' CH₂NHCOArCONHCH₂), 3.36 (t, *J* = 6.60, 6.60 Hz, 2H, CH₂NH), 2.73 (dd, *J* = 12.64, 6.85 Hz, 4H, CH₂S CH₂S'), 2.69-2.59 (m, 6H, SCH₂ SCH₂' H3-e H3-e'), 2.21-2.12 (m, 4H, CH₂CO CH₂COO), 2.04-2.00 (m, 2H, ArOCH₂CH₂), 1.98 (s, 6H, NHCOCH₃ NHCOCH₃'), 1.85-1.77 (m, 4H, CH₂CH₂CH₂ CH₂CH₂CH₂'), 1.73 (t, *J* = 12.38, 12.38 Hz, 2H, H3-a H3-a'), 1.63-1.56 (m, 4H, CH₂CH₂CH₂CH₂) ¹³C NMR (75MHz, CD₃OD): δ ppm 182.4 176.4 175.1 171.0 170.3 169.0 167.5 158.3 145.6 141.2 139.6 134.4 132.2 130.1 129.1 129.0 128.1 128.0 126.4 120.6 112.8 100.4 74.8 72.3 71.1 68.5 67.7 63.7 53.9 53.4 45.1 41.8 41.7 41.0 40.5 38.7 37.2 37.1 32.1 31.9 30.8 30.1 29.2 29.0 27.2 27.1 22.8

### Verbindung 24

Hergestellt analog zu Verbindung 23 mit 3,3'-Dithiopropionsäure. R_{f} 0,63 in 1:3:1 EtOH:EE:HAc(20%);

### Verbindung 25

13 mg Verbindung 23 wurden in Wasser gelöst, mit 2 M NaOH auf pH 12-13 eingestellt, nach 2 h neutralisiert und über RP18 gereinigt. Ausbeute: 10 mg; R_{f} 0,31 in 1:3:1 EtOH:EE:HAc(20%); ¹H-NMR (500MHz, CD₃OD): δ ppm 7.90-7.87 (m, 4H, Ar), 7.84 (dd, *J* = 8.01, 0.75 Hz, 1H, Ar), 7.68-7.63 (m, 4H, Ar), 7.63-7.59 (m, 4H, Ar), 7.48 (d, *J* = 1.08 Hz, 1H, Ar), 7.45-7.40 (m, 5H, Ar), 7.37-7.33 (m, 2H, Ar), 4.15 (t*, J* = 6.40, 6.40 Hz, 2H, CH₂OAr), 4.13-4.07 (m, 2H, H8 H8'), 3.90 (td, *J* = 9.67, 6.23, 6.23 Hz, 2H, OCH₂a OCH₂a'), 3.85 (dd, *J* = 13.37, 2.41 Hz, 2H, H9a H9a'), 3.73-3.41 (m, 20H, H4 H4' H5 H5' H6 H6' H7 H7' H9b H9b' OCH₂b OCH₂b' CH₂NH CH₂NH'), 3.37 (t, *J* = 6.76, 6.76 Hz, 2H, CH₂NHCOCH₂), 2.86-2.80 (m, 2H, H3-e H3-e), 2.76-2.72 (m, 4H, CH₂S CH₂S'), 2.69-2.61 (m, 4H, SCH₂ SCH₂'), 2.26-2.18 (m, 4H, CH₂CO CH₂COO), 2.06-2.02 (m, 2H, OCH₂CH₂), 2.00 (s, 6H, COCH₃ COCH₃'), 1.86-1.79 (m, 4H, CH₂CH₂CH₂ CH₂CH₂CH₂'), 1.66-1.54 (m, 6H, H3-a H3-a' CH₂CH₂CH₂CH₂) ¹³C NMR (75MHz, CD₃OD): δ ppm 176.2 175.5 174.5 174.4 170.0 169.9 169.0 167.7 158.0 145.5 141.3 139.6 134.5 132.1 130.0 129.1 129.0 128.1 128.0 126.5 120.6 112.8 102.0 (2C, C2 C2'), 74.3 71.5 71.4 69.7 67.8 64.0 63.9 54.2 44.6 42.8 41.1 40.5 37.3 36.9 32.2 31.9 31.5 31.4 30.1 29.4 29.3 26.8 26.3 22.7

### Verbindung 26

9 mg Verbindung 16 wurden in 1 ml abs. DMF gelöst und 53 mg Adipinsäure-diNHS und 46 µl DIPEA zugegeben. Nach 1 h wurde eingeengt, mit wenig Wasser versetzt, mit EE dreimal ausgeschüttelt. Die wässrige Phase wurde filtriert, und über RP18 (H₂O>>CH₃CN) gereinigt. Das Acetonitril wurde bei RT im Vakuum entfernt. Der wässrige Rückstand wurde mit flüssigem Stickstoff gefroren und lyophilisiert. Die gesamte wässrige Aufarbeitung wurde in max. 2 h durchgeführt. Ausbeute: 7 mg

### Verbindung 27

2 mg 6-Maleinimidohexansäure-NHS wurden in 1 ml DMF gelöst und nach Zugabe von 3,5 mg HATU und 3,5 mg DIPEA zu 15 mg Verbindung 16 gegeben. Die Lösung wurde nach 5 Min eingeengt und über RP18 gereinigt. Ausbeute: 14 mg; R_{f} 0,29 in 1:3:1 EtOH:EE:HAc(20%); ¹H-NMR (500MHz, CD₃OD): δ ppm 7.93-7.87 (m, 5H, Ar), 7.69-7.65 (m, 4H, Ar), 7.61 (d, *J* = 7.21 Hz, 4H, Ar), 7.52 (d, *J* = 1.10 Hz, 1H, Ar), 7.47-7.40 (m, 5H, Ar), 7.37-7.33 (m, 2H, Ar), 6.77 (s, 2H, CH=CH), 4.30-4.27 (m, 2H, ArOCH₂), 4.13-4.05 (m, 2H, H8 H8'), 3.94-3.40 (m, 44H, H4 H4' H5 H5' H6 H6' H7 H7' H9a H9a' H9b H9b' OCH₂a OCH₂a' OCH₂b OCH₂b' CH₂NH CH₂NH' CH₂NH" CH₂N 5xCH₂OCH₂), 2.87-2.80 (m, 2H, H3-e H3-e'), 2.76-2.72 (m, 4H, CH₂S CH₂S'), 2.69-2.62 (m, 4H, SCH₂ SCH₂'), 2.15 (t, *J* = 7.45, 7.45 Hz, 2H, CH₂CO CH₂CO'), 2.00 (s, 6H, COCH₃ COCH₃'), 1.86-1.79 (m, 4H, CH₂CH₂CH₂ CH₂CH₂CH₂'), 1.63-1.50 (m, 6H, H3-a H3-a' CH₂CH₂CH₂CH₂CH₂), 1.38-1.22 (m, 2H, CH₂CH₂CH₂CH₂CH₂) ¹³C NMR (125MHz, CD₃OD): δ ppm 179.3 176.1 175.8 175.5 172.6 169.9 169.8 168.8 167.2 158.2 145.5 141.3 141.2 139.8 135.4 134.5 132.3 130.1 129.1 129.0 128.1 128.0 126.2 120.9 113.5 102.0 74.3 72.7 71.6 71.5 71.4 71.2 70.8 70.2 69.9 69.7 69.6 64.0 63.9 54.2 44.7 42.8 41.0 40.8 40.3 38.5 36.8 32.2 32.0 31.5 29.5 29.4 29.3 27.4 26.5 22.7

### Verbindung 28

50 mg Verbindung 15 und 16 mg Fmoc-protected monohydrazinobernsteinsäure wurden in 1 ml DMF gelöst. 15 mg HATU und 39 µl DIPEA wurden zugegeben, nach 5min wurde die Lösung eingeengt und über RP18 gereinigt. Das Produkt wurde in wenig EtOH gelöst, mit Wasser verdünnt bis eine Trübung auftrat und mit verd. NaOH auf pH 12-13 eingestellt. Nach 3 h wurde neutralisiert und über RP18 gereinigt. Ausbeute 16 mg; R_{f} 0,33 in 1:3:1 EtOH:EE:HAc(20%); ¹H NMR (500 MHz, CD₃OD) δ ppm 7.93-7.86 (m, 5H, Ar), 7.68-7.64 (m, 4H, Ar), 7.62-7.59 (m, 4H, Ar), 7.51 (d, *J* = 1.34 Hz, 1H, Ar), 7.47-7.40 (m, 5H, Ar), 7.37-7.32 (m, 2H, Ar), 4.28 (dd, *J* = 4.63, 4.03 Hz, 2H, ArOCH₂), 4.13-4.05 (m, 2H, H8 H8'), 3.93-3.81 (m, 6H, OCH₂a OCH₂a' H9a H9a' ArOCH₂CH₂), 3.72-3.40 (m, 32H, H4 H4' H5 H5' H6 H6' H7 H7' H9b H9b' OCH₂b OCH₂b' 5xOCH₂CH₂), 3.32-3.28 (m, 4H, 2xCH₂NH), 2.85-2.80 (m, 2H, H3-e H3-e'), 2.74 (t, *J* = 6.87 Hz, 4H, CH₂S CH₂S'), 2.69-2.61 (m, 4H, SCH₂ SCH₂'), 2.51-2.44 (m, 2H, COCH₂), 2.43-2.39 (m, 2H, COCH₂), 2.00 (s, 6H, COCH₃ COCH₃'), 1.86-1.78 (m, 4H, CH₂CH₂CH₂ CH₂CH₂CH₂'), 1.62-1.52 (m, 2H, H3-a H3-a')) ¹³C-NMR (125MHz, CD₃OD): δ ppm 175.5 174.6 174.5 174.4 174.0 169.9 169.8 168.9 167.3 158.2 145.5 141.2 139.8 134.5 132.3 130.1 130.0 129.1 129.0 128.1 128.0 126.2 120.9 113.5 102.0 101.3 74.3 72.7 71.6 71.4 71.3 71.2 70.8 70.3 69.8 69.7 64.0 63.9 54.2 44.7 42.9 42.8 41.0 40.8 40.4 32.2 32.0 31.5 30.5 29.5 29.4 22.7; HRMS-ESI (m/z): calcd for C82H109N9Na2028S2 (M-2Na) 865.8417 found 865.8499

### Verbindung 29

Hergestellt mit 2-(9-Fmoc-amino)terephthalsäure analog Verbindung 10. Verseifung erfolgte analog Verbindung 12. R_{f} 0,32 (1:3:1 EtOH:EE:HAc20%); ¹H NMR (300MHz, CD₃OD): δ ppm 7.88 (d, *J* = 8.37 Hz, 4H, Ar), 7.66 (d, *J* = 8.36 Hz, 4H, Ar), 7.61 (d, *J* = 7.08 Hz, 4H, Ar), 7.39-7.49 (m, 5H, Ar), 7.35 (t, *J* = 7.24, 7.24 Hz, 2H, Ar), 7.13 (d, *J* = 1.59 Hz, 1H, Ar), 6.97 (dd, *J* = 8.16, 1.70 Hz, 1H, Ar), 4.10 (dt, *J* = 8.65, 8.39, 3.23 Hz, 2H, H8 H8'), 3.94-3.43 (m, 20H, H4 H4' H5 H5' H6 H6' H7 H7' H9a H9a' H9b H9b' OCH₂a OCH₂a' OCH₂b OCH₂b' CH₂NH CH₂NH'), 2.75-2.58 (m, 10H, H3-e H3-e' CH₂SCH₂SCH₂'), 1.86-1.76 (m, 4H, CH₂CH₂CH₂ CH₂CH₂CH₂'), 1.72 (t, *J* = 11.29, 11.29 Hz, 2H, H3-a H3-a') ¹³C-NMR (75MHz, CD₃OD): δ ppm 175.3 172.2 171.1 170.3 170.0 145.6 141.3 140.0 134.4 130.0 129.1 129.0 128.1 128.0 120.0 116.9 115.6 100.2 74.8 72.3 71.4 68.9 63.7 53.9 44.9 41.9 40.9 40.5 32.1 32.0 31.0 29.3 22.8; HRMS-ESI (m/z): calcd for C66H78Na2N6O20S2 [M-2Na] 676.7416 found 676.7445

### Verbindung 30

Hergestellt analog Verbindung 8.

### Verbindung 31

Hergestellt analog Verbindung 9.

### Verbindung 32

Hergestellt analog Verbindung 13.

### Verbindung 33

Hergestellt aus Verbindung 32 analog Verbindung 10. Verseift analog Verbindung 12. HRMS-ESI (m/z): calcd for C78H101N9Na2028S2 (M-2Na) 837.8104 found 837.8132

### Verbindung 34

Hergestellt mit 2-Hydroxyterephtalsäure analog Verbindung 29. R_{f} 0,34 (1:3:1 EtOH:EE:HAc20%); ¹H NMR (300MHz, CD₃OD): δ ppm 7.93-7.88 (m, 4H, Ar), 7.85 (d, *J* = 8.22 Hz, 1H, Ar), 7.67 (d, *J* = 8.48 Hz, 4H, Ar), 7.62 (d, *J* = 7.14 Hz, 4H, Ar), 7.47-7.39 (m, 4H, Ar), 7.35 (t, *J* = 7.23, 7.23 Hz, 2H, Ar), 7.11 (d, J = 1.51 Hz, 1H, Ar), 6.92 (d, *J* = 7.99 Hz, 1H, Ar), 4.13-4.04 (m, 2H, H8 H8'), 3.94-3.39 (m, 20H, H4 H4' H5 H5' H6 H6' H7 H7' H9a H9a' H9b H9b' OCH₂a OCH₂a' CH₂COArCONHCH₂ OCH₂b OCH₂b'), 2.88-2.80 (m, 2H, H3-e H3-e'), 2.75-2.60 (m, 8H, CH₂SCH₂ CH₂SCH₂'), 1.997 (s, 3H, COCH₃), 1.995 (s, 3H, COCH₃), 1.88-1.76 (m, 4H, CH₂CH₂CH₂ CH₂CH₂CH₂'), 1.65-1.54 (m, 2H, H3-a H3-a') ¹³C-NMR (75MHz, CD₃OD): δ ppm 175.5 174.5 170.7 170.4 170.1 145.6 141.3 134.5 130.0 129.1 129.0 139.5 128.1 128.0 102.0 74.3 72.6 71.6 69.7 64.0 54.2 44.5 42.8 40.9 40.2 32.4 31.9 31.5 31.4 29.4 29.3 22.7; HRMS-ESI (m/z): calcd for C66H78Na2N6021S2 [M-2Na] 677.2336 found 677.2319

### Verbindung 41

240 mg HO(O)CCH₂CH₂C(O)NH-[CH₂CH₂O]ₙCH₂CH₂-NHC(O)CH₂CH₂C(O)OH (Iris Biotec GmbH, alpha,omega-bis-Carboxy poly(ethylen glycol), PEG-MW 2000 Dalton) wurden in 0,5 ml abs. DMF gelöst und 4,9 mg HATU und 46,7 µl DIPEA zugegeben. Die Lösung wurde direkt zu 22 mg, in 0,5 ml DMF gelöster, Verbindung 15 gegeben. Nach Entfernen des Lösungsmittels wurde die Substanz über RP18 gereinigt (erst verd. NaHCO₃ pH 9, dann 5 Säulenvolumen verd. NaHCO₃ pH 9 : EtOH 7:3, dann 1 Säulenvolumen Wasser:EtOH 7:3, dann Wasser>>EtOH) und gefriergetrocknet. Ausbeute 43 mg (99%); R_{f} 0,21 (1:1:1:2 EtOH:EE:HAc20%:Dioxan); Das Maldi-Tof Spektrum ergab Molmassen von 3412 bis 4163 mit einer mittleren Molmasse von 3806 (n = 35 bis 51).

### 2-(3-Fmoc amino)propyloxy-Terephthalsäure (Verbindung 44)

Hergestellt analog zu Verbindung 2.

### Verbindung 45

Verbindung 5 wurde analog der Herstellung von Verbindung 9 mit Cysteamin bestrahlt und gereinigt. Das Produkt wurde anschliessend analog der Herstellung von Verbindung 10 mit Terephtalsäure umgesetzt und analog zu Verbindung 12 verseift. HRMS-ESI (m/z): calcd for C40H60Na2N4O20S2 [M-2Na] 490.1627 found 490.1617

### Verbindung 46

Hergestellt analog zu Verbindung 10. Verseift analog Verbindung 12. HRMS-ESI (m/z): calcd for C66H78Na2N6O20S2 [M-2Na] 669.2362 found 669.2353

In Tabelle I sind die hergestellten Verbindungen der Formel (I) aufgelistet.

**Tabelle I**

| Nr. | Struktur |
|---|---|
| 11 | 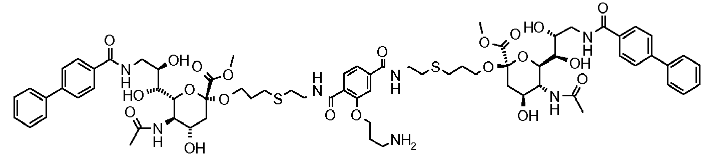 |
| 12 | 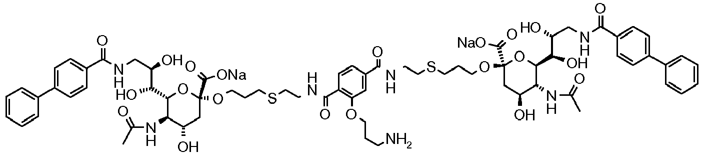 |
| 13 | 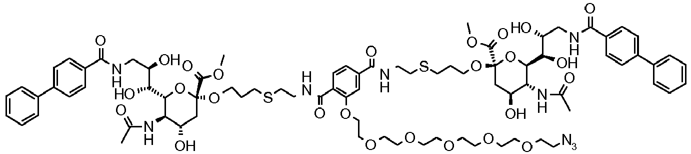 |
| 15 | 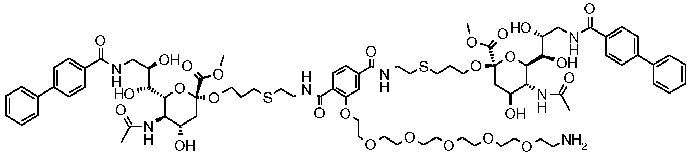 |
| 16 | 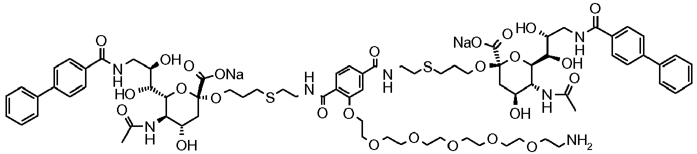 |
| 17 | 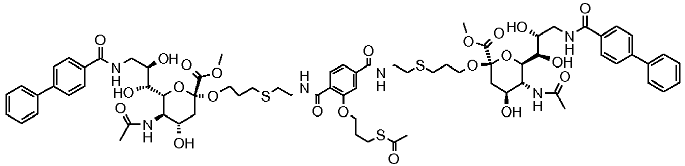 |
| 18 | 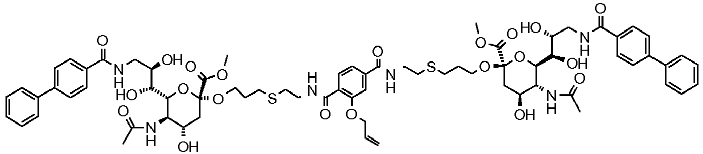 |
| 19 | 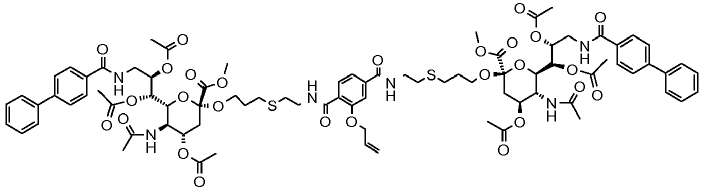 |
| 21 | 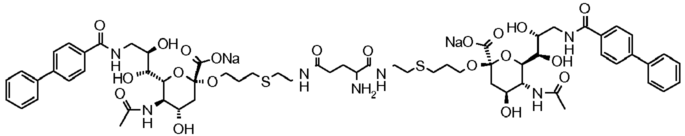 |
| 22 | 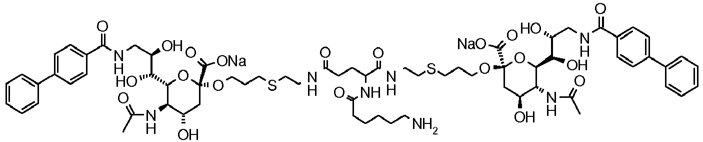 |
| 23 | 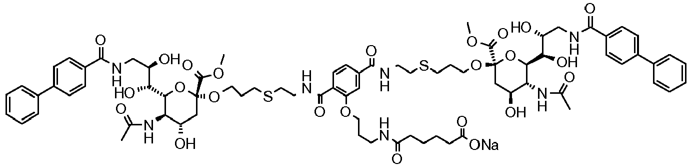 |
| 24 | 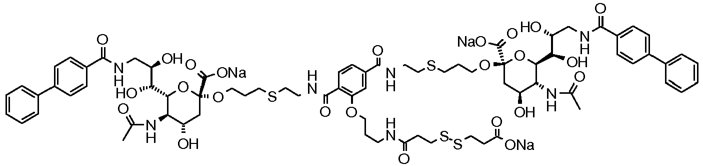 |
| 25 | 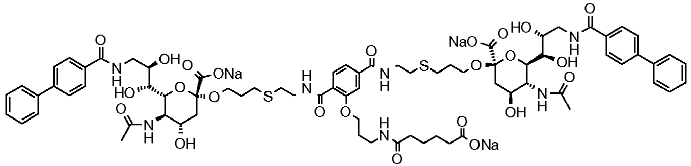 |
| 26 | 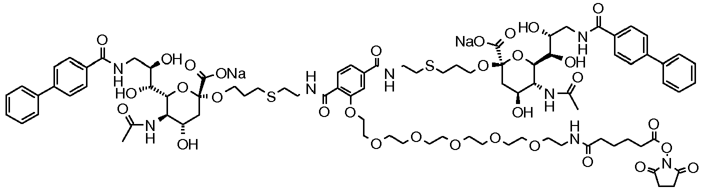 |
| 27 | 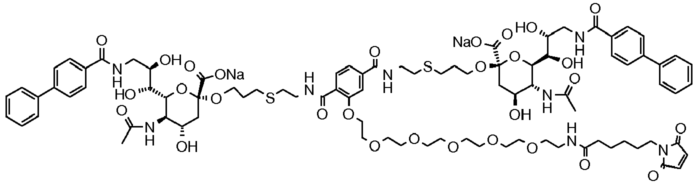 |
| 28 | 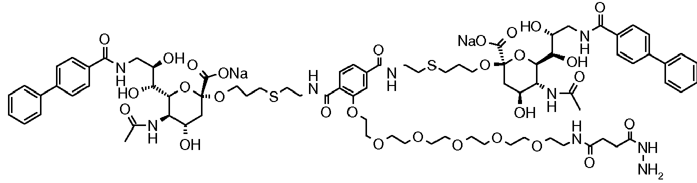 |
| 29 | 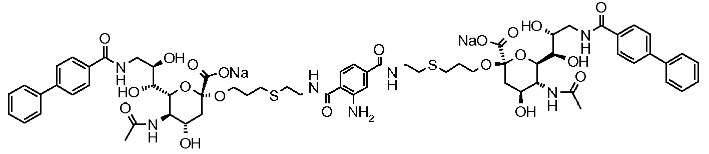 |
| 32 | 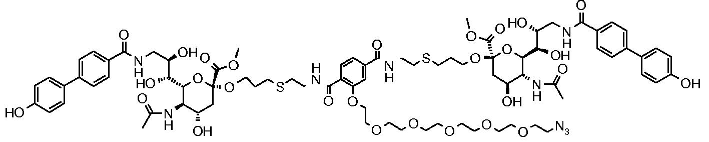 |
| 33 | 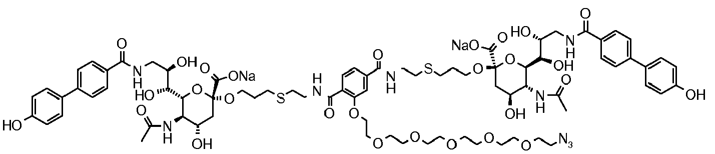 |
| 34 | 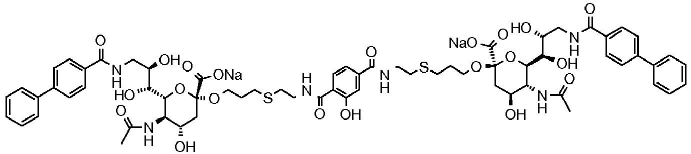 |
| 41 | 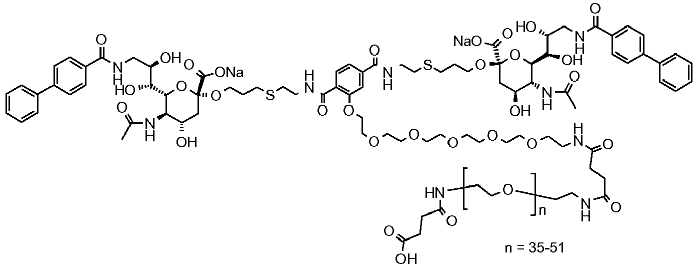 |

### Herstellung niedermolekularer Konjugate

### Verbindung 35

4,7 mg Biotin wurden in 1 ml abs. DMF gelöst, und nach Zugabe von 7,3 mg HATU und 6,2 mg DIPEA wurden 28 mg Verbindung 16 zugegeben. Die Lösung wurde 10 min gerührt, eingeengt und der Rückstand über RP18 gereinigt. Ausbeute 28 mg; R_{f} 0,14 (1:3:1 EtOH:EE:HAc20%); ¹H NMR (500MHz, CD₃OD): δ ppm 7.92-7.87 (m, 4H, Ar), 7.94 (d, *J* = 8.08 Hz, 1H, Ar), 7.69-7.65 (m, 4H, Ar), 7.63-7.60 (m, 4H, Ar), 7.52 (d, *J* = 1.37 Hz, 1H, Ar), 7.46 (dd, *J* = 8.19, 1.55 Hz, 1H, Ar), 7.46-7.40 (m, 4H, Ar), 7.38-7.33 (m, 2H, Ar), 4.46 (ddd, *J* = 7.86, 5.05, 0.66 Hz, 1H, CH-Biotin), 4.30-4.25 (m, 3H, ArOCH₂ CH-Biotin), 4.13-4.06 (m, 2H, H8 H8'), 3.94-3.83 (m, 6H, H9a H9a' OCH₂a OCH₂a' ArOCH₂CH₂), 3.75-3.42 (m, 34H, H4 H4' H5 H5' H6 H6' H7 H7' H9b H9b' OCH₂b OCH₂b' CH₂NH CH₂NH' 4xOCH₂CH₂ CH₂CH₂NH), 3.33-3.31 (m, 2H, CH₂NH), 3.15 (ddd, *J* = 8.76, 5.64, 4.59 Hz, 1H, Biotin-SCH), 2.89 (dd, *J* = 12.75, 5.00 Hz, 1H, Biotin-SCH₂b), 2.86-2.81 (m, 2H, H3-e H3-e'), 2.77-2.72 (m, 4H, CH₂S CH₂S'), 2.71-2.61 (m, 5H, SCH₂ SCH₂' Biotin-SCH₂a), 2.19 (t, *J* = 7.37 Hz, 2H, CH₂CO), 2.01 (s, 6H, COCH₃ COCH₃'), 1.87-1.79 (m, 4H, SCH₂CH₂ SCH₂CH₂'), 1.75-1.51 (m, 6H, H3-a H3-a' CHCH₂CH₂CH₂), 1.44-1.32 (2H, m, CHCH₂CH₂CH₂) ¹³C-NMR (125MHz, CD₃OD): δ ppm 175.5 173.7 170.0 169.3 145.5 141.3 138.4 134.5 130.0 129.1 129.0 128.6 128.1 128.0 101.4 74.5 72.5 71.5 69.4 63.8 54.1 44.7 42.5 41.0 32.0 31.3 29.4 22.7; HRMS-ESI (m/z): calcd for C88H117N9Na2O28S3 (M-2Na+H) 1844.7254 found 1844.7362

### Verbindung 36

15 mg Verbindung 28 und 10 mg Doxorubicin wurden in 2 ml abs. MeOH gelöst und 17 h gerührt. Die Lösung wurde eingeengt und über Kieselgel gereinigt (Laufmittel EtOH:Dioxan:NH₄OH 25% 1:4:1, dann 1:2:1). Ausbeute: 12 mg; R_{f} 0,50 (1-Butanol:HAc100%:H₂O 5:2:3); HRMS-ESI (m/z): calcd for C109H136N10Na2O38S2 (M-2Na) 865.8417 found 865.8408

### Verbindung 37

11 mg Verbindung 15 und 7,9 mg N-Succinylcolchicine wurden in 1 ml abs. DMF gelöst, mit 3,77 mg HATU und 6,8 µl DIPEA versetzt und nach 10 min eingeengt. Die Reaktion wurde über RP18 gereinigt und Verseifung des Produktes erfolgte analog zu Verbindung 12.
DC-RF: 0,22 (1:3:1 EtOH:EE:HAc20%); HRMS-ESI (m/z): calcd for C102H128N8Na2O33S2 (M-2Na) 1028.4018 found 1028.4022

### N-(7-Carboxy-5,4-dithia-heptanyol)colchicine Natrium Salz

44,6 mg 3,3'-Dithiodipropionsäure in 1 ml abs. DMF wurden mit 8,9 mg HATU und 14,5 µl DIPEA versetzt, nach 10 s wurden 10 mg Deacetylcolchicine zugegeben, die Reaktion eingeengt und der Rückstand über Kieselgel (EtOH:EE:HAc20% 1:8:1) gereinigt. Der Rückstand wurde mit verd. NaHCO₃ gelöst und über RP18 von Resten 3,3'-Dithiodipropionsäure befreit.

### Verbindung 38

8,6 mg N-(7-Carboxy-5,4-dithia-heptanyol)colchicine Natrium Salz in 1ml abs. DMF wurden mit 3,4 mg HATU und 4,6 µl DIPEA versetzt, nach 10 s wurden 10 mg Verbindung 16 in 0,5 ml abs. DMF zugegeben, eingeengt und über RP18 gereinigt. DC-RF: 0,72 (EtOH:Dioxan:NH₄OH25%); HRMS-ESI (m/z): calcd for C104H132N8Na2O33S4 (M-2Na) 1074.3895 found 1074.3908

### Verbindung 39

15 mg Verbindung 28 und 9,5 mg 2-(4-Acetylphenoxy)-N-acetamidocolchicine wurden in 1 ml abs. MeOH gelöst. Nach 24 h wurde eingeengt und über Kieselgel (EtOH:Dioxan:NH₄OH25%) gereinigt. DC-RF: 0,69 ((EtOH:Dioxan:NH₄OH25%); HRMS-ESI (m/z): calcd for C112H138N10Na2O35S2 (M-2Na) 1123.4389 found 1123.4375

In Tabelle X sind die Strukturen der niedermolekularen Konjugate aufgelistet.

**Tabelle X**

| Nr. | Struktur |
|---|---|
| 35 | 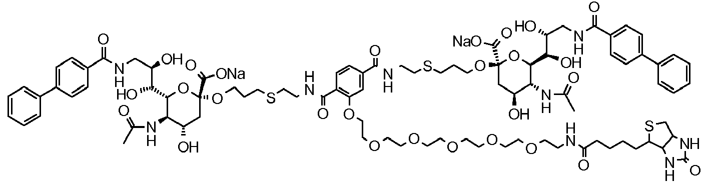 |
| 36 | 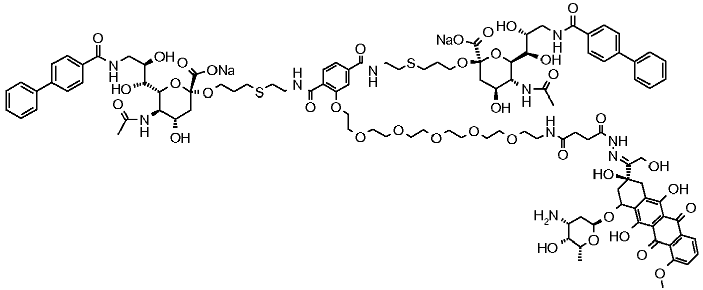 |
| 37 | 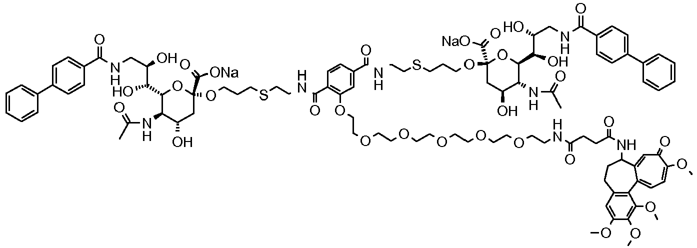 |
| 38 | 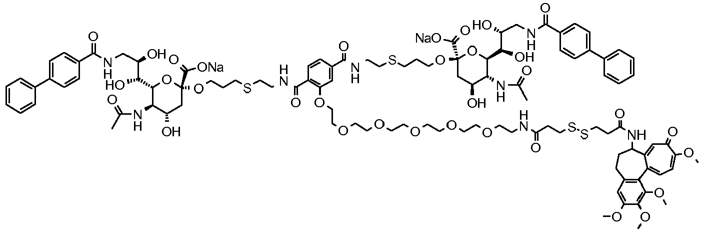 |
| 39 | 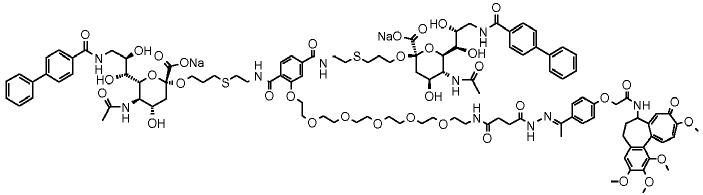 |

### Herstellung von Protein Konjugaten

### Herstellung eines Konjugates mit Albumin (Verbindung 40)

500µg Albumin wurden in 200 µl 1mM Phosphatpuffer pH8 gelöst. 24h nach Zugabe von 1,2mg Verbindung 26 wurde dialysiert.

### Herstellung von polymeren Konjugaten

Beispiele sind in Schemata 7 und 8 dargestellt.

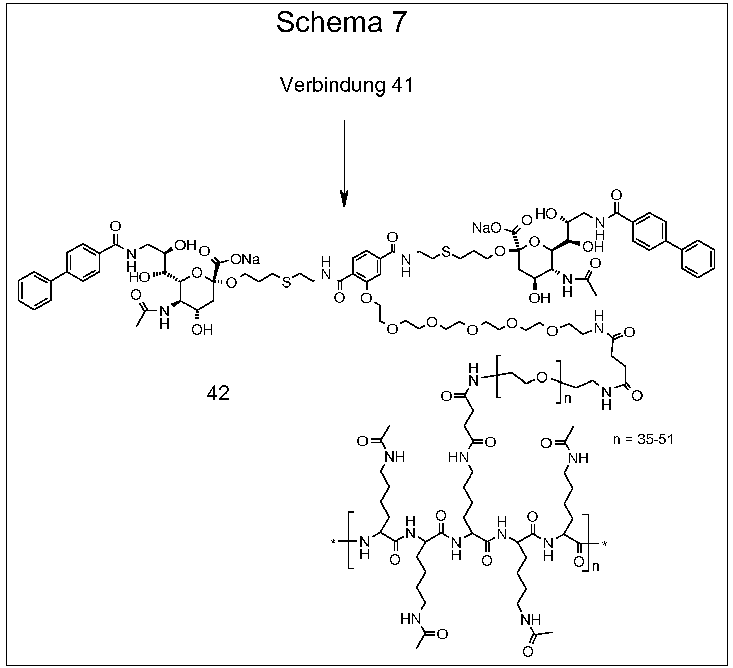

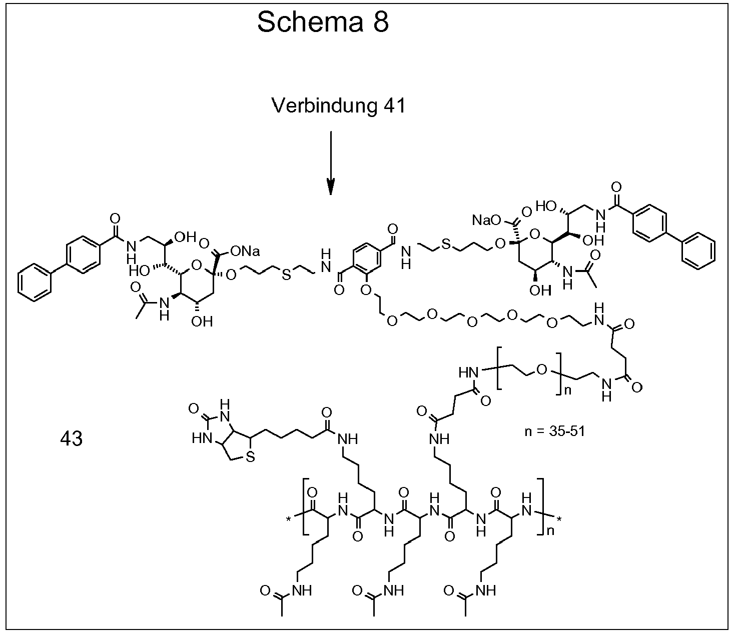

### Verbindung 42

22 mg Verbindung 41 wurden in 1 ml abs. DMF gelöst, 2,52 mg HATU und 3,1 µl DIPEA zugegeben und die Lösung direkt zur einer Lösung von 4,8 mg Poly-L-Lysin Hydrobromid (MW 300-5000) und 0,1 ml TEA in 0,5 ml DMSO gegeben. Nach 5min wurden 12,5 mg Essigsäureanhydrid zugegeben. Nach weiteren 30 min wurde das Lösungsmittel entfernt, der Rückstand mit Ethanol-Wasser-Gemisch gelöst und mit 2 M NaOH auf pH 12-13 eingestellt. Nach 17 h wurde die Lösung mit verd. HCl auf pH 7-8 neutralisiert und lyophilisiert. Der Rückstand wurde mit Wasser versetzt, über Watte filtriert, über Sephadex G25 von Salzen gereinigt und lyophilisiert. Ausbeute 11 mg (41%)

### Verbindung 43

8 mg Verbindung 41 wurden in 0,25 ml abs. DMF gelöst, 0,46 mg HATU und 1,26 µl DIPEA zugegeben und die Lösung direkt zur einer Lösung von 2,3 mg Poly-L-Lysin Hydrobromid (MW 300-5000) und 0,05 ml TEA in 0,5 ml DMSO gegeben. 0,52 mg Biotin in 0,5 ml abs. DMF wurden mit 0,90 mg HATU und 2,2 µl DIPEA versetzt und zu der Lösung gegeben. Nach 5 min wurden 12,5 mg Essigsäureanhydrid zugegeben. Nach weiteren 30 min wurde mit Wasser verdünnt und mit 2 M NaOH auf pH 12-13 eingestellt. Nach 17 h wurde die Lösung mit verd. HAc auf pH 7-8 neutralisiert und über Sephadex G25 von Salzen gereinigt und lyophilisiert. Ausbeute 7 mg (70%).

### Biologische Tests

Um die Affinität der neuen Derivate zu CD22 bestimmen zu können wurde ein neuer Test entwickelt. Für diesen Test wurde Verbindung 35 (Konjugat von einer Verbindung der Formel (I) und Biotin) hergestellt.

| | |
|---|---|
| 35 | 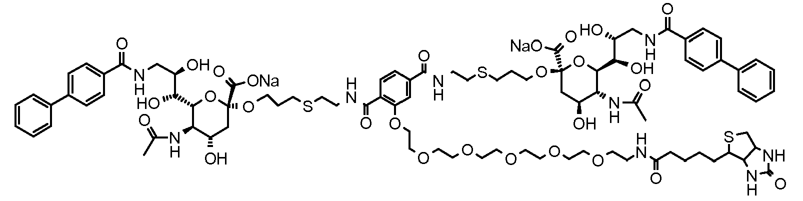 |

Zur Bestimmung der Affinität der Verbindung der Formel (I) wurden ca. 2x10⁸ Zellen der humanen B-cell-lymphoma-Linie "Daudi" mit Sialidase (A. ureafaciens) 1h bei 37°C inkubiert. Anschliessend wurde das Enzym mit 1,5mM 2,3-DehydroNeu5Ac blockiert und die Zellen zweimal mit PBS gewaschen. Je 1-2x10⁶ Zellen wurden in Probengefässe überführt und auf Eis 15 min mit seriellen Konzentrationen (10fache Verdünnungen) der zu messenden Substanz (Inhibitor) und anschliessend 20 min mit 2 µmolar Substanz 35 inkubiert. Das Reaktionsvolumen betrug 50 µl. Nach Waschen mit PBS wurde 15 min mit Streptavidin-PE im Dunkeln inkubiert, gewaschen und die gebundene Menge an Substanz 35 über Streptavidin-PE im FACS gemessen. Zur Bestimmung von 0% Verdrängung der Substanz 35 wurden Zellen ohne Inhibitor gemessen. Zur Bestimmung von 100% Verdrängung wurden Zellen ohne Substanz 35 und ohne Inhibitor gemessen. Pro Test wurden 4 Substanzen und eine Referenzsubstanz gemessen. Die bestimmten IC50-Werte jeden Testes wurden anschliessend in rIP-Werte umgerechnet. Hierzu lief die Substanz 46 als Referenz mit.

Die Literatur zeigt im Vergleich zu dem Monomeren "BPC-Neu5Ac" (J. Exp. Med. 2002, 195, 1207-1213) zwei Liganden "BPC-NeuAc-LN" und "BPA-NeuGc-LN", welche eine reaktive Gruppe zur Kopplung an ein Cargo oder einen Träger besitzen (J. of Immunology 2006, 177, 2994-3003). Ein direkter Vergleich der bekannten Substanzen mit den Verbindungen der Formel (I) war nicht möglich, da sich die IC50-Werte der beiden Substanzklassen von Test zu Test unabhängig voneinander verhalten. Der Grund für das unterschiedliche Verhalten der beiden Substanzklassen liegt in den unterschiedlichen Eigenschaften, insbesondere der Valenz.. Es wurde daher eine Referenzsubstanz mit gleicher Valenz eingeführt, die IC50 Werte der Verbindungen schwankten von Test zu Test, es war aber möglich stabile rIP-Werte zu bilden.

Tabelle II zeigt die Affinitäten von der Referenzverbindungen 45, 46 sowie von den Verbindungen BPC-Neu5Ac, BPC-NeuAc-LN, BPA-NeuGc-LN und Verbindung der Formel (I) (Verbindung 16) zu CD22 (Siglec-2).

**Tabelle II**

| | Struktur | rIP (Literatur -Assay) | rIP (Neuer Assay) |
|---|---|---|---|
| BPC Neu Ac | 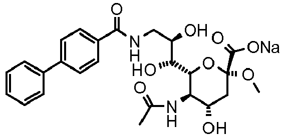 | 1 | 1 |
| BPC Neu Ac-LN | 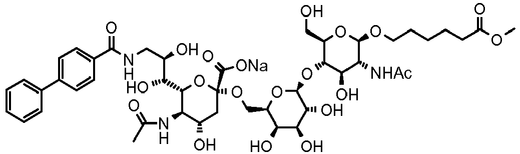 | 15,9 | - |
| BPA Neu Gc-LN | 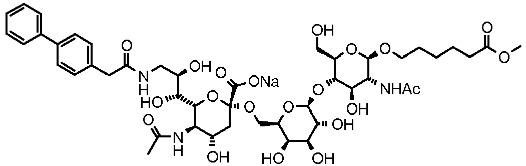 | 15,9 | - |
| 45 | 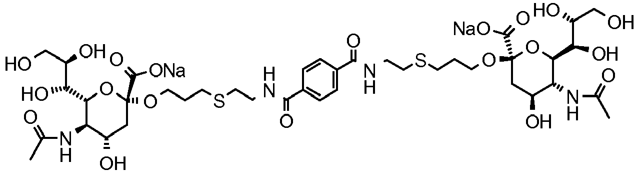 | - | ~1-3 |
| 46 | 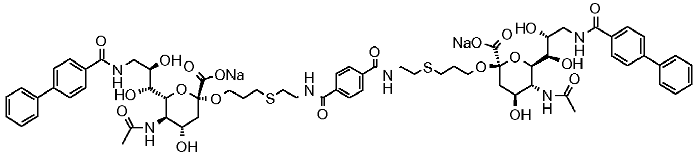 | - | > 2000 |
| 16 | 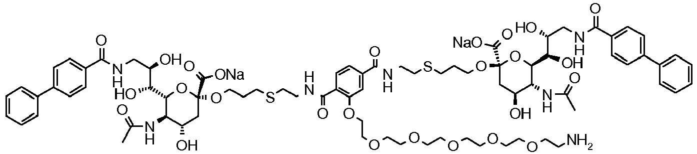 | - | >1000 |

Die rIP-Werte aller Sialinsäurederivate der Formel (I), bei denen die Affinität bestimmt werden konnte, sind in Tabelle III dargestellt. Eine Affinitätsbestimmung von einigen Verbindungen der Formel (I) ist nicht erfolgt, da für die Affinität essentielle Gruppen erst nach der Reaktion mit einem Cargo entschützt werden. Die Werte sind auf Verbindung 45 bezogen.

**Tabelle III**

| **Nr.** | **rIP** |
|---|---|
| 45 | 1 |
| 34 | 953 |
| 29 | 327 |
| 12 | 244 |
| 16 | 774 |
| 33 | 500 |
| 21 | 780 |
| 22 | 1847 |
| 25 | 864 |

In Tabelle (V) sind die rIP-Werte einiger Konjugate von Sialinsäurederivaten der Formel (I) und einem Trägermolekül beschrieben. Die Werte sind in Bezug auf Verbindung 45 gesetzt.

**Tabelle V**

| Nr. | rIP |
|---|---|
| 45 | 1 |
| 40 | 4,5x10⁵ |
| 42 | 2,1x10⁶ |

Die Verbindungen der Formel (I) können an einen polymeren Träger gekoppelt werden und können ein, an diesen Träger gekoppeltes, Cargo in CD22 exprimierende Zellen transportieren. Beispielhaft wurde das Konjugat 43 mit Polylysin als polymeren Träger und Biotin als Cargo hergestellt.

| | |
|---|---|
| 43 | 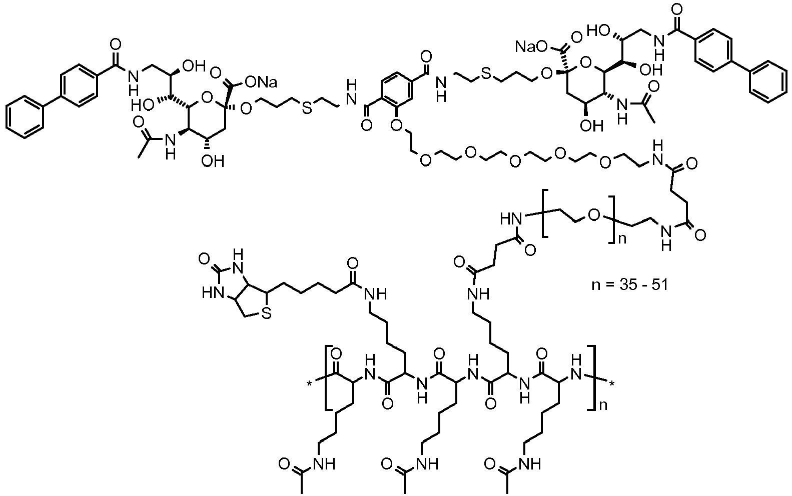 |

Die CD22 positive Zelllinie Nalm-6 und die CD22 negative Zelllinie HL-60 wurden geernted, bei 0°C mit Verbindung 43 für 1 h inkubiert. Anschließend wurde gewaschen und die Zellen in 37°C warmen RPMI medium mit 10% FCS suspendiert. Nach verschiedenen Zeitpunkten wurden Zellen entnommen und mit niedrigem pH Puffer (0.2 M Glycin HCl, pH 2.4) gewaschen, um Reste an der Zelloberfläche gebundenen Konjugats zu entfernen. Nach Fixierung mit Paraformaldehyd wurden die Zellen mit 70% MeOH permeabilisiert. Nach zweifachem Waschen mit FACS-Puffer wurde das internalisierte Konjugat mit Streptavidin-Phycoerythrin angefärbt und im FACS bestimmt. Es wurde eine Aufnahme der Verbindung 43 in CD22 positive Zellen festgestellt, in die CD22 negative Zelllinie wurde keine Verbindung aufgenommen.

## Patentansprüche

1. Verbindung der Formel (I), 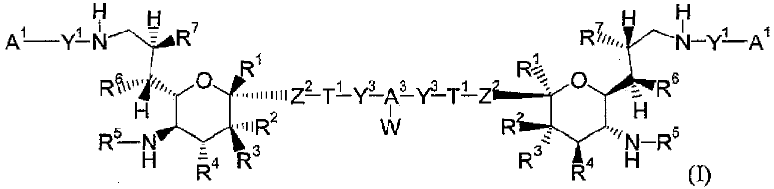 wobei die Symbole folgende Bedeutungen haben:
A¹ ist gleich 4-Biphenyl, 4-(2-Thienyl)benzoyl, 4-(3-Thienyl)benzoyl, 1-Naphtyl und 2-Naphtyl, wobei die genannten Reste unsubstituiert oder ein- oder mehrfach durch eine Gruppe X¹ substituiert sind;
X¹ ist gleich oder verschieden Fluor, Chlor, Hydroxy, Carboxy, SO₃M, OSO₃M, SO₂NH₂, SO₂CF₃, Alkyl, Cycloalkyl, Halogenalkyl, Alkyloxy, Halogenalkyloxy, Alkylcarbonyloxy, Alkylcyclocarbonyloxy, Amino-carbonyl, Alkylaminocarbonyl, Cycloalkylaminocarbonyl, Alkylcarbonyl-amino, Cycloalkylcarbonylamino, Alkylamin, Cycloalkylamino, Dialkyl-amino, Dicycloalkylamino oder Alkylcycloalkylamino wobei die Alkyl-gruppen in diesen Resten 1 bis 4 und die Cycloalkylgruppen 3 oder 4 Kohlenstoffatome enthalten;
Y¹ ist gleich ∼(C₁-C₂-Alkyl)-, ~C(O)- oder ~CH₂C(O)- wobei ~ die Bindung zur Gruppe A¹ bezeichnet;
Z² ist gleich -O-, -S- oder -CH₂-;
T¹ ist gleich eine geradkettige oder verzweigte Alkandiylgruppe mit 3 bis 10 C-Atomen, wobei
(i) gegebenenfalls eine oder mehrere nicht terminale CH₂-Gruppen durch -O- und/oder -S- ersetzt sind und/oder
(ii) gegebenenfalls ein oder mehrere H-Atome durch F und/oder Cl ersetzt sind und/oder
(iii) gegebenenfalls eine nicht terminale -CH₂CH₂- Gruppe durch-NHCO- ersetzt ist;
Y³ ist gleich -C(O)-, ∼C(O)-NH- oder ~NH-C(O)-, wobei ∼ die Bindung zu Gruppe A³ bezeichnet;
A³ ist
a) ein C₁-C₈-Alkantriyl, wobei gegebenenfalls mehrere nicht terminale CH₂-Gruppen durch O, S, S(O), S(O)₂, NR^{x} und/oder C(O) ersetzt sind und wobei in den genannten Gruppen gegebenenfalls ein oder mehrere H-Atome durch eine Gruppe X² ersetzt sind,
b) ein gesättigter, partiell ungesättigter oder aromatischer, mono- oder polycyclischer Kohlenwasserstoffrest mit 3 bis 14 C-Atomen oder ein drei- bis achtgliedriger, aromatischer, partiell ungesättigter oder gesättigter mono oder polycyclischer heterocyclischer Rest, wobei die genannten Gruppen jeweils gegebenenfalls durch eine oder mehrere Gruppen X² substituiert sind,
c) ein tertiärer Stickstoff;
X² ist gleich Fluor, Chlor, Alkyl, Halogenalkyl oder Alkyloxy wobei die Alkylgruppen in diesen Resten 1 bis 2 Kohlenstoffatome enthalten;
W ist -Y⁵-T²-V oder -V;
V ist ∼C(O)O-4-Nitrophenyl, ∼C(O)O-Pentafluorphenyl, Malein-2-yl-säureanhydrid, ~C(O)-1-Azetidin-2-on, ∼4-O-Phenyl-C(O)-1-Azetidin-2-on, ∼N=C=O, ∼N=S=O, ∼C(O)N₂, ∼S(O)₂Cl, ∼C(NH₂)OR^{y}, ∼P(CH₂OH)₃, ∼SH, ∼SC(O)CH₃, ∼NH₂, ∼OH, ~CH=CH₂, ∼C≡CH, -COOM, ∼C(O)H, ∼C(O)CH₃, ∼C(O)C(O)H, ∼I, ∼N₃, Ethyl-2-(3-Indol)amin-1∼, ∼S(O)₂N₃, Phenyl-CH=CH-C(N₂)-C(O)O∼, ∼CH=CHCH₂OC(O)R^{y}, ∼CH=CHCH₂OC(O)NHR^{y}, ~OC(O)OR^{y}, ∼C(O)NHNH₂, ∼N-Maleinimid, Aziridin-2∼, Pyridin-2-S-S∼, Phenyl-1-Carboxy-2-Nitro-5-S-S∼, ∼S(O)₂CH=CH₂, ∼C(O)-S-Phenyl, ∼C(O)CH=N₂, ∼C(O)O-N-Succinimidyl, oder C(O)O-N-Sulfosuccinimidyl;
Y⁵ ist eine Bindung, -O-, -S-, -NR^{x}-, -C(O)-, ∼C(O)-NR^{x}- oder ∼NR^{x}-C(O)-, wobei ∼ die Bindung zu Gruppe A³ bezeichnet;
T² ist eine geradkettige oder verzweigte Alkandiylgruppe mit 1 bis 200 C-Atomen, wobei
(i) gegebenenfalls eine oder mehrere nicht terminale CH₂-Gruppen durch -O-, NH und/oder -S- ersetzt sind und/oder
(ii) gegebenenfalls ein oder mehrere nicht terminale H-Atome durch F, Cl, (=O), NR^{z} und/oder NR^{y}, ersetzt sind und/oder
(iii) gegebenenfalls eine oder mehrere nicht terminale -CH₂CH₂- Gruppe durch -NHCO- oder -S-S- ersetzt ist;
R¹ ist gleich C(O)OM;
R², R³ sind gleich H;
R⁴, R⁶, R⁷ sind gleich oder verschieden OH oder OR^{z};
R⁵ ist gleich C(O)CH₃ oder C(O)CH₂OH;
M ist gleich ein C₁-C₄-Alkyl, C₃-C₄-Cycloalkyl oder ein Kation;
R^{x} ist gleich oder verschieden H, R^{y} oder R^{z};
R^{y} ist gleich oder verschieden C₁-C₄-Alkyl, C₃-C₄-Cycloalkyl, Phenyl oder Benzyl und
R^{z} ist gleich oder verschieden -C(O)-C₁-C₄-Alkyl, -C(O)-C₃-C₄-Cycloalkyl, -C(O)-Phenyl oder C(O)-CH₂-Phenyl.

2. Verbindung der Formel (I) gemäß Anspruch 1, wobei die Symbole in der Formel (I) folgende Bedeutungen haben:
A¹ ist gleich eine Gruppe 4-Biphenyl, 1-Naphtyl und 2-Naphtyl wobei die genannten Gruppen unsubstituiert oder durch eine oder mehrere Gruppen X¹ substuiert sind;
X¹ ist gleich oder verschieden Fluor, Chlor, Hydroxy, Alkyl, Halogenalkyl, Alkyloxy, Halogenalkyloxy, Alkylcarbonyloxy, wobei die Alkylgruppen in diesen Resten 1 bis 2 Kohlenstoffatome enthalten;
Y¹ ist gleich ∼C(O)- oder ∼CH₂C(O)-, wobei ∼ die Bindung zur Gruppe A¹ bezeichnet;
Z² ist gleich -O-;
T¹ ist gleich eine geradkettige oder verzweigte Alkandiylgruppe mit 4 bis 8 C-Atomen, wobei
(i) gegebenenfalls eine oder mehrere nicht terminale CH₂-Gruppen durch -O- und/oder -S- ersetzt sind und/oder
(ii) gegebenenfalls eine nicht terminale -CH₂CH₂- Gruppe durch-NHCO- ersetzt ist;
Y³ ist gleich eine Bindung, ∼C(O)-NH- oder ∼NH-C(O)-, wobei ∼ die Bindung zu Gruppe A³ bezeichnet;
A³ ist ein
a) C₁-C₅-Alkantriyl, wobei gegebenenfalls eine oder mehrere nicht terminale CH₂-Gruppen durch O, S, NR^{x} und/oder C(O) ersetzt sind und wobei in den genannten Gruppen gegebenenfalls ein oder mehrere H-Atome durch eine Gruppe X² ersetzt sind,
b) Phenylen-1,2,4-triyl oder 1*H*(1,2,3)Triazol-1,4,5-triyl, wobei die genannten Gruppen jeweils gegebenenfalls durch eine oder mehrere Gruppen X² substituiert sind,
c) tertiärer Stickstoff;
X² ist gleich Fluor, Chlor, Methyl, Methyloxy;
W ist -Y⁵-T²-V oder -V;
V ist ∼C(O)O-4-Nitrophenyl, ∼C(O)O-Pentafluorphenyl, Malein-2-yl-säureanhydrid, ∼N=C=O, ∼N=S=O, ∼C(O)N₂, ∼P(CH₂OH)₃, ∼SH, ∼NH₂, ∼OH, ∼CH=CH₂, ∼C=CH, -COOM, ∼C(O)H, ∼C(O)CH₃, ∼C(O)C(O)H, ∼I, ∼N₃, ∼C(O)NHNH₂, ∼N-Maleinimid, Aziridin-2∼, Pyridin-2-S-S∼, Phenyl-1-Carboxy-2-Nitro-5-S-S∼, ~C(O)O-N-Succinimidyl;
Y⁵ ist eine Bindung, -O-, -NH-, -C(O)-, ∼C(O)-NH- oder ∼NH-C(O)-, wobei ∼ die Bindung zu Gruppe A³ bezeichnet;
T² ist eine geradkettige oder verzweigte Alkandiylgruppe mit 1 bis 200 C-Atomen, wobei
(i) gegebenenfalls eine oder mehrere nicht terminale CH₂-Gruppen durch -O- oder -NH- ersetzt sind und/oder
(ii) gegebenenfalls ein oder mehrere nicht terminale H-Atome durch F oder (=O) ersetzt sind und/oder
(iii) gegebenenfalls eine oder mehrere nicht terminale -CH₂CH₂- Gruppe durch -NHCO- oder -S-S- ersetzt ist;
R¹ ist gleich C(O)OM;
R², R³ sind gleich H;
R⁴, R⁶, R⁷ sind gleich oder verschieden OH oder OR^{z};
R⁵ ist gleich C(O)CH₃;
M ist gleich ein C₁-C₂-Alkyl oder ein Kation;
R^{x} ist gleich oder verschieden H, R^{y} oder R^{z};
R^{y} ist gleich oder verschieden C₁-C₃-Alkyl, Cyclopropyl, Phenyl oder Benzyl und
R^{z} ist gleich oder verschieden -C(O)-C₁-C₄-Alkyl, -C(O)-Phenyl oder -C(O)-CH₂-Phenyl.

3. Verbindung gemäß Anspruch 1 oder 2, wobei die Symbole in der Formel (I) folgende Bedeutungen haben:
A¹ ist gleich 4-Biphenyl, wobei die genannte Gruppe unsubstituiert oder durch eine oder mehrere Hydroxylgruppen substuiert ist;
Y¹ ist gleich ∼C(O)-, wobei ∼ die Bindung zur Gruppe A¹ bezeichnet;
Z² ist gleich -O-;
T¹ ist gleich Hexan-1,6-diyl, wobei gegebenenfalls eine nicht terminale CH₂-Gruppen durch -S- ersetzt ist;
Y³ ist gleich ∼C(O)-NH-, wobei ∼ die Bindung zu Gruppe A³ bezeichnet;
A³ ist Propan-1,1,3-triyl oder Phenyl-1,2,4-triyl;
W ist -Y⁵-T²-V oder -V;
V ist ∼SH, ∼NH₂, ∼CH=CH₂, ∼C≡CH, ∼COOM, ∼I, ∼N₃, ∼C(O)NHNH₂, ∼N-Maleinimid, ∼C(O)O-N-Succinimidyl
Y⁵ ist eine Bindung, -O- oder ∼NHCO-, wobei ∼ die Bindung zur Gruppe A³ bezeichnet;
T² ist eine geradkettige oder verzweigte Alkandiylgruppe mit 1 bis 200 C-Atomen, wobei
(i) gegebenenfalls eine oder mehrere nicht terminale CH₂-Gruppen durch ∼O- ersetzt sind und/oder
(ii) gegebenenfals eine oder mehrere nicht terminale -CH₂CH₂-Gruppen durch -NHCO- oder -S-S- ersetzt sind;
R¹ ist gleich C(O)OM;
R², R³ sind gleich H;
R⁴, R⁶, R⁷ sind gleich OH oder OC(O)CH₃;
R⁵ ist gleich C(O)CH₃;
M ist CH₃ oder Natrium.

4. Verbindung der Formel (I) gemäß einem der Ansprüche 1 bis 3 mit der Formel (Ia) oder (Ib), wobei die Symbole die in der Formel (I) angegebenen Bedeutungen haben:

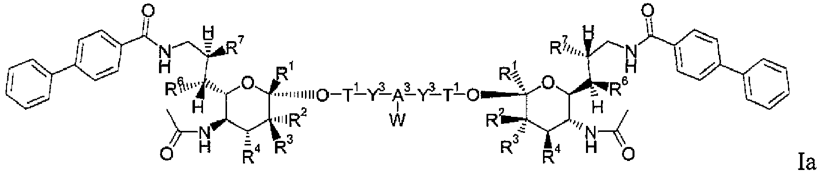

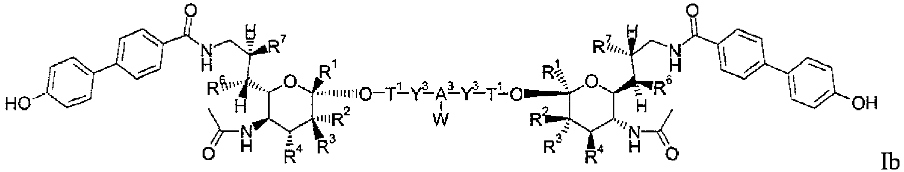

5. Verbindung der Formel (I) gemäß einem der Ansprüche 1 bis 4, ausgewählt aus der Gruppe bestehend aus:

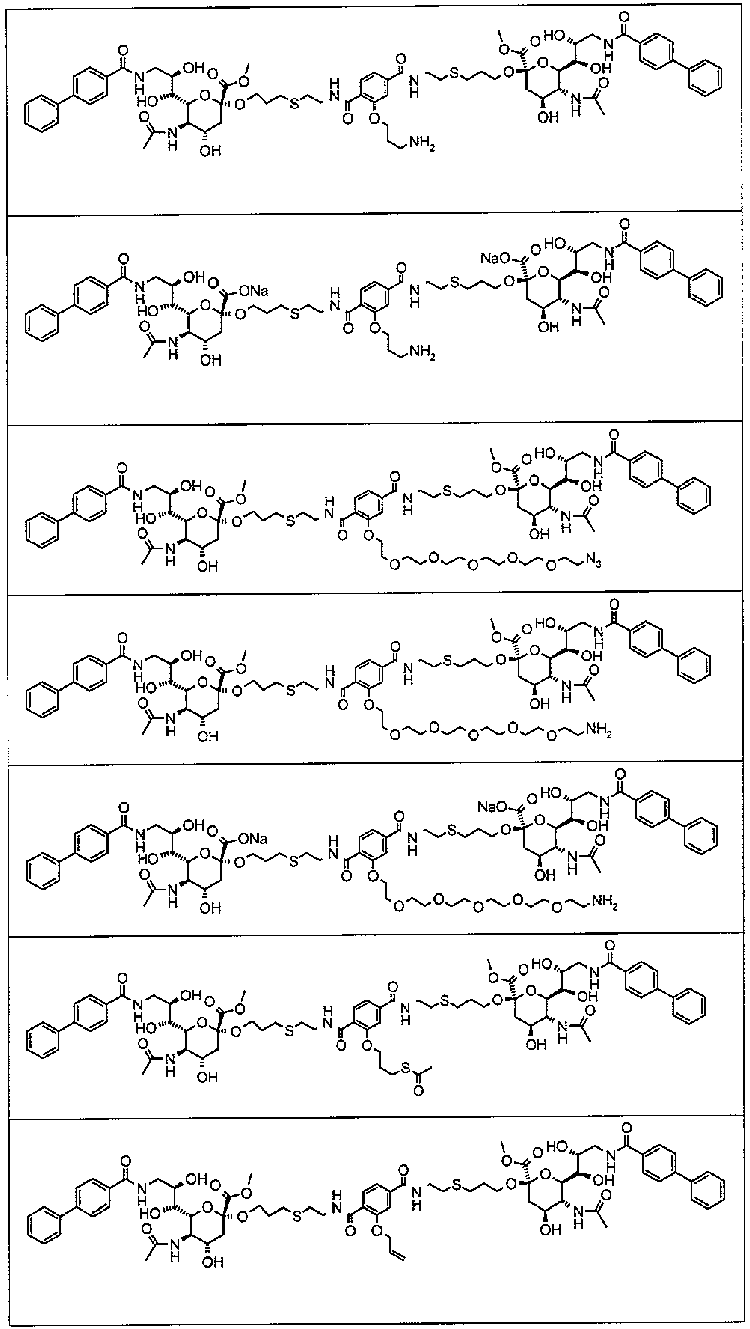

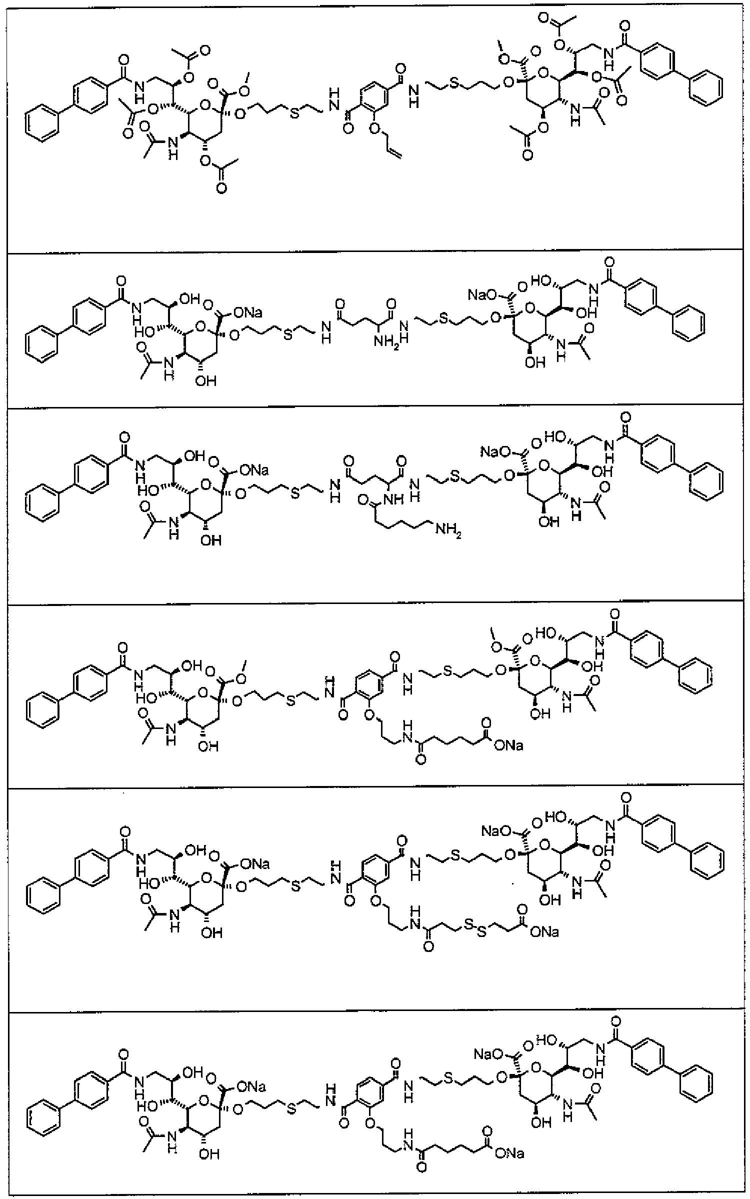

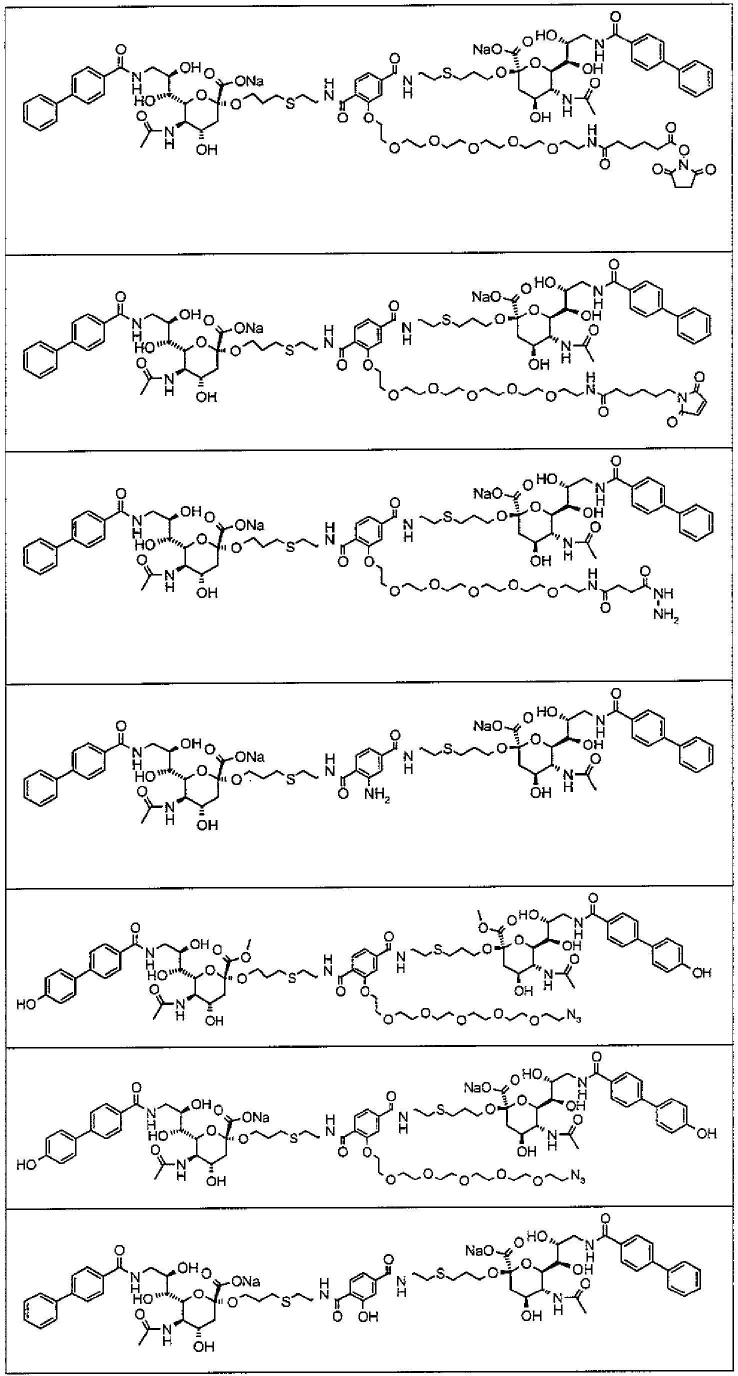

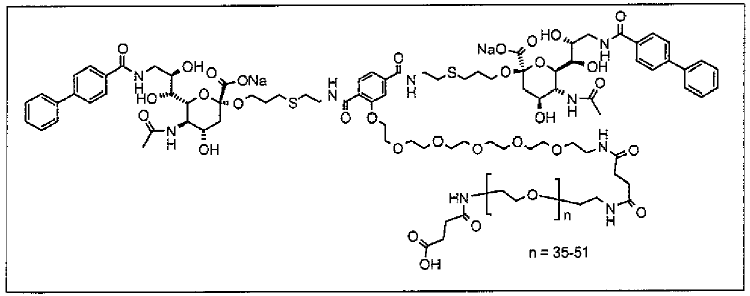

6. Konjugat, ausgewählt aus der Gruppe bestehend aus:

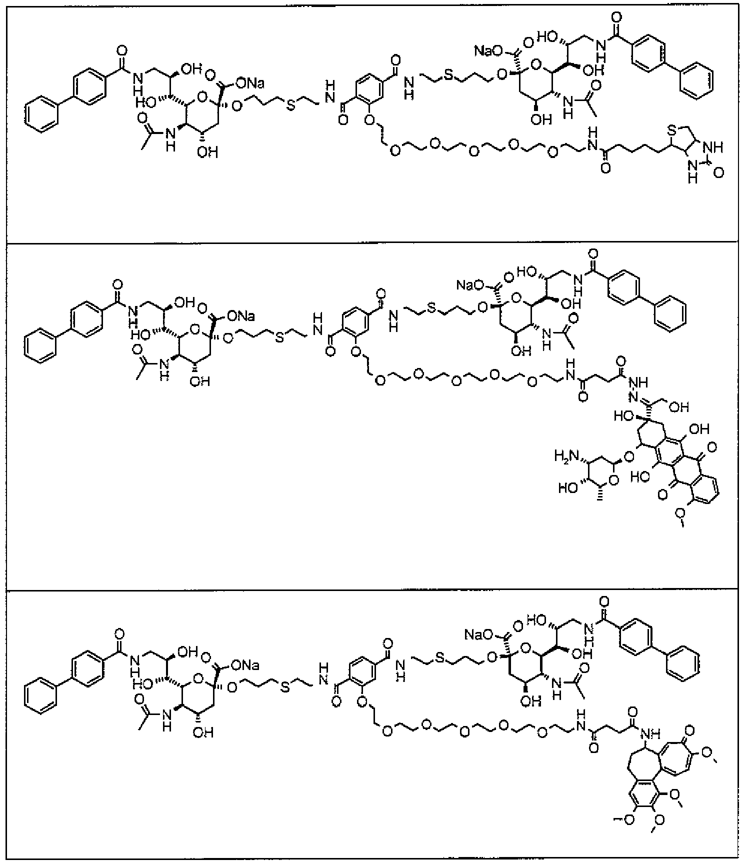

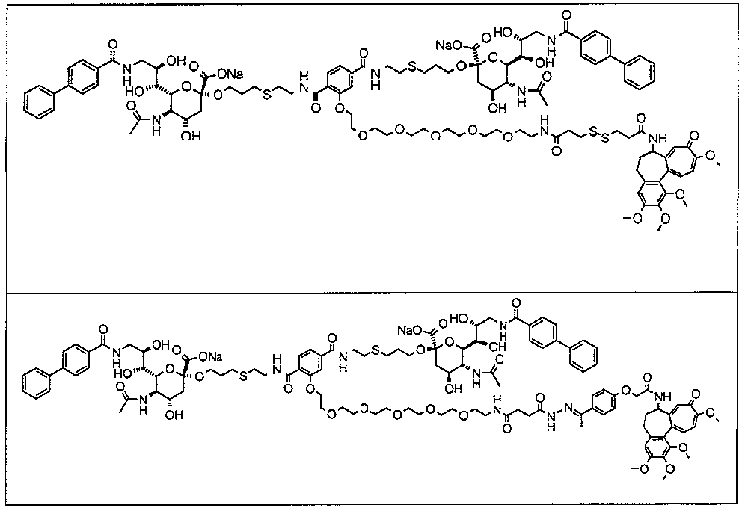

7. Pharmazeutische Zubereitung, enthaltend mindestens eine Verbindung der Formel (I) gemäß einem der Ansprüche 1 bis 5 oder ein pharmakologisch verträgliches Salz davon und einen pharmakologisch verträglichen Träger.

8. Verbindung der Formel (I) gemäß einem der Ansprüche 1 bis 5 zur Verwendung zur Herstellung eines Arzneimittels für die Behandlung bakterieller, viraler, parasitärer, Tumor-Autoimmun- und Immunschwäche-Krankheiten.

9. Verbindung der Formel (I) gemäß einem der Ansprüche 1 bis 5 zur Verknüpfung mit einem Cargo, ausgewählt aus der Gruppe bestehend aus RNA, DNA, Peptiden, niedermolekularen Antigenen, antigenen Proteinen, Enzymen und niedermolekularen pharmakologisch wirksamen Substanzen, zur Verwendung in einem Verfahren zur Regulierung von Stoffwechselvorgängen, Immunreaktionen, Immunisierungen oder Desensibilisierungen des Zielorganismus.

10. Pharmakologisch wirksames Konjugat einer Verbindung der Formel (I) gemäß einem der Ansprüche 1 bis 5 und eines Cargo, ausgewählt aus der Gruppe bestehend aus RNA, DNA, Peptiden, Zytostatika, Enzymen, metallorganischen Komplexen und niedermolekularen pharmakologisch wirksamen Substanzen, zur Verwendung in einem Verfahren zur Behandlung von Infektionen, Tumorerkrankungen oder Allergien.

11. Verwendung einer Verbindung der Formel (I) gemäß einem der Ansprüche 1 bis 5 zur Verknüpfung mit Liposomen, Nanopartikeln, metallorganischen Komplexen, Metallnanopartikeln, Micromicellen und Kohlenstoffnanoröhrchen.

12. Verwendung einer Verbindung der Formel (I) gemäß einem der Ansprüche 1 bis 5 zur Herstellung von Liposomen, Nanopartikeln, Micromicellen und Kohlenstoffnanoröhrchen.

13. Verwendung einer Verbindung der Formel (I) gemäß einem der Ansprüche 1 bis 5 zur Verknüpfung mit Molekülen zu diagnostischen Zwecken.

14. Verwendung einer Verbindung der Formel (I) gemäß einem der Ansprüche 1 bis 5 zur Herstellung von polyvalenten Liganden.

15. Verwendung einer Verbindung der Formel (I) gemäß einem der Ansprüche 1 bis 5 zur Verknüpfung mit einem Cargo tragenden Polymer, wobei das Cargo ausgewählt ist aus der Gruppe bestehend aus RNA, DNA, Zytostatika, Peptiden, niedermolekularen Antigenen, antigenen Proteinen, Enzymen, metallorganischen Komplexen und niedermolekularen pharmakologisch wirksamen Substanzen,

## Claims

1. Compound of the formula (I), 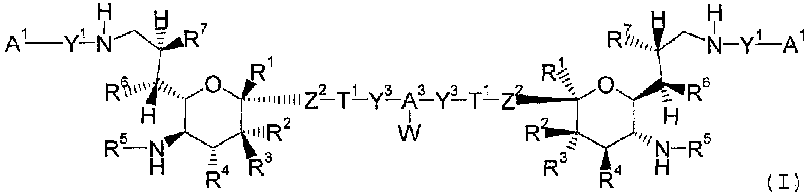 where the symbols are defined as follows:
A¹ is equal to 4-biphenyl, 4-(2-thienyl)benzoyl, 4-(3-thienyl)benzoyl, 1-naphthyl and 2-naphthyl, in which the residues mentioned are unsubstituted or mono- or polysubstituted by a group X¹;
X¹ is identically or differently fluorine, chlorine, hydroxyl, carboxy, SO₃M, OSO₃M, SO₂NH₂, SO₂CF₃, alkyl, cycloalkyl, haloalkyl, alkyloxy, haloalkyloxy, alkylcarbonyloxy, alkylcyclocarbonyloxy, aminocarbonyl, alkylaminocarbonyl, cycloalkylaminocarbonyl, alkylcarbonylamino, cycloalkylcarbonylamino, alkylamino, cycloalkylamino, dialkylamino, dicycloalkylamino or alkylcycloalkylamino, in which the alkyl groups in these residues comprise 1 to 4, and the cycloalkyl groups 3 or 4, carbon atoms;
Y¹ is equal to ∼ (C₁-C₂-alkyl) -, ∼C(O)- or ∼CH₂C(O)-, in which - denotes the bond to the group A¹;
Z² is equal to -O-, -S- or -CH₂-;
T¹ is equal to a straight-chain or branched alkanediyl group having 3 to 10 C atoms, in which
(i) optionally one or more non-terminal CH₂ groups are replaced by -O- and/or -S-and/or
(ii) optionally one or more H atoms are replaced by F and/or Cl and/or
(iii)optionally one non-terminal -CH₂CH₂-group is replaced by -NHCO-;
Y³ is equal to -C(O)-, ∼C(O)-NH- or ∼NH-C(O)-, in which - denotes the bond to group A³;
A³ is
a) a C₁-C₈-alkanetriyl, in which optionally more than one non-terminal CH₂ groups are replaced by 0, S, S(O), S(O)₂, NR^{x} and/or C(O) and wherein optionally one or more H atoms in the groups mentioned are replaced by a group X²,
b) a saturated, partially unsaturated or aromatic, mono- or polycyclic hydrocarbon residue having 3 to 14 C atoms or one three- to eight-membered, aromatic, partially unsaturated or saturated mono- or polycyclic heterocyclic residue, in which the groups mentioned are optionally substituted in each case by one or more groups X²,
c) a tertiary nitrogen;
X² is equal to fluorine, chlorine, alkyl, haloalkyl or alkyloxy, in which the alkyl groups in these residues comprise 1 to 2 carbon atoms;
W is -Y⁵-T²-V or -V;
V is ∼C(O)O-4-nitrophenyl, ∼C(O)C-pentafluorophenyl, maleic-2-yl anhydride, ~C(O)-1-azetidin-2-one, ~4-O-phenyl-C(O)-1-azetidin-2-one, ∼N=C=O, ∼N=S=O, ~C(O)N₂, ∼S(O)₂Cl, ~C(NH₂)OR^{y}, ∼P(CH₂OH)₃, ∼SH, ~SC(O)CH₃, ∼NH₂, ∼OH, ~CH=CH₂, ∼C≡CH, ∼COOM, ~C(O)H, ~C(O)CH₃, ~C(O)C(O)H, ~I, ~N₃, ethyl-2-(3-indol)amine-1~, ~S(O)₂N₃, phenyl-CH=CH-C(N₂)-C(O)O∼, ~CH=CHCH₂OC(O)R^{y}, ~CH=CHCH₂OC(O)NHR^{y}, ∼OC(O)OR^{y}, ∼C(O)NHNH₂, ∼N-maleimide, aziridine-2∼, pyridine-2-S-S~, phenyl-1-carboxy-2-nitro-5-S-S~, ~S(O)₂CH=CH₂, ~C(O)-S-phenyl, ∼C(O)CH=N₂, ∼C(C)O-N-succinimidyl, or C(O)O-N-sulphosuccinimidyl;
Y⁵ is a bond, -O-, -S-, -NR^{x}-, -C(O)-, -C(O)-NR^{x}-or ∼NR^{x}-C(O)-, in which - denotes the bond to group A³;
T² is a straight-chain or branched alkanediyl group having 1 to 200 C atoms, in which
(i) optionally one or more non-terminal CH₂ groups are replaced by -0-, NH and/or-S- and/or
(ii) optionally one or more non-terminal H atoms are replaced by F, Cl, (=O), NR^{z} and/or NR^{y} and/or
(iii)optionally one or more non-terminal-CH₂CH₂- group is replaced by -NHCO- or-S-S-;
R¹ is equal to C(O)OM;
R², R³ are equal to H;
R⁴, R⁶, R⁷ are identically or differently OH or OR^{z};
R⁵ is equal to C(O)CH₃ or C(O)CH₂OH;
M is equal to a C₁-C₄-alkyl, C₃-C₄-cycloalkyl or a cation;
R^{x} is identically or differently H, R^{y} or R^{z};
R^{y} is identically or differently C₁-C₄-alkyl, C₃-C₄-cycloalkyl, phenyl or benzyl and
R^{z} is identically or differently -C(O)-C₁-C₄-alkyl, -C(O)-C₃-C₄-cycloalkyl, -C(O)-phenyl or C(O)-CH₂-phenyl.

2. Compound of the formula (I) according to Claim 1, where the symbols in the formula (I) are defined as follows:
A¹ is equal to a group 4-biphenyl, 1-naphthyl and 2-naphthyl, in which the groups mentioned are unsubstituted or are substituted by one or more groups X¹;
X¹ is identically or differently fluorine, chlorine, hydroxyl, alkyl, haloalkyl, alkyloxy, haloalkyloxy, alkylcarbonyloxy, in which the alkyl groups in these residues comprise 1 to 2 carbon atoms;
Y¹ is equal to -C(O)- or ∼CH₂C(O)-, in which ∼ denotes the bond to group A¹;
Z² is equal to -0-;
T¹ is equal to a straight-chain or branched alkanediyl group having 4 to 8 C atoms, in which
(i) optionally one or more non-terminal CH₂ groups are replaced by -O- and/or -S-and/or
(ii) optionally one non-terminal -CH₂CH₂-group is replaced by -NHCO-;
Y³ is equal to a bond, ∼C(O)-NH- or ∼NH-C(O)-, in which - denotes the bond to group A³;
A³ is a
a) C₁-C₅-alkanetriyl, in which optionally one or more non-terminal CH₂ groups are replaced by 0, S, NR^{x} and/or C(O) and wherein optionally one or more H atoms in the groups mentioned are replaced by a group X²,
b) phenylene-1,2,4-triyl or 1H(1,2,3)triazole-1,4,5-triyl, in which the groups mentioned are in each case optionally substituted by one or more groups X²,
c) tertiary nitrogen;
X² is equal to fluorine, chlorine, methyl, methyloxy;
W is -Y⁵-T²-V or -V;
V is ∼C(O)O-4-nitrophenyl, ∼C(O)O-pentafluorophenyl, maleic-2-yl anhydride, ∼N=C=O, ∼N=S=O, ∼C(O)N₂, ~P(CH₂OH)₃, ∼SH, ~NH₂, ∼OH, ∼CH=CH₂, ~C≡CH, ∼COOM, ∼C(O)H, ~C(O)CH₃, ∼C(O)C(O)H, ∼I, ∼N₃, ∼C(O)NHNH₂, ∼N-maleimide, aziridine-2~, pyridine-2-S-S~, phenyl-1-carboxy-2-nitro-5-S-S∼, ∼C(O)O-N-succinimidyl;
Y⁵ is a bond, -O-, -NH-, -C(O)-, ∼C(O)-NH- or ∼NH-C(O)-, in which ∼ denotes the bond to group A³;
T² is a straight-chain or branched alkanediyl group having 1 to 200 C atoms, in which
(i) optionally one or more non-terminal CH₂ groups are replaced by -O- or -NH-and/or
(ii) optionally one or more non-terminal H atoms are replaced by F or (=O) and/or
(iii) optionally one or more non-terminal -CH₂CH₂- group is replaced by-NHCO- or -S-S-;
R¹ is equal to C(O)OM;
R², R³ are equal to H;
R⁴, R⁶, R⁷ are identically or differently OH or OR^{z};
R⁵ is equal to C(O)CH₃;
M is equal to a C₁-C₂-alkyl or a cation;
R^{x} is identically or differently H, R^{y} or R^{z};
R^{y} is identically or differently C₁-C₃-alkyl, cyclopropyl, phenyl or benzyl and
R^{z} is identically or differently -C(O)-C₁-C₄-alkyl, -C(O)-phenyl or -C(O)-CH₂-phenyl.

3. Compound according to Claim 1 or 2, where the symbols in the formula (I) are defined as follows:
A¹ is equal to 4-biphenyl, in which the group mentioned is unsubstituted or is substituted by one or more hydroxyl groups;
Y¹ is equal to -C(O)-, in which ∼ denotes the bond to group A¹;
Z² is equal to -O-;
T¹ is equal to hexane-1,6-diyl, in which optionally one non-terminal CH₂ group is replaced by -S-;
Y³ is equal to ∼C(O)-NH-, in which - denotes the bond to group A³;
A³ is propane-1, 1, 3-triyl or phenyl-1, 2, 4-triyl;
W is -Y⁵-T²-V or -V;
V is ∼SH, ∼NH₂, ∼CH=CH₂, ∼C≡CH, ∼COOM, ∼I, ∼N₃, ∼C(O)NHNH₂, ∼N-maleimide, ∼C(O)O-N-succinimidyl;
Y⁵ is a bond, -O- or ∼NHCO-, in which - denotes the bond to group A³;
T² is a straight-chain or branched alkanediyl group having 1 to 200 C atoms, in which
(i) optionally one or more non-terminal CH₂ groups are replaced by ∼O- and/or
(ii) optionally one or more non-terminal-CH₂CH₂- groups are replaced by -NHCO- or -S-S-;
R¹ is equal to C(O)OM;
R², R³ are equal to H;
R⁴, R⁶, R⁷ are equal to OH or OC(O)CH₃;
R⁵ is equal to C(O)CH₃;
M is CH₃ or sodium.

4. Compound of the formula (I) according to any of Claims 1 to 3 having the formula (Ia) or (Ib), in which the symbols are as defined in the formula (I) :

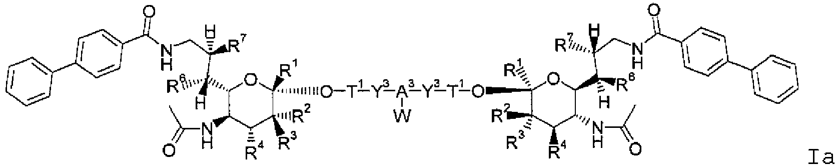

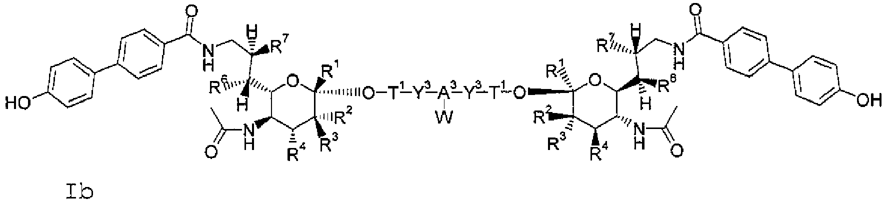

5. Compound of the formula (I) according to any of Claims 1 to 4, selected from the group consisting of:

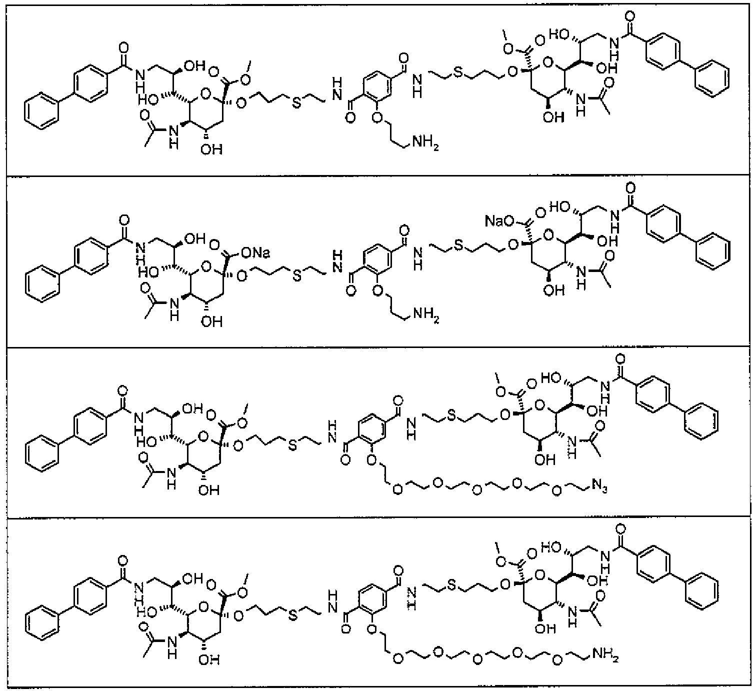

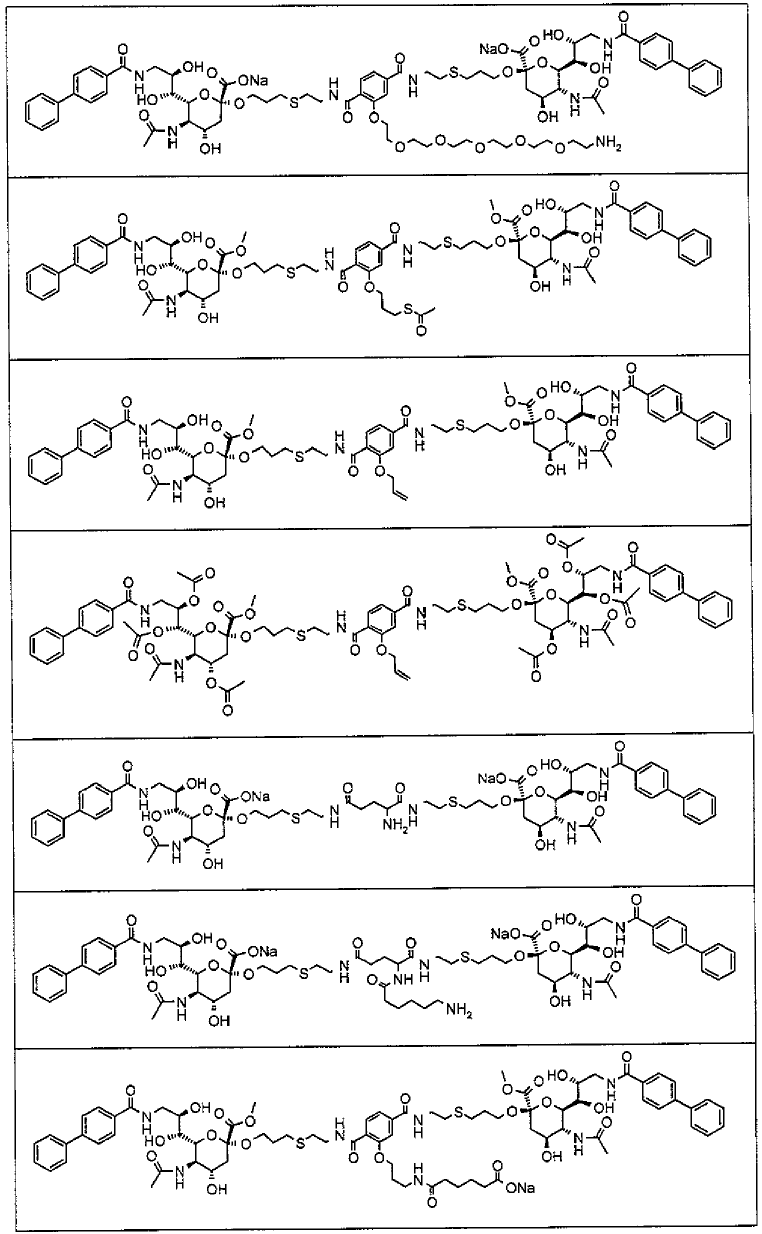

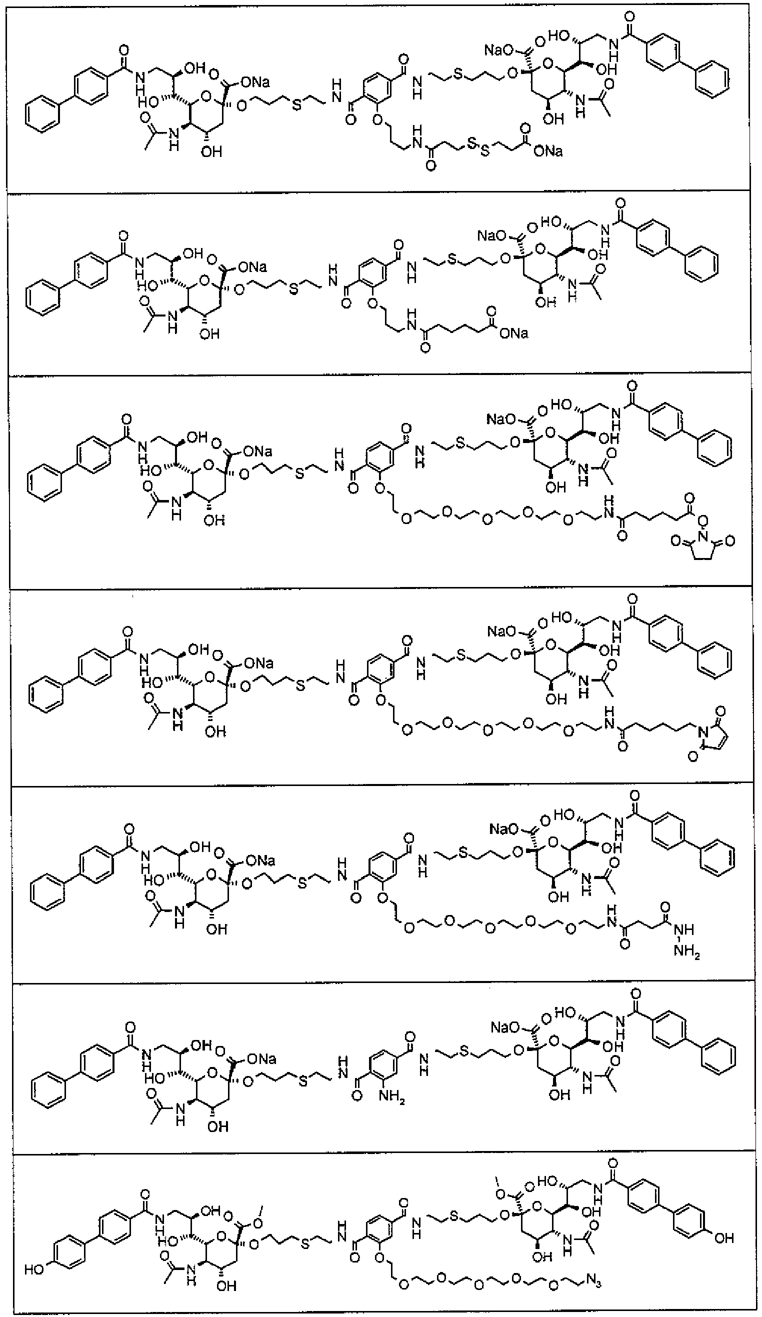

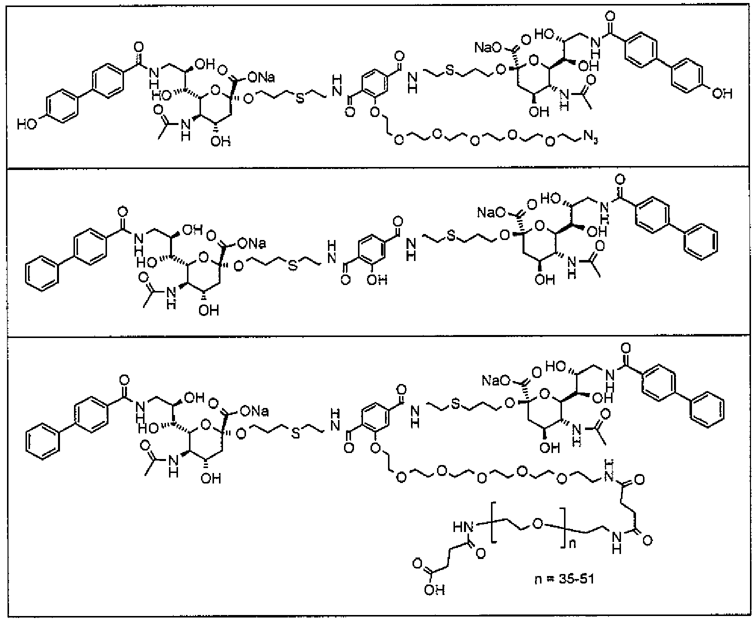

6. Conjugate, selected from the group consisting of:

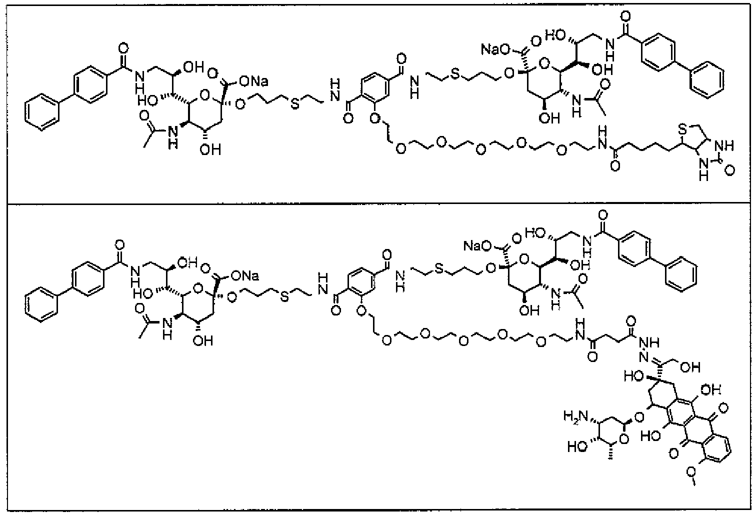

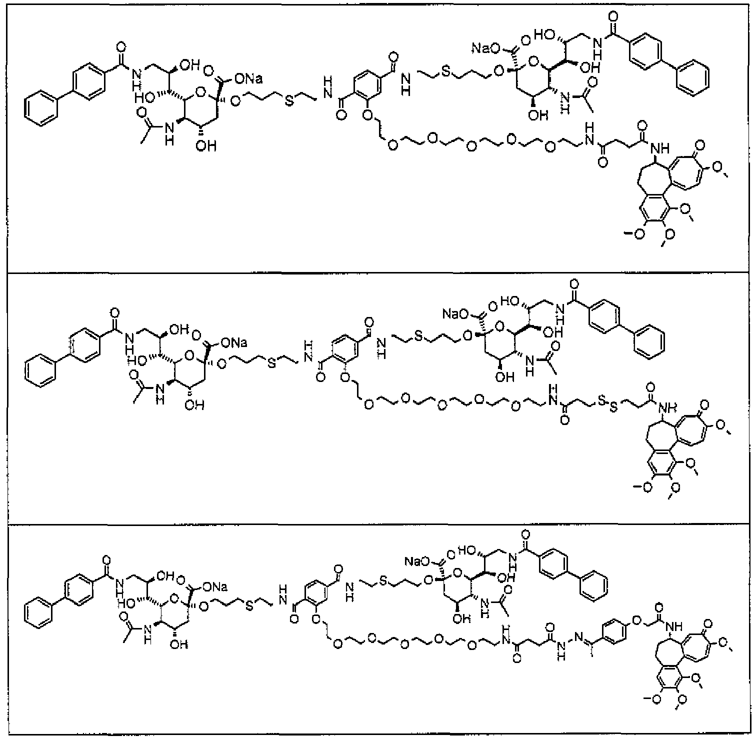

7. Pharmaceutical formulation comprising at least one compound of the formula (I) according to any of Claims 1 to 5 or a pharmacologically acceptable salt thereof and a pharmacologically acceptable carrier.

8. Compound of the formula (I) according to any of Claims 1 to 5 for use in the preparation of a medicament for the treatment of bacterial, viral, parasitic, tumour autoimmune and immunodeficiency diseases.

9. Compound of the formula (I) according to any of Claims 1 to 5 for linking with a cargo, selected from the group consisting of RNA, DNA, peptides, low molecular weight antigens, antigenic proteins, enzymes and low molecular weight pharmacologically active substances, for use in a method for regulating metabolic processes, immune reactions, immunizations or desensitizations of the target organism.

10. Pharmacologically active conjugate of a compound of the formula (I) according to any of Claims 1 to 5 and of a cargo, selected from the group consisting of RNA, DNA, peptides, cytostatics, enzymes, organometallic complexes and low molecular weight pharmacologically active substances, for use in a method for the treatment of infections, tumours or allergies.

11. Use of a compound of the formula (I) according to any of Claims 1 tc 5 for linking with liposomes, nanoparticles, organometallic complexes, metal nanoparticles, micromicelles and carbon nanotubules.

12. Use of a compound of the formula (I) according to any of Claims 1 to 5 for preparing liposomes, nanoparticles, micromicelles and carbon nanotubules.

13. Use of a compound of the formula (I) according to any of Claims 1 to 5 for linking with molecules for diagnostic purposes.

14. Use of a compound of the formula (I) according to any of Claims 1 to 5 for preparing polyvalent ligands.

15. Use of a compound of the formula (I) according to any of Claims 1 to 5 for linking with a cargo-bearing polymer, wherein the cargo is selected from the group consisting of RNA, DNA, cytostatics, peptides, low molecular weight antigens, antigenic proteins, enzymes, organometallic complexes and low molecular weight pharmacologically active substances.

## Revendications

1. Composé de formule (I), 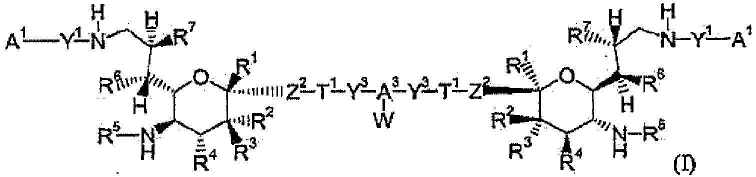 dans laquelle les symboles présentent les significations suivantes :
A¹ représente 4-biphényle, 4-(2-thiényl)benzoyle, 4-(3-thiényl)benzoyle, 1-naphtyle et 2-naphtyle, les radicaux mentionnés étant non substitués ou monosubstitués ou polysubstitués par un groupe X¹ ;
X¹ identique ou différent, représente fluor, chlore, hydroxy, carboxy, SO₃M, OSO₃M, SO₂NH₂, SO₂CF₃, alkyle, cycloalkyle, halogénoalkyle, alkyloxy, halogénoalkyloxy, alkylcarbonyloxy, alkylcyclocarbonyloxy, aminocarbonyle, alkylaminocarbonyle, cycloalkylaminocarbonyle, alkylcarbonylamino, cycloalkylcarbonylamino, alkylamino, cycloalkylamino, dialkylamino, dicycloalkylamino ou alkylcycloalkylamino, les groupes alkyle dans ces radicaux contenant 1 à 4 atomes de carbone et les groupes cycloalkyle contenant 3 ou 4 atomes de carbone ;
Y¹ représente ∼(C₁-C₂-alkyl)-, ∼C(O)- ou ∼CH₂C(O)-, ∼ désignant la liaison au groupe A¹ ;
Z² représente -0-, -S- ou -CH₂- ;
T¹ représente un groupe alcanediyle linéaire ou ramifié, comprenant 3 à 10 atomes de carbone,
(i) un ou plusieurs groupes CH₂ non terminaux étant le cas échéant remplacés par -O- et/ou -S- et/ou
(ii) un ou plusieurs atomes d'H étant le cas échéant remplacés par F et/ou Cl et/ou
(iii) un groupe -CH₂CH₂- non terminal étant le cas échéant remplacé par -NHCO-;
Y³ représente -C(O)-, ∼C(O)-NH- ou ∼NH-C(O)-, ∼ désignant la liaison au groupe A³ ;
A³ représente
a) C₁-C₈-alcanetriyle, plusieurs groupes CH₂ non terminaux étant le cas échéant remplacés par O, S, S(O), S(O)₂, NR^{x} et/ou C(O) et un ou plusieurs atomes d'H étant le cas échéant remplacés par un groupe X² dans les groupes mentionnés
b) un radical hydrocarboné, saturé, partiellement insaturé ou aromatique, monocyclique ou polycyclique, comprenant 3 à 14 atomes de carbone ou un radical hétérocyclique de trois à huit chaînons, aromatique, partiellement insaturé ou saturé, monocyclique ou polycyclique, les groupes mentionnés étant le cas échéant à chaque fois substitués par un ou plusieurs groupes X²,
c) un azote tertiaire ;
X² représente fluor, chlore, alkyle, halogénoalkyle ou alkyloxy, les groupes alkyle dans ces radicaux contenant 1 à 2 atomes de carbone ;
W représente -Y⁵-T²-V ou -V ;
V représente ∼C(O)O-4-nitrophényle, ∼C(O)O-pentafluorophényle, anhydride de l'acide maléin-2-ylique, ∼C(O)-1-azétidin-2-one, ∼4-O-phényl-C(O)-1-azétidin-2-one, ∼N=C=O, ∼N=S=O, ~C(O)N₂, ∼S(O)₂Cl, ~C(NH₂)OR^{y}, ∼P(CH₂OH)₃, ~SH, ~SC(O)CH₃, ∼NH₂, ∼OH, ∼CH=CH₂, ∼C≡CH, ∼COOM, ~C(O)H, ~C(O)CH₃, ∼C(O)C(O)H, ∼I, ∼N₃, éthyl-2-(3-indol)amino-1~, ∼S(O)₂N₃, phényl-CH=CH-C(N₂)-C(O)O∼, ∼CH=CHCH₂OC(O)R^{y}, ∼CH=CHCH₂OC(O)NHR^{y}, ∼OC(O)OR^{y}, ~C(O)NHNH₂, ∼N-maléinimide, aziridin-2∼, pyridine-2-S-S∼, phényl-1-carboxy-2-nitro-5-S-S∼, ∼S(O)₂CH=CH₂, ∼C(O)-S-phényle, ~C(O)CH=N₂, ∼C(O)O-N-succinimidyle ou C(O)O-N-sulfosuccinimidyle ;
Y⁵ représente une liaison, -O-, -S-, -NR^{x}-, -C(O)-, ∼C(O)-NR^{x}- ou ∼NR^{x}-C(O)-, ∼ désignant la liaison au groupe A³ ;
T² représente un groupe alcanediyle linéaire ou ramifié, comprenant 1 à 200 atomes de carbone,
(i) un ou plusieurs groupes CH₂ non terminaux étant le cas échéant remplacés par -O-, NH et/ou -S- et/ou
(ii) un ou plusieurs atomes d'H non terminaux étant le cas échéant remplacés par F, Cl, (=O), NR^{z} et/ou NR^{y} et/ou
(iii) un ou plusieurs groupes -CH₂CH₂- non terminaux étant le cas échéant remplacés par -NHCO- ou -S-S- ;
R¹ représente C(O)OM ;
R², R³ représentent H ;
R⁴, R⁶, R⁷ identiques ou différents, représentent OH ou OR^{z} ;
R⁵ représente C(O)CH₃ ou C(O)CH₂OH ;
M représente C₁-C₄-alkyle, C₃-C₄-cycloalkyle ou un cation ;
R^{x} identique ou différent, représente H, R^{y} ou R^{z} ;
R^{y} identique ou différent, représente C₁-C₄-alkyle, C₃-C₄-cycloalkyle, phényle ou benzyle et
R^{z} identique ou différent, représente -C(O)-C₁-C₄-alkyle, -C(O)-C₃-C₄-cycloalkyle, -C(O)-phényle ou C(O)-CH₂-phényle.

2. Composé de formule (I) selon la revendication 1, les symboles dans la formule (I) présentant les significations suivantes :
A¹ représente un groupe 4-biphényle, 1-naphtyle et 2-naphtyle, les groupes mentionnés étant non substitués ou substitués par un ou plusieurs groupes X¹ ;
X¹ identique ou différent, représente fluor, chlore, hydroxy, alkyle, halogénoalkyle, alkyloxy, halogénoalkyloxy, alkylcarbonyloxy, les groupes alkyle dans ces radicaux contenant 1 à 2 atomes de carbone ;
Y¹ représente ∼C(O)- ou -CH₂C(O)-, ∼ désignant la liaison au groupe A¹ ;
Z² représente -0- ;
T¹ représente un groupe alcanediyle linéaire ou ramifié, comprenant 4 à 8 atomes de carbone,
(i) un ou plusieurs groupes CH₂ non terminaux étant le cas échéant remplacés par -O- et/ou -S- et/ou
(ii) un groupe -CH₂CH₂- non terminal étant le cas échéant remplacé par -NHCO-;
Y³ représente une liaison, ∼C(O)-NH- ou ∼NH-C(O)-, ∼ désignant la liaison au groupe A³ ;
A³ représente
a) C₁-C₅-alcanetriyle, un ou plusieurs groupes CH₂ non terminaux étant le cas échéant remplacés par 0, S, NR^{x} et/ou C(O) et un ou plusieurs atomes d'H étant le cas échéant remplacés par un groupe X² dans les groupes mentionnés,
b) phénylène-1,2,4-triyle ou 1H(1,2,3)triazol-1,4,5-triyle, les groupes mentionnés étant le cas échéant substitués par un ou plusieurs groupes X²,
c) un azote tertiaire ;
X² représente fluor, chlore, méthyle, méthyloxy ;
W représente -Y⁵-T²-V ou -V ;
V représente ∼C(O)O-4-nitrophényle, ∼C(O)O-pentafluorophényle, anhydride de l'acide maléin-2-ylique, ∼N=C=O, ∼N=S=O, ∼C(O)N₂, ∼P(CH₂OH)₃, ∼SH, ∼NH₂, ∼OH, ∼CH=CH₂, ∼C≡CH, ∼COOM, ∼C(O)H, ∼C(O)CH₃, ∼C(O)C(O)H, ∼I, ∼N₃, ∼C(O)NHNH₂, ∼N-maléinimide, aziridin-2∼, pyridine-2-S-S∼, phényl-1-carboxy-2-nitro-5-S-S∼, ∼C(O)O-N-succinimidyle ;
Y⁵ représente une liaison, -0-, -NH-, -C(O)-, ∼OC(O)-NH- ou ∼NH-C(O) -, ∼ désignant la liaison au groupe A³ ;
T² représente un groupe alcanediyle linéaire ou ramifié, comprenant 1 à 200 atomes de carbone,
(i) un ou plusieurs groupes CH₂ non terminaux étant le cas échéant remplacés par -O- ou -NH- et/ou
(ii) un ou plusieurs atomes d'H non terminaux étant le cas échéant remplacés par F ou (=O) et/ou
(iii) un ou plusieurs groupes -CH₂CH₂- non terminaux étant le cas échéant remplacés par -NHCO- ou -S-S- ;
R¹ représente C(O)OM ;
R², R³ représentent H ;
R⁴, R⁶, R⁷ identiques ou différents, représentent OH ou OR² ;
R⁵ représente C(O)CH₃ ;
M représente C₁-C₂-alkyle ou un cation ;
R^{x} identique ou différent, représente H, R^{y} ou R² ;
R^{Y} identique ou différent, représente C₁-C₃-alkyle, cyclopropyle, phényle ou benzyle et
R^{z} identique ou différent, représente -C(O)-C₁-C₄-alkyle, -C(O)-phényle ou C(O)-CH₂-phényle.

3. Composé selon la revendication 1 ou 2, les symboles dans la formule (I) présentant les significations suivantes :
A¹ représente 4-biphényle, le groupe mentionné étant non substitué ou substitué par un ou plusieurs groupes hydroxyle ;
Y¹ représente ∼C(O)-, ∼ désignant la liaison au groupe A¹ _{;}
Z² représente -O- ;
T¹ représente hexane-1,6-diyle, un groupe CH₂ non terminal étant le cas échéant remplacé par -S- ;
Y³ représente ∼C(O)-NH-, ∼ désignant la liaison au groupe A³ _{;}
A³ représente propane-1,1,3-triyle ou phényl-1,2,4-triyle ;
W représente -Y⁵-T²-V ou -V ;
V représente ∼SH, ~NH₂, -CH=CH₂, ∼C=CH, ∼COOM, ∼I, ∼N₃, ∼C(O)NHNH2, ∼N-maléinimide, -C(O)O-N-succinimidyle
Y⁵ représente une liaison, -0- ou ∼NHCO-, ∼ désignant la liaison au groupe A³ ;
T² représente un groupe alcanediyle linéaire ou ramifié, comprenant 1 à 200 atomes de carbone,
(i) un ou plusieurs groupes CH₂ non terminaux étant le cas échéant remplacés par ∼O- et/ou
(ii) un ou plusieurs groupes -CH₂CH₂- non terminaux étant le cas échéant remplacés par -NHCO- ou -S-S- ;
R¹ représente C(O)OM ;
R² R³ représentent H ;
R⁴, R⁶, R⁷ représentent OH ou OC(O)CH₃ ;
R⁵ représente C(O)CH₃ :
M représente CH₃ ou sodium.

4. Composé de formule (I) selon l'une quelconque des revendications 1 à 3 présentant les formules (Ia) ou (Ib), les symboles présentant les significations indiquées dans la formule (I) :

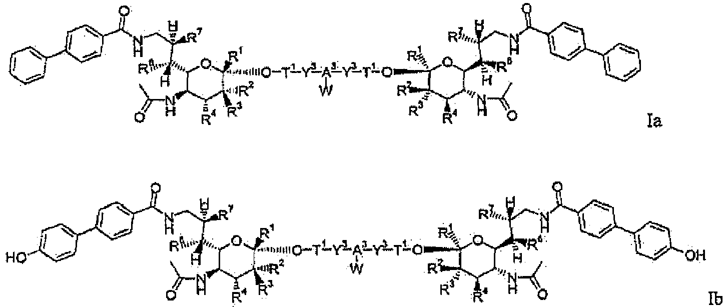

5. Composé de formule (I) selon l'une quelconque des revendications 1 à 4, choisi dans le groupe constitué par :

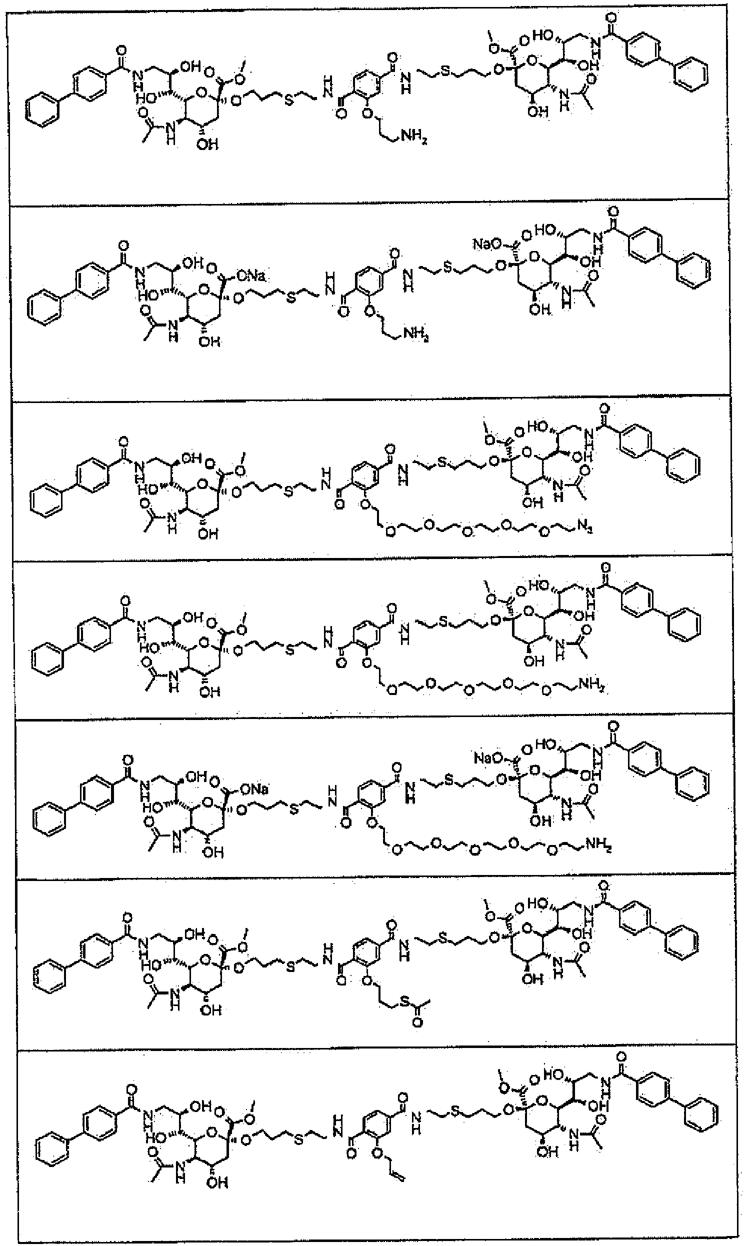

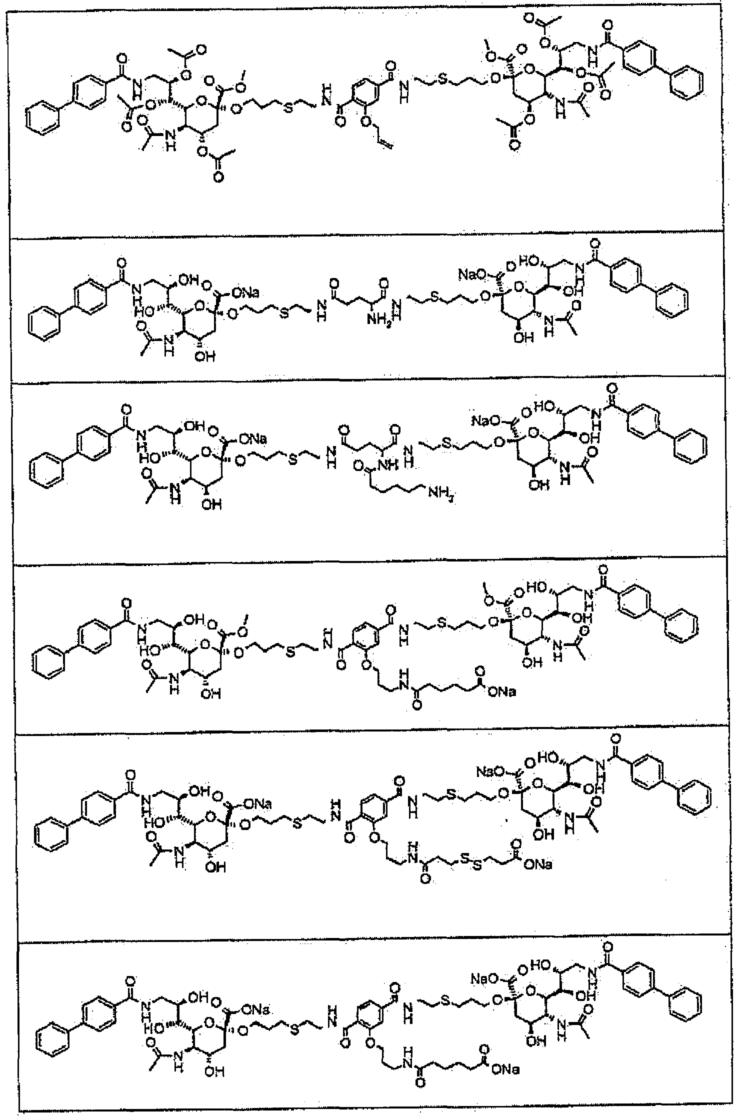

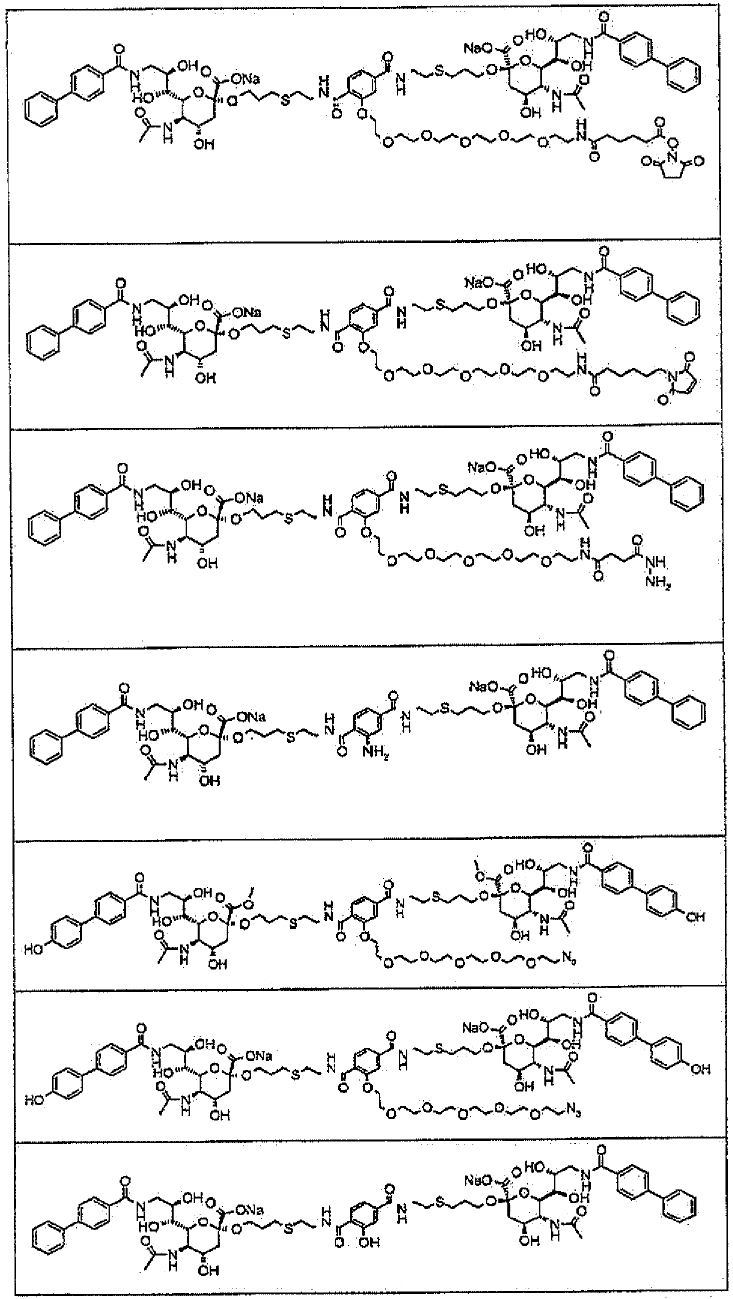

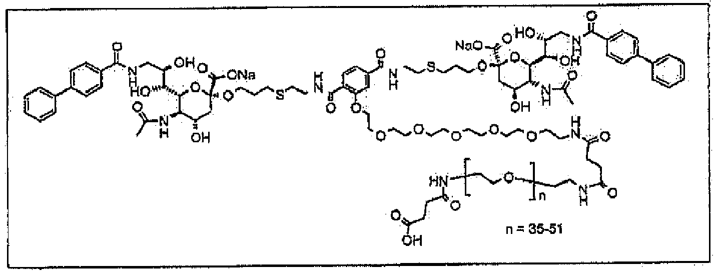

6. Conjugué, choisi dans le groupe constitué par :

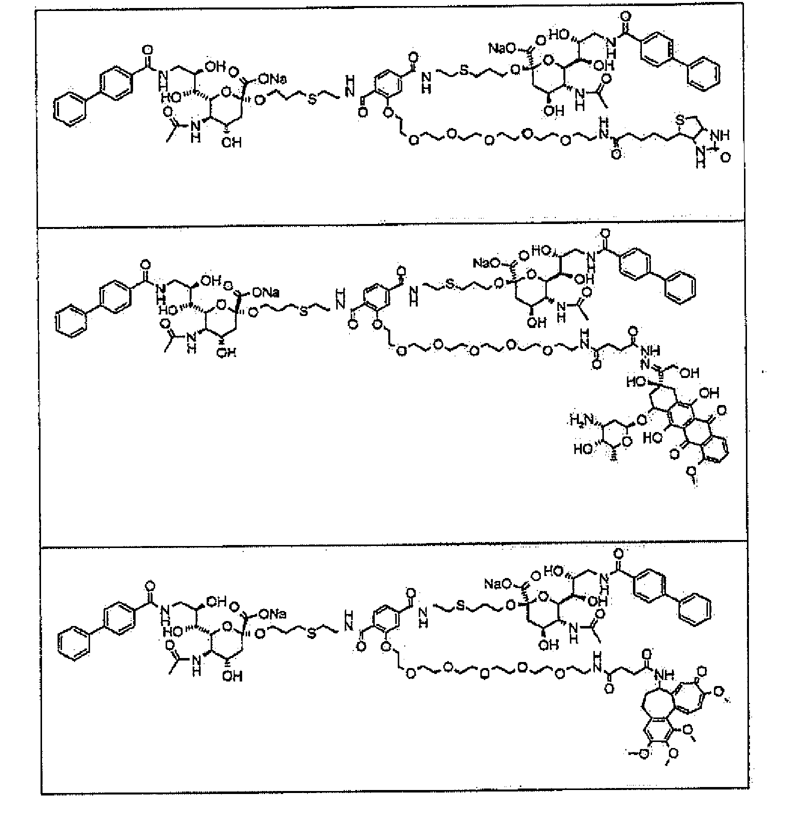

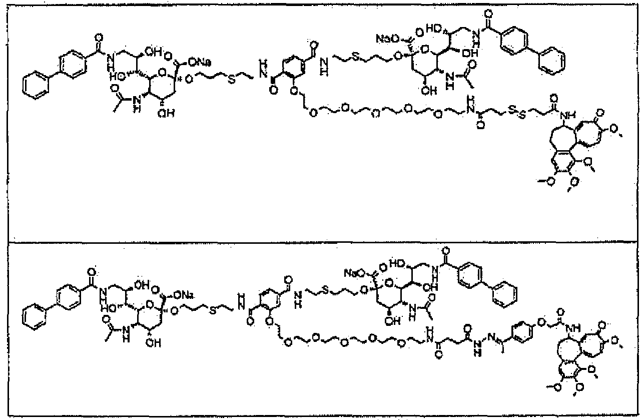

7. Préparation pharmaceutique, contenant au moins un composé de formule (I) selon l'une quelconque des revendications 1 à 5 ou un sel pharmacologiquement acceptable de celui-ci et un support pharmacologiquement acceptable.

8. Composé de formule (I) selon l'une quelconque des revendications 1 à 5 destiné à être utilisé pour la préparation d'un médicament pour le traitement de maladies bactériennes, virales, parasitaires, tumorales, auto-immunes et immunodéficientes.

9. Composé de formule (I) selon l'une quelconque des revendications 1 à 5 pour la liaison à un cargo, choisi dans le groupe constitué par l'ARN, l'ADN, les peptides, les antigènes de bas poids moléculaire, les protéines antigènes, les enzymes et les substances pharmacologiquement actives de bas poids moléculaire, destiné à être utilisé dans un procédé pour la régulation de processus métaboliques, de réactions immunes, d'immunisations ou de désensibilisations de l'organisme cible.

10. Conjugué pharmacologiquement actif d'un composé de formule (I) selon l'une quelconque des revendications 1 à 5 et d'un cargo, choisi dans le groupe constitué par l'ARN, l'ADN, les peptides, les cytostatiques, les enzymes, les complexes métallo-organiques et les substances pharmacologiquement actives de bas poids moléculaire, destiné à être utilisé dans un procédé pour le traitement d'infections, de maladies tumorales ou d'allergies.

11. Utilisation d'un composé de formule (I) selon l'une quelconque des revendications 1 à 5 pour la liaison à des liposomes, des nanoparticules, des complexes métallo-organiques, des nanoparticules métalliques, des micromicelles et des nanotubes de carbone.

12. Utilisation d'un composé de formule (I) selon l'une quelconque des revendications 1 à 5 pour la préparation de liposomes, de nanoparticules, de micromicelles et de nanotubes de carbone.

13. Utilisation d'un composé de formule (I) selon l'une quelconque des revendications 1 à 5 pour la liaison à des molécules à des fins diagnostiques.

14. Utilisation d'un composé de formule (I) selon l'une quelconque des revendications 1 à 5 pour la préparation de ligands polyvalents.

15. Utilisation d'un composé de formule (I) selon l'une quelconque des revendications 1 à 5 pour la liaison à un polymère portant un cargo, le cargo étant choisi dans le groupe constitué par l'ARN, l'ADN, les cytostatiques, les peptides, les antigènes de bas poids moléculaire, les protéines antigènes, les enzymes, les complexes métallo-organiques et les substances pharmacologiquement actives de bas poids moléculaire.
